# EUROPEAN PATENT SPECIFICATION

(11) **EP 0 693 078 B1**
(45) Date of publication and mention of the grant of the patent: **23.06.1999**
(21) Application number: 94909742.2
(22) Date of filing: 22.02.1994
(51) Int. Cl.: C12N 15/54, C12N 9/12, C12Q 1/68

(54) **DNA POLYMERASES WITH ENHANCED THERMOSTABILITY AND ENHANCED LENGTH AND EFFICIENCY OF PRIMER EXTENSION**
DNS-POLYMERASEN MIT ERHÖHTER TEMPERATURSTABILITÄT UND VERGRÖSSTERTER LÄNGE UND EFFIZIENT DER PRIMER-VERLÄNGERUNG
ADN POLYMERASES PRESENTANT UNE EXTENSION D'AMORCE DE THERMOSTABILITE, DE LONGUEUR ET D'EFFICACITE ACCRUES

(30) Priority: 19.02.1993 US 21623
(43) Date of publication of application: 24.01.1996
(73) Proprietor: Takara Shuzo Co, Ltd., Shiga (JP)
(72) Inventor: Barnes, Wayne M., Chesterfield, Missouri 63017 (US)
(74) Representative: Allard, Susan Joyce
(86) International application number: PCT/US94/01867
(87) International publication number: WO 94/26766

(56) References cited:
- EP-A- 0 265 293
- EP-A- 0 386 857
- EP-A- 0 416 755
- WO-A-89/06691
- WO-A-91/02090
- WO-A-92/06188
- WO-A-92/06200
- WO-A-92/09689
- US-A- 5 001 050
- US-A- 5 079 352
- NUCLEIC ACIDS RESEARCH, vol. 19, no. 9, 11 May 1991, page 2511 XP000570402 YU SHENG ZHU ET AL: "THE USE OF EXONUCLEASE III FOR POLYMERASE CHAIN REACTION STERILIZATION"
- THE NEB TRANSCRIPT, vol. 3, 1991, BEVERLY, MA, US, page 4 XP000391405 ANONYMOUS: "Recombinant VentR (TM) DNA polymerase"
- GENE, vol. 112, no. 1, 1 March 1992, ELSEVIER SCIENCE PUBLISHERS,B.V.,AMSTERDAM,NL;, pages 29-35, XP002030132 W.M. BARNES: "The fildelity of Taq polymerase catalyzing PCR is improved by an N-terminal deletion"
- PROC. NATL.ACAD SCI., vol. 91, April 1994, NATL. ACAD SCI.,WASHINGTON,DC,US;, pages 2216-2220, XP002030133 W.M. BARNES: "PCR amplification of up to 35-kb DNA with high fildelity and high yield from lambda bacteriophage templates"
- PROC. NATL.ACAD SCI., vol. 91, June 1994, NATL. ACAD SCI.,WASHINGTON,DC,US;, pages 5695-5699, XP002030134 S. CHENG ET AL.: "Effective amplification of long targets from cloned inserts and human genomic DNA"
- Gene, Volume 108, issued 1991, K.S. LUNDBERG et al., "High-Fidelity Amplification Using a Thermostable DNA Polymerase Isolated from Pyrococcus Furiosus", pages 1-6, see entire document.
- Nucleic Acids Research, Volume 19, No. 18, issued 1991, P. MATTILA et al., "Fidelity of DNA Synthesis by the Thermococcus Litoralis DNA Polymerase--an Extremely Heat Stable Enzyme with Proofreading Activity", pages 4967-4973, especially Abstract, pages 4972-4973.
- PCR Methods and Applications, Volume 2, No. 4, issued May 1993, F.C. LAWYER et al., "High-Level Expression, Purification, and Enzymatic Characterization of Full-Length Thermus Aquaticus DNA Polymerase and a Truncated for Deficient in 5' to 3' Exonuclase Activity", pages 275-287, especially Abstract.

### SEQUENCE LISTING

### (1) GENERAL INFORMATION:

(i) APPLICANT: Barnes Ph.D., Wayne M
(ii) TITLE OF INVENTION: DNA polymerases with enhanced thermostability and enhanced length and efficiency of primer extension
(iii) NUMBER OF SEQUENCES: 33
(iv) CORRESPONDENCE ADDRESS:
   (A) ADDRESSEE: Senniger, Powers, Leavitt & Roedel
   (B) STREET: One Metropolitan Square
   (C) CITY: St. Louis
   (D) STATE: Missouri
   (E) COUNTRY: USA
   (F) ZIP: 63102
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE: 22-FEB-1994
   (C) CLASSIFICATION:
(viii) ATTORNEY/AGENT INFORMATION:
   (A) NAME: Blosser, G.Harley
   (B) REGISTRATION NUMBER: 33,650
   (C) REFERENCE/DOCKET NUMBER: WNB4903
(ix) TELECOMMUNICATION INFORMATION:
   (A) TELEPHONE: (314) 231-5400
   (B) TELEFAX: (314) 231-4342
   (C) TELEX: 6502697583 MCI

### (2) INFORMATION FOR SEQ ID NO:1:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(iv) ANTI-SENSE: NO
(v) FRAGMENT TYPE: N-terminal
(vi) ORIGINAL SOURCE:
   (A) ORGANISM: Thermus aquaticus
   (B) STRAIN: YT1
(vii) IMMEDIATE SOURCE:
   (A) LIBRARY: synthetic
   (B) CLONE: KT1
(ix) FEATURE:
   (A) NAME/KEY: CDS
   (B) LOCATION: 6..35
(xi) SEQUENCE DESCRIPTION: SEQ ID NO:1:
   GAGCC ATG GGC CTC CTC CAC GAG TTC GGC CTT CTG G                    36
         Met Gly Leu Leu His Glu Phe Gly Leu Leu
           1               5                  10

### (2) INFORMATION FOR SEQ ID NO:2:

(i) SEQUENCE CHARACTERISTICS:
   (A) LENGTH: 10 amino acids
   (B) TYPE: amino acid
   (D) TOPOLOGY: linear
(ii) MOLECULE TYPE: protein
(xi) SEQUENCE DESCRIPTION: SEQ ID NO:2:
   Met Gly Leu Leu His Glu Phe Gly Leu Leu
     1               5                  10

### (2) INFORMATION FOR SEQ ID NO:3:

(i) SEQUENCE CHARACTERISTICS:
   (A) LENGTH: 35 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(iv) ANTI-SENSE: YES
(v) FRAGMENT TYPE: C-terminal
(vi) ORIGINAL SOURCE:
   (A) ORGANISM: Thermus aquaticus
   (B) STRAIN: YT1
(vii) IMMEDIATE SOURCE:
   (A) LIBRARY: synthetic
   (B) CLONE: Klentaq32
(ix) FEATURE:
   (A) NAME/KEY: CDS
   (B) LOCATION: complement (8..34)
(xi) SEQUENCE DESCRIPTION: SEQ ID NO:3:
   GCGAAGCTTA CTACTCCTTG GCGGAGAGCC AGTCC                                35

### (2) INFORMATION FOR SEQ ID NO:4:

(i) SEQUENCE CHARACTERISTICS:
   (A) LENGTH: 9 amino acids
   (B) TYPE: amino acid
   (D) TOPOLOGY: linear
(ii) MOLECULE TYPE: protein
(xi) SEQUENCE DESCRIPTION: SEQ ID NO:4:
   Asp Trp Leu Ser Ala Lys Glu  *   *
    1               5

### (2) INFORMATION FOR SEQ ID NO:5:

(i) SEQUENCE CHARACTERISTICS:
   (A) LENGTH: 6714 base pairs
   (B) TYPE: nucleic acid
   (C) STRANDEDNESS: double
   (D) TOPOLOGY: circular
(ii) MOLECULE TYPE: DNA (genomic)
(iii) HYPOTHETICAL: NO
(iv) ANTI-SENSE: NO
(vi) ORIGINAL SOURCE:
   (A) ORGANISM: Expression vector
(vii) IMMEDIATE SOURCE:
   (B) CLONE: pWB254b
(ix) FEATURE:
   (A) NAME/KEY: CDS
   (B) LOCATION: 1..1665
(xi) SEQUENCE DESCRIPTION: SEQ ID NO:5:
   ATG GGC CTC CTC CAC GAG TTC GGC CTT CTG GAA AGC CCC AAG GCC CTG      48
   Met Gly Leu Leu His Glu Phe Gly Leu Leu Glu Ser Pro Lys Ala Leu
    1               5                  10                  15
   GAG GAG GCC CCC TGG CCC CCG CCG GAA GGG GCC TTC GTG GGC TTT GTG      96
   Glu Glu Ala Pro Trp Pro Pro Pro Glu Gly Ala Phe Val Gly Phe Val
               20                  25                  30
   CTT TCC CGC AAG GAG CCC ATG TGG GCC GAT CTT CTG GCC CTG GCC GCC     144
   Leu Ser Arg Lys Glu Pro Met Trp Ala Asp Leu Leu Ala Leu Ala Ala
           35                  40                  45
   GCC AGG GGG GGC CGG GTC CAC CGG GCC CCC GAG CCT TAT AAA GCC CTC     192
   Ala Arg Gly Gly Arg Val His Arg Ala Pro Glu Pro Tyr Lys Ala Leu
       50                  55                  60
   AGG GAC CTG AAG GAG GCG CGG GGG CTT CTC GCC AAA GAC CTG AGC GTT     240
   Arg Asp Leu Lys Glu Ala Arg Gly Leu Leu Ala Lys Asp Leu Ser Val
    65                  70                  75                  80
   CTG GCC CTG AGG GAA GGC CTT GGC CTC CCG CCC GGC GAC GAC CCC ATG     288
   Leu Ala Leu Arg Glu Gly Leu Gly Leu Pro Pro Gly Asp Asp Pro Met
                   85                  90                  95
   CTC CTC GCC TAC CTC CTG GAC CCT TCC AAC ACC ACC CCC GAG GGG GTG     336
   Leu Leu Ala Tyr Leu Leu Asp Pro Ser Asn Thr Thr Pro Glu Gly Val
               100                 105                 110
   GCC CGG CGC TAC GGC GGG GAG TGG ACG GAG GAG GCG GGG GAG CGG GCC     384
   Ala Arg Arg Tyr Gly Gly Glu Trp Thr Glu Glu Ala Gly Glu Arg Ala
           115                 120                 125
   GCC CTT TCC GAG AGG CTC TTC GCC AAC CTG TGG GGG AGG CTT GAG GGG      432
   Ala Leu Ser Glu Arg Leu Phe Ala Asn Leu Trp Gly Arg Leu Glu Gly
       130                 135                 140
   GAG GAG AGG CTC CTT TGG CTT TAC CGG GAG GTG GAG AGG CCC CTT TCC      480
   Glu Glu Arg Leu Leu Trp Leu Tyr Arg Glu Val Glu Arg Pro Leu Ser
   145                 150                 155                 160
   GCT GTC CTG GCC CAC ATG GAG GCC ACG GGG GTG CGC CTG GAC GTG GCC      528
   Ala Val Leu Ala His Met Glu Ala Thr Gly Val Arg Leu Asp Val Ala
                   165                 170                 175
   TAT CTC AGG GCC TTG TCC CTG GAG GTG GCC GAG GAG ATC GCC CGC CTC      576
   Tyr Leu Arg Ala Leu Ser Leu Glu Val Ala Glu Glu Ile Ala Arg Leu
               180                 185                 190
   GAG GCC GAG GTC TTC CGC CTG GCC GGC CAC CCC TTC AAC CTC AAC TCC      624
   Glu Ala Glu Val Phe Arg Leu Ala Gly His Pro Phe Asn Leu Asn Ser
           195                 200                 205
   CGG GAC CAG CTG GAA AGG GTC CTC TTT GAC GAG CTA GGG CTT CCC GCC      672
   Arg Asp Gln Leu Glu Arg Val Leu Phe Asp Glu Leu Gly Leu Pro Ala
       210                 215                 220
   ATC GGC AAG ACG GAG AAG ACC GGC AAG CGC TCC ACC AGC GCC GCC GTC      720
   Ile Gly Lys Thr Glu Lys Thr Gly Lys Arg Ser Thr Ser Ala Ala Val
   225                 230                 235                 240
   CTG GAG GCC CTC CGC GAG GCC CAC CCC ATC GTG GAG AAG ATC CTG CAG      768
   Leu Glu Ala Leu Arg Glu Ala His Pro Ile Val Glu Lys Ile Leu Gln
                   245                 250                 255
   TAC CGG GAG CTC ACC AAG CTG AAG AGC ACC TAC ATT GAC CCC TTG CCG      816
   Tyr Arg Glu Leu Thr Lys Leu Lys Ser Thr Tyr Ile Asp Pro Leu Pro
               260                 265                 270
   GAC CTC ATC CAC CCC AGG ACG GGC CGC CTC CAC ACC CGC TTC AAC CAG      864
   Asp Leu Ile His Pro Arg Thr Gly Arg Leu His Thr Arg Phe Asn Gln
           275                 280                 285
   ACG GCC ACG GCC ACG GGC AGG CTA AGT AGC TCC GAT CCC AAC CTC CAG      912
   Thr Ala Thr Ala Thr Gly Arg Leu Ser Ser Ser Asp Pro Asn Leu Gln
       290                 295                 300
   AAC ATC CCC GTC CGC ACC CCG CTT GGG CAG AGG ATC CGC CGG GCC TTC      960
   Asn Ile Pro Val Arg Thr Pro Leu Gly Gln Arg Ile Arg Arg Ala Phe
   305                 310                 315                 320
   ATC GCC GAG GAG GGG TGG CTA TTG GTG GCC CTG GAC TAT AGC CAG ATA     1008
   Ile Ala Glu Glu Gly Trp Leu Leu Val Ala Leu Asp Tyr Ser Gln Ile
                   325                 330                 335
   GAG CTC AGG GTG CTG GCC CAC CTC TCC GGC GAC GAG AAC CTG ATC CGG     1056
   Glu Leu Arg Val Leu Ala His Leu Ser Gly Asp Glu Asn Leu Ile Arg
               340                 345                 350
   GTC TTC CAG GAG GGG CGG GAC ATC CAC ACG GAG ACC GCC AGC TGG ATG     1104
   Val Phe Gln Glu Gly Arg Asp Ile His Thr Glu Thr Ala Ser Trp Met
           355                 360                 365
   TTC GGC GTC CCC CGG GAG GCC GTG GAC CCC CTG ATG CGC CGG GCG GCC     1152
   Phe Gly Val Pro Arg Glu Ala Val Asp Pro Leu Met Arg Arg Ala Ala
       370                 375                 380
   AAG ACC ATC AAC TTC GGG GTC CTC TAC GGC ATG TCG GCC CAC CGC CTC     1200
   Lys Thr Ile Asn Phe Gly Val Leu Tyr Gly Met Ser Ala His Arg Leu
   385                 390                 395                 400
   TCC CAG GAG CTA GCC ATC CCT TAC GAG GAG GCC CAG GCC TTC ATT GAG      1248
   Ser Gln Glu Leu Ala Ile Pro Tyr Glu Glu Ala Gln Ala Phe Ile Glu
                   405                 410                 415
   CGC TAC TTT CAG AGC TTC CCC AAG GTG CGG GCC TGG ATT GAG AAG ACC      1296
   Arg Tyr Phe Gln Ser Phe Pro Lys Val Arg Ala Trp Ile Glu Lys Thr
               420                 425                 430
   CTG GAG GAG GGC AGG AGG CGG GGG TAC GTG GAG ACC CTC TTC GGC CGC      1344
   Leu Glu Glu Gly Arg Arg Arg Gly Tyr Val Glu Thr Leu Phe Gly Arg
           435                 440                 445
   CGC CGC TAC GTG CCA GAC CTA GAG GCC CGG GTG AAG AGC GTG CGG GAG      1392
   Arg Arg Tyr Val Pro Asp Leu Glu Ala Arg Val Lys Ser Val Arg Glu
       450                 455                 460
   GCG GCC GAG CGC ATG GCC TTC AAC ATG CCC GTC CAG GGC ACC GCC GCC      1440
   Ala Ala Glu Arg Met Ala Phe Asn Met Pro Val Gln Gly Thr Ala Ala
   465                 470                 475                 480
   GAC CTC ATG AAG CTG GCT ATG GTG AAG CTC TTC CCC AGG CTG GAG GAA      1488
   Asp Leu Met Lys Leu Ala Met Val Lys Leu Phe Pro Arg Leu Glu Glu
                   485                 490                 495
   ATG GGG GCC AGG ATG CTC CTT CAG GTC CAC GAC GAG CTG GTC CTC GAG      1536
   Met Gly Ala Arg Met Leu Leu Gln Val His Asp Glu Leu Val Leu Glu
               500                 505                 510
   GCC CCA AAA GAG AGG GCG GAG GCC GTG GCC CGG CTG GCC AAG GAG GTC      1584
   Ala Pro Lys Glu Arg Ala Glu Ala Val Ala Arg Leu Ala Lys Glu Val
           515                 520                 525
   ATG GAG GGG GTG TAT CCC CTG GCC GTG CCC CTG GAG GTG GAG GTG GGG      1632
   Met Glu Gly Val Tyr Pro Leu Ala Val Pro Leu Glu Val Glu Val Gly
       530                 535                 540
   ATA GGG GAG GAC TGG CTC TCC GCC AAG GAG TAGTAAGCTT ATCGATGATA        1682
   Ile Gly Glu Asp Trp Leu Ser Ala Lys Glu
   545                 550                 555
   AGCTGTCAAA CATGAGAATT AGCCCGCCTA ATGAGCGGGC TTTTTTTTAA TTCTTGAAGA    1742
   CGAAAGGGCC TCGTGATACG CCTATTTTTA TAGGTTAATG TCATGATAAT AATGGTTTCT    1802
   TAGCGTCAAA GCAACCATAG TACGCGCCCT GTAGCGGCGC ATTAAGCGCG CCGGGTGTGG    1862
   TGGTTACGCG CAGCGTGACC GCTACACTTG CCAGCGCCCT AGCGCCCGCT CCTTTCGCTT    1922
   TCTTCCCTTC CTTTCTCGCC ACGTTCGCCG GCTTTCCCCG TCAAGCTCTA AATCGGGGGC    1982
   TCCCTTTAGG GTTCCGATTT AGTGCTTTAC GGCACCTCGA CCCCAAAAAA CTTGATTTGG    2042
   GTGATGGTTC ACGTAGTGGG CCATCGCCCT GATAGACGGT TTTTCGCCCT TTGACGTTGG    2102
   AGTCCACGTT CTTTAATAGT GGACTCTTGT TCCAAACTTG AACAACACTC AACCCTATCT    2162
   CGGGCTATTC TTTTGATTTA TAAGGGATTT TGCCGATTTC GGCCTATTGG TTAAAAAATG    2222
   AGCTGATTTA ACAAAAATTT AACGCGAATT TTAACAAAAT ATTAACGTTT ACAATTTCAG    2282
   GTGGCACTTT TCGGGGAAAT GTGCGCGGAA CCCCTATTTG TTTATTTTTC TAAATACATT    2342
   CAAATATGTA TCCGCTCATG AGACAATAAC CCTGATAAAT GCTTCAATAA TATTGAAAAA    2402
   GGAAGAGTAT GAGTATTCAA CATTTCCGTG TCGCCCTTAT TCCCTTTTTT GCGGCATTTT    2462
   GCCTTCCTGT TTTTGCTCAC CCAGAAACGC TGGTGAAAGT AAAAGATGCT GAAGATCAGT    2522
   TGGGTGCACG AGTGGGTTAC ATCGAACTGG ATCTCAACAG CGGTAAGATC CTTGAGAGTT 2582
   TTCGCCCCGA AGAACGTTTT CCAATGATGA GCACTTTTAA AGTTCTGCTA TGTGGCGCGG 2642
   TATTATCCCG TGTTGACGCC GGGCAAGAGC AACTCGGTCG CCGCATACAC TATTCTCAGA 2702
   ATGACTTGGT TGAGTACTCA CCAGTCACAG AAAAGCATCT TACGGATGGC ATGACAGTAA 2762
   GAGAATTATG CAGTGCTGCC ATAACCATGA GTGATAACAC TGCGGCCAAC TTACTTCTGA 2822
   CAACGATCGG AGGACCGAAG GAGCTAACCG CTTTTTTGCA CAACATGGGG GATCATGTAA 2882
   CTCGCCTTGA TCGTTGGGAA CCGGAGCTGA ATGAAGCCAT ACCAAACGAC GAGCGTGACA 2942
   CCACGATGCC TGCAGCAATG GCAACAACGT TGCGCAAACT ATTAACTGGC GAACTACTTA 3002
   CTCTAGCTTC CCGGCAACAA TTAATAGACT GGATGGAGGC GGATAAAGTT GCAGGACCAC 3062
   TTCTGCGCTC GGCCCTTCCG GCTGGCTGGT TTATTGCTGA TAAATCTGGA GCCGGTGAGC 3122
   GTGGGTCTCG CGGTATCATT GCAGCACTGG GGCCAGATGG TAAGCCCTCC CGTATCGTAG 3182
   TTATCTACAC GACGGGGAGT CAGGCAACTA TGGATGAACG AAATAGACAG ATCGCTGAGA 3242
   TAGGTGCCTC ACTGATTAAG CATTGGTAAC TGTCAGACCA AGTTTACTCA TATATACTTT 3302
   AGATTGATTT AAAACTTCAT TTTTAATTTA AAAGGATCTA GGTGAAGATC CTTTTTGATA 3362
   ATCTCATGAC CAAAATCCCT TAACGTGAGT TTTCGTTCCA CTGAGCGTCA GACCCCGTAG 3422
   AAAAGATCAA AGGATCTTCT TGAGATCCTT TTTTTCTGCG CGTAATCTGC TGCTTGCAAA 3482
   CAAAAAAACC ACCGCTACCA GCGGTGGTTT GTTTGCCGGA TCAAGAGCTA CCAACTCTTT 3542
   TTCCGAAGGT AACTGGCTTC AGCAGAGCGC AGATACCAAA TACTGTCCTT CTAGTGTAGC 3602
   CGTAGTTAGG CCACCACTTC AAGAACTCTG TAGCACCGCC TACATACCTC GCTCTGCTAA 3662
   TCCTGTTACC AGTGGCTGCT GCCAGTGGCG ATAAGTCGTG TCTTACCGGG TTGGACTCAA 3722
   GACGATAGTT ACCGGATAAG GCGCAGCGGT CGGGCTGAAC GGGGGGTTCG TGCACACAGC 3782
   CCAGCTTGGA GCGAACGACC TACACCGAAC TGAGATACCT ACAGCGTGAG CTATGAGAAA 3842
   GCGCCACGCT TCCCGAAGGG AGAAAGGCGG ACAGGTATCC GGTAAGCGGC AGGGTCGGAA 3902
   CAGGAGAGCG CACGAGGGAG CTTCCAGGGG GAAACGCCTG GTATCTTTAT AGTCCTGTCG 3962
   GGTTTCGCCA CCTCTGACTT GAGCGTCGAT TTTTGTGATG CTCGTCAGGG GGGCGGAGCC 4022
   TATGGAAAAA CGCCAGCAAC GCGGCCTTTT TACGGTTCCT GGCCTTTTGC TGGCCTTTTG 4082
   CTCACATGTT CTTTCCTGCG TTATCCCCTG ATTCTGTGGA TAACCGTATT ACCGCCTTTG 4142
   AGTGAGCTGA TACCGCTCGC CGCAGCCGAA CGACCGAGCG CAGCGAGTCA GTGAGCGAGG 4202
   AAGCGGAAGA GCGCCTGATG CGGTATTTTC TCCTTACGCA TCTGTGCGGT ATTTCACACC 4262
   GCATATGGTG CACTCTCAGT ACAATCTGCT CTGATGCCGC ATAGTTAAGC CAGTATACAC 4322
   TCCGCTATCG CTACGTGACT GGGTCATGGC TGCGCCCCGA CACCCGCCAA CACCCGCTGA 4382
   CGCGCCCTGA CGGGCTTGTC TGCTCCCGGC ATCCGCTTAC AGACAAGCTG TGACCGTCTC 4442
   CGGGAGCTGC ATGTGTCAGA GGTTTTCACC GTCATCACCG AAACGCGCGA GGCAGAACGC 4502
   CATCAAAAAT AATTCGCGTC TGGCCTTCCT GTAGCCAGCT TTCATCAACA TTAAATGTGA 4562
   GCGAGTAACA ACCCGTCGGA TTCTCCGTGG GAACAAACGG CGGATTGACC GTAATGGGAT 4622
   AGGTTACGTT GGTGTAGATG GGCGCATCGT AACCGTGCAT CTGCCAGTTT GAGGGGACGA 4682
   CGACAGTATC GGCCTCAGGA AGATCGCACT CCAGCCAGCT TTCCGGCACC GCTTCTGGTG 4742
   CCGGAAACCA GGCAAAGCGC CATTCGCCAT TCAGGCTGCG CAACTGTTGG GAAGGGCGAT 4802
   CGGTGCGGGC CTCTTCGCTA TTACGCCAGC TGGCGAAAGG GGGATGTGCT GCAAGGCGAT 4862
   TAAGTTGGGT AACGCCAGGG TTTTCCCAGT CACGACGTTG TAAAACGACG GCCAGTGAAT 4922
   CCGTAATCAT GGTCATAGCT GTTTCCTGTG TGAAATTGTT ATCCGCTCAC AATTCCACAC 4982
   AACATACGAG CCGGAAGCAT AAAGTGTAAA GCCTGGGGTG CCTAATGAGT GAGCTAACTC 5042
   ACATTAATTG CGTTGCGCTC ACTGCCCGCT TTCCAGTCGG GAAACCTGTC GTGCCAGCTG 5102
   CATTAATGAA TCGGCCAACG CGCGGGGAGA GGCGGTTTGC GTATTGGGCG CCAGGGTGGT 5162
   TTTTCTTTTC ACCAGTGAGA CGGGCAACAG CTGATTGCCC TTCACCGCCT GGCCCTGAGA 5222
   GAGTTGCAGC AAGCGGTCCA CGCTGGTTTG CCCCAGCAGG CGAAAATCCT GTTTGATGGT 5282
   GGTTGACGGC GGGATATAAC ATGAGCTGTC TTCGGTATCG TCGTATCCCA CTACCGAGAT 5342
   ATCCGCACCA ACGCGCAGCC CGGACTCGGT AATGGCGCGC ATTGCGCCCA GCGCCATCTG 5402
   ATCGTTGGCA ACCAGCATCG CAGTGGGAAC GATGCCCTCA TTCAGCATTT GCATGGTTTG 5462
   TTGAAAACCG GACATGGCAC TCCAGTCGCC TTCCCGTTCC GCTATCGGCT GAATTTGATT 5522
   GCGAGTGAGA TATTTATGCC AGCCAGCCAG ACGCAGACGC GCCGAGACAG AACTTAATGG 5582
   GCCCGCTAAC AGCGCGATTT GCTGGTGACC CAATGCGACC AGATGCTCCA CGCCCAGTCG 5642
   CGTACCGTCT TCATGGGAGA AAATAATACT GTTGATGGGT GTCTGGTCAG AGACATCAAG 5702
   AAATAACGCC GGAACATTAG TGCAGGCAGC TTCCACAGCA ATGGCATCCT GGTCATCCAG 5762
   CGGATAGTTA ATGATCAGCC CACTGACGCG TTGCGCGAGA AGATTGTGCA CCGCCGCTTT 5822
   ACAGGCTTCG ACGCCGCTTC GTTCTACCAT CGACACCACC ACGCTGGCAC CCAGTTGATC 5882
   GGCGCGAGAT TTAATCGCCG CGACAATTTG CGACGGCGCG TGCAGGGCCA GACTGGAGGT 5942
   GGCAACGCCA ATCAGCAACG ACTGTTTGCC CGCCAGTTGT TGTGCCACGC GGTTGGGAAT 6002
   GTAATTCAGC TCCGCCATCG CCGCTTCCAC TTTTTCCCGC GTTTTCGCAG AAACGTGGCT 6062
   GGCCTGGTTC ACCACGCGGG AAACGGTCTG ATAAGAGACA CCGGCATACT CTGCGACATC 6122
   GTATAACGTT ACTGGTTTCA CATTCACCAC CCTGAATTGA CTCTCTTCCG GGCGCTATCA 6182
   TGCCATACCG CGAAAGGTTT TGCGCCATTC GATGGTGTCC CAGTGAATCC GTAATCATGG 6242
   TCATAGCTGT TTCCTGTGTG AAATTGTTAT CCGCTCACAA TTCCACACAT TATACGAGCC 6302
   GGAAGCATAA AGTGTAAAGC CTGGGGTGCC TAATGAGTGA GCTAACTCAC ATTAATTGCG 6362
   TTGCGCTCAC TGCCCGCTTT CCAGTCGGGA AACCTGTCGT GCCAGCTGCA TTAATGAATC 6422
   GGAGCTTACT CCCCATCCCC CTGTTGACAA TTAATCATCG GCTCGTATAA TGTGTGGAAT 6482
   TGTGAGCGGA TAACAATTTC ACACAGGAAA CAGGATCGAT CCAGCTTACT CCCCATCCCC 6542
   CTGTTGACAA TTAATCATCG GCTCGTATAA TGTGTGGAAT TGTGAGCGGA TAACAATTTC 6602
   ACACAGGAAA CAGGATCTGG GCCCTTCGAA ATTAATACGA CTCACTATAG GGAGACCACA 6662
   ACGGTTTCCC TCTAGAAATA ATTTTGTTTA ACTTTAAGAA GGAGATATAT CC 6714

### (2) INFORMATION FOR SEQ ID NO:6:

(i) SEQUENCE CHARACTERISTICS:
   (A) LENGTH: 554 amino acids
   (B) TYPE: amino acid
   (D) TOPOLOGY: linear
(ii) MOLECULE TYPE: protein
(xi) SEQUENCE DESCRIPTION: SEQ ID NO:6:
   Met Gly Leu Leu His Glu Phe Gly Leu Leu Glu Ser Pro Lys Ala Leu
   1 5 10 15
   Glu Glu Ala Pro Trp Pro Pro Pro Glu Gly Ala Phe Val Gly Phe Val
   20 25 30
   Leu Ser Arg Lys Glu Pro Met Trp Ala Asp Leu Leu Ala Leu Ala Ala
   35 40 45
   Ala Arg Gly Gly Arg Val His Arg Ala Pro Glu Pro Tyr Lys Ala Leu
   50 55 60
   Arg Asp Leu Lys Glu Ala Arg Gly Leu Leu Ala Lys Asp Leu Ser Val
   65 70 75 80
   Leu Ala Leu Arg Glu Gly Leu Gly Leu Pro Pro Gly Asp Asp Pro Met
   85 90 95
   Leu Leu Ala Tyr Leu Leu Asp Pro Ser Asn Thr Thr Pro Glu Gly Val
   100 105 110
   Ala Arg Arg Tyr Gly Gly Glu Trp Thr Glu Glu Ala Gly Glu Arg Ala
   115 120 125
   Ala Leu Ser Glu Arg Leu Phe Ala Asn Leu Trp Gly Arg Leu Glu Gly
   130 135 140
   Glu Glu Arg Leu Leu Trp Leu Tyr Arg Glu Val Glu Arg Pro Leu Ser
   145 150 155 160
   Ala Val Leu Ala His Met Glu Ala Thr Gly Val Arg Leu Asp Val Ala
   165 170 175
   Tyr Leu Arg Ala Leu Ser Leu Glu Val Ala Glu Glu Ile Ala Arg Leu
   180 185 190
   Glu Ala Glu Val Phe Arg Leu Ala Gly His Pro Phe Asn Leu Asn Ser
   195 200 205
   Arg Asp Gln Leu Glu Arg Val Leu Phe Asp Glu Leu Gly Leu Pro Ala
   210 215 220
   Ile Gly Lys Thr Glu Lys Thr Gly Lys Arg Ser Thr Ser Ala Ala Val
   225 230 235 240
   Leu Glu Ala Leu Arg Glu Ala His Pro Ile Val Glu Lys Ile Leu Gln
   245 250 255
   Tyr Arg Glu Leu Thr Lys Leu Lys Ser Thr Tyr Ile Asp Pro Leu Pro
   260 265 270
   Asp Leu Ile His Pro Arg Thr Gly Arg Leu His Thr Arg Phe Asn Gln
           275                 280                 285
   Thr Ala Thr Ala Thr Gly Arg Leu Ser Ser Ser Asp Pro Asn Leu Gln
       290                 295                 300
   Asn Ile Pro Val Arg Thr Pro Leu Gly Gln Arg Ile Arg Arg Ala Phe
   305                 310                 315                 320
   Ile Ala Glu Glu Gly Trp Leu Leu Val Ala Leu Asp Tyr Ser Gln Ile
                   325                 330                 335
   Glu Leu Arg Val Leu Ala His Leu Ser Gly Asp Glu Asn Leu Ile Arg
               340                 345                 350
   Val Phe Gln Glu Gly Arg Asp Ile His Thr Glu Thr Ala Ser Trp Met
           355                 360                 365
   Phe Gly Val Pro Arg Glu Ala Val Asp Pro Leu Met Arg Arg Ala Ala
       370                 375                 380
   Lys Thr Ile Asn Phe Gly Val Leu Tyr Gly Met Ser Ala His Arg Leu
   385                 390                 395                 400
   Ser Gln Glu Leu Ala Ile Pro Tyr Glu Glu Ala Gln Ala Phe Ile Glu
                   405                 410                 415
   Arg Tyr Phe Gln Ser Phe Pro Lys Val Arg Ala Trp Ile Glu Lys Thr
               420                 425                 430
   Leu Glu Glu Gly Arg Arg Arg Gly Tyr Val Glu Thr Leu Phe Gly Arg
           435                 440                 445
   Arg Arg Tyr Val Pro Asp Leu Glu Ala Arg Val Lys Ser Val Arg Glu
       450                 455                 460
   Ala Ala Glu Arg Met Ala Phe Asn Met Pro Val Gln Gly Thr Ala Ala
   465                 470                 475                 480
   Asp Leu Met Lys Leu Ala Met Val Lys Leu Phe Pro Arg Leu Glu Glu
                   485                 490                 495
   Met Gly Ala Arg Met Leu Leu Gln Val His Asp Glu Leu Val Leu Glu
               500                 505                 510
   Ala Pro Lys Glu Arg Ala Glu Ala Val Ala Arg Leu Ala Lys Glu Val
           515                 520                 525
   Met Glu Gly Val Tyr Pro Leu Ala Val Pro Leu Glu Val Glu Val Gly
       530                 535                 540
   Ile Gly Glu Asp Trp Leu Ser Ala Lys Glu
   545                 550

### (2) INFORMATION FOR SEQ ID NO:7:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: MBL
(viii) POSITION IN GENOME:
   (B) MAP POSITION: 27940
(xi) SEQUENCE DESCRIPTION: SEQ ID NO:7:
   GCTTATCTGC TTCTCATAGA GTCTTGC 27

### (2) INFORMATION FOR SEQ ID NO:8:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriphage lambda
   (B) STRAIN: PaPa
(vii) IMMEDIATE SOURCE:
   (B) CLONE: MBR
(xi) SEQUENCE DESCRIPTION: SEQ ID NO:8:
   ATAACGATCA TATACATGGT TCTCTCC 27

### (2) INFORMATION FOR SEQ ID NO:9:

(i) SEQUENCE CHARACTERISTICS:
   (A) LENGTH: 27 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
(vii) IMMEDIATE SOURCE:
   (B) CLONE: MBL-1.7
(xi) SEQUENCE DESCRIPTION: SEQ ID NO:9:
   TTTTGCTGGG TCAGGTTGTT CTTTAGG 27

### (2) INFORMATION FOR SEQ ID NO:10:

(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: DNA (genomic)
(v) FRAGMENT TYPE: C-terminal
(vi) ORIGINAL SOURCE:
   (A) ORGANISM: E.coli
   (B) STRAIN: K12
(vii) IMMEDIATE SOURCE:
   (B) CLONE: MSA19
(viii) POSITION IN GENOME:
   (B) MAP POSITION: lacZ
(xi) SEQUENCE DESCRIPTION: SEQ ID NO:10:
   GGAAGCTTAT TTTTGACACC AGACCAAC 28

### (2) INFORMATION FOR SEQ ID NO:11:

(i) SEQUENCE CHARACTERISTICS:
   (A) LENGTH: 37 base pairs
   (B) TYPE: nucleic acid
   (C) STRANDEDNESS: single
   (D) TOPOLOGY: linear
(ii) MOLECULE TYPE: cDNA to mRNA
(v) FRAGMENT TYPE: N-terminal
(vi) ORIGINAL SOURCE:
   (A) ORGANISM: Zea maize
(vii) IMMEDIATE SOURCE:
   (B) CLONE: Lc5
(viii) POSITION IN GENOME:
   (B) MAP POSITION: 5' end of color control gene Lc
(xi) SEQUENCE DESCRIPTION: SEQ ID NO:11:
   GTGATGGATC CTTCAGCTTC CCGAGTTCAG CAGGCGG 37

### (2) INFORMATION FOR SEQ ID NO:12:

(i) SEQUENCE CHARACTERISTICS:
   (A) LENGTH: 37 base pairs
   (B) TYPE: nucleic acid
   (C) STRANDEDNESS: single
   (D) TOPOLOGY: linear
(ii) MOLECULE TYPE: cDNA to mRNA
(iv) ANTI-SENSE: YES
(v) FRAGMENT TYPE: C-terminal
(vi) ORIGINAL SOURCE:
   (A) ORGANISM: Zea maize
(vii) IMMEDIATE SOURCE:
   (B) CLONE: Lc3
(viii) POSITION IN GENOME:
   (B) MAP POSITION: 3' end of color control gene Lc
(xi) SEQUENCE DESCRIPTION: SEQ ID NO:12:
   GGTCTCGAGC GAAGCTTCCC TATAGCTTTG CGAAGAG 37

### (2) INFORMATION FOR SEQ ID NO:13:

(i) SEQUENCE CHARACTERISTICS:
   (A) LENGTH: 37 base pairs
   (B) TYPE: nucleic acid
   (C) STRANDEDNESS: single
   (D) TOPOLOGY: linear
(ii) MOLECULE TYPE: DNA (genomic)
(v) FRAGMENT TYPE: internal
(vi) ORIGINAL SOURCE:
   (A) ORGANISM: Thermus aquaticus
   (B) STRAIN: YT1
(vii) IMMEDIATE SOURCE:
   (B) CLONE: KT2
(xi) SEQUENCE DESCRIPTION: SEQ ID NO:13:
   GAGCCATGGC CAACCTGTGG GGGAGGCTTG AGGGGGA                    37

### (2) INFORMATION FOR SEQ ID NO:14:

(i) SEQUENCE CHARACTERISTICS:
   (A) LENGTH: 36 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Thermus aquaticus
   (B) STRAIN: YT1
(vii) IMMEDIATE SOURCE:
   (B) CLONE: Genbank Accession no. J04639
(viii) POSITION IN GENOME:
   (A) CHROMOSOME/SEGMENT: DNA polymerase gene
   (B) MAP POSITION: 950
(xi) SEQUENCE DESCRIPTION: SEQ ID NO:14:
   AGTTTGGCAG CCTCCTCCAC GAGTTCGGCC TTCTGG 36

### (2) INFORMATION FOR SEQ ID NO:15:

(i) SEQUENCE CHARACTERISTICS:
   (A) LENGTH: 32 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Thermus aquaticus
   (B) STRAIN: YT1
(vii) IMMEDIATE SOURCE:
   (B) CLONE: Genbank Accession No. J04639
(viii) POSITION IN GENOME:
   (A) CHROMOSOME/SEGMENT: DNA polymerase gene
   (B) MAP POSITION: 2595
(xi) SEQUENCE DESCRIPTION: SEQ ID NO:15:
   GGACTGGCTC TCCGCCAAGG AGTGATACCA CC 32

### (2) INFORMATION FOR SEQ ID NO:16:

(i) SEQUENCE CHARACTERISTICS:
   (A) LENGTH: 36 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Thermus flavis
(vii) IMMEDIATE SOURCE:
   (B) CLONE: Genbank Accession No. X66105
(viii) POSITION IN GENOME:
   (A) CHROMOSOME/SEGMENT: DNA polymerase
   (B) MAP POSITION: 1378
(xi) SEQUENCE DESCRIPTION: SEQ ID NO:16:
   AGTTTGGAAG CCTCCTCCAC GAGTTCGGCC TCCTGG 36

### (2) INFORMATION FOR SEQ ID NO:17:

(i) SEQUENCE CHARACTERISTICS:
   (A) LENGTH: 35 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Thermus flavis
(vii) IMMEDIATE SOURCE:
   (B) CLONE: Genbank Accession No. X66105
(viii) POSITION IN GENOME:
   (A) CHROMOSOME/SEGMENT: DNA polymerase gene
   (B) MAP POSITION: 3023
(xi) SEQUENCE DESCRIPTION: SEQ ID NO:17:
   GGACTGGCTC TCCGCCAAGG AGTAGGGGGG TCCTG 35

### (2) INFORMATION FOR SEQ ID NO:18:

(i) SEQUENCE CHARACTERISTICS:
   (A) LENGTH: 39 base pairs
   (B) TYPE: nucleic acid
   (C) STRANDEDNESS: single
   (D) TOPOLOGY: linear
(ii) MOLECULE TYPE: DNA (genomic)
(iv) ANTI-SENSE: YES
(v) FRAGMENT TYPE: internal
(vi) ORIGINAL SOURCE:
   (A) ORGANISM: Bacillus thuringiensis
   (B) STRAIN: CryV
   (C) INDIVIDUAL ISOLATE: NRD12
(vii) IMMEDIATE SOURCE:
   (B) CLONE: BtV3
(xi) SEQUENCE DESCRIPTION: SEQ ID NO:18:
   GCGAAGCTTC TCGAGTTACG CTCAATATGG AGTTGCTTC 39

### (2) INFORMATION FOR SEQ ID NO:19:

(i) SEQUENCE CHARACTERISTICS:
   (A) LENGTH: 43 base pairs
   (B) TYPE: nucleic acid
   (C) STRANDEDNESS: single
   (D) TOPOLOGY: linear
(ii) MOLECULE TYPE: DNA (genomic)
(iv) ANTI-SENSE: NO
(v) FRAGMENT TYPE: internal
(vi) ORIGINAL SOURCE:
   (A) ORGANISM: Bacillus thuringiensis
   (B) STRAIN: NRD12
(vii) IMMEDIATE SOURCE:
   (B) CLONE: BtV5
(xi) SEQUENCE DESCRIPTION: SEQ ID NO:19:
   CCGAGATCTC CATGGATCCA AAGAATCAAG ATAAGCATCA AAG 43

### (2) INFORMATION FOR SEQ ID NO:20:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: L36
(viii) POSITION IN GENOME:
   (B) MAP POSITION: left end
(xi) SEQUENCE DESCRIPTION: SEQ ID NO:20:
   GGGCGGCGAC CTCGCGGGTT TTCGCTATTT ATGAAA 36

### (2) INFORMATION FOR SEQ ID NO:21:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(iv) ANTI-SENSE: YES
(v) FRAGMENT TYPE: N-terminal
(vi) ORIGINAL SOURCE:
   (A) ORGANISM: E.coli
   (B) STRAIN: K12
(vii) IMMEDIATE SOURCE:
   (B) CLONE: lacZ'533
(xi) SEQUENCE DESCRIPTION: SEQ ID NO:21:
   CGACGGCCAG TGAATCCGTA ATCATGGTCA TAG 33

### (2) INFORMATION FOR SEQ ID NO:22:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(v) FRAGMENT TYPE: C-terminal
(vi) ORIGINAL SOURCE:
   (A) ORGANISM: E.coli
   (B) STRAIN: K12
(vii) IMMEDIATE SOURCE:
   (B) CLONE: lacZ333
(viii) POSITION IN GENOME:
   (B) MAP POSITION: lacZ
(xi) SEQUENCE DESCRIPTION: SEQ ID NO:22:
   ACCAGCCATC GCCATCTGCT GCACGCGGAA GAA 33

### (2) INFORMATION FOR SEQ ID NO:23:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: DNA (genomic)
(iv) ANTI-SENSE: NO
(v) FRAGMENT TYPE: N-terminal
(vi) ORIGINAL SOURCE:
   (A) ORGANISM: E.coli
   (B) STRAIN: K12
(vii) IMMEDIATE SOURCE:
   (B) CLONE: lacZ536
(viii) POSITION IN GENOME:
   (B) MAP POSITION: lacZ
(xi) SEQUENCE DESCRIPTION: SEQ ID NO:23:
   CTATGACCAT GATTACGGAT TCACTGGCCG TCGTTT 36

### (2) INFORMATION FOR SEQ ID NO:24:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: MBL002
(xi) SEQUENCE DESCRIPTION: SEQ ID NO:24:
   GCAAGACTCT ATGAGAAGCA GATAAGCGAT AAG 33

### (2) INFORMATION FOR SEQ ID NO:25:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: MBL101
(viii) POSITION IN GENOME :
   (B) MAP POSITION: 27840
(xi) SEQUENCE DESCRIPTION: SEQ ID NO:25:
   ATCATTATTT GATTTCAATT TTGTCCCACT CCC 33

### (2) INFORMATION FOR SEQ ID NO:26:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: MBR001
(viii) POSITION IN GENOME:
   (B) MAP POSITION: 34576
(xi) SEQUENCE DESCRIPTION: SEQ ID NO:26:
   GGAGAGAACC ATGTATATGA TCGTTATCTG GGT 33

### (2) INFORMATION FOR SEQ ID NO:27:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: MBR202
(viii) POSITION IN GENOME:
   (B) MAP POSITION: 34793
(xi) SEQUENCE DESCRIPTION: SEQ ID NO:27:
   GCGCACAAAA CCATAGATTG CTCTTCTGTA AGG 33

### (2) INFORMATION FOR SEQ ID NO:28:

(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: DNA (genomic)
(v) FRAGMENT TYPE: C-terminal
(vi) ORIGINAL SOURCE:
   (A) ORGANISM: E.coli
   (B) STRAIN: K12
(vii) IMMEDIATE SOURCE:
   (B) CLONE: MSA1933
(viii) POSITION IN GENOME:
   (B) MAP POSITION: lacZ
(xi) SEQUENCE DESCRIPTION: SEQ ID NO:28:
   CCCGGTTATT ATTATTTTTG ACACCAGACC AAC 33

### (2) INFORMATION FOR SEQ ID NO:29:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: DNA (genomic)
(vi) ORIGINAL SOURCE:
   (A) ORGANISM: bacteriophage lambda
   (B) STRAIN: PaPa
(vii) IMMEDIATE SOURCE:
   (B) CLONE: R36
(viii) POSITION IN GENOME:
   (B) MAP POSITION: right end
(xi) SEQUENCE DESCRIPTION: SEQ ID NO:29:
   AGGTCGCCGC CCCGTAACCT GTCGGATCAC CGGAAA 36

### (2) INFORMATION FOR SEQ ID NO:30:

(i) SEQUENCE CHARACTERISTICS:
   (A) LENGTH: 53 amino acids
   (B) TYPE: amino acid
   (D) TOPOLOGY: linear
(ii) MOLECULE TYPE: protein
(v) FRAGMENT TYPE: internal
(vi) ORIGINAL SOURCE:
   (A) ORGANISM: phi 29
(viii) POSITION IN GENOME:
   (A) CHROMOSOME/SEGMENT: DNA polymerase
   (B) MAP POSITION: ending at 435
(xi) SEQUENCE DESCRIPTION: SEQ ID NO:30:
   Lys Leu Met Leu Asn Ser Leu Tyr Gly Lys Phe Ala Ser Asn Pro Asp
   1               5                   10                  15
   Val Thr Gly Lys Val Pro Tyr Leu Lys Glu Asn Gly Ala Leu Gly Phe
               20                  25                  30
   Arg Leu Gly Glu Glu Glu Thr Lys Asp Pro Val Tyr Thr Pro Met Gly
           35                  40                  45
   Val Phe Ile Thr Ala
       50

### (2) INFORMATION FOR SEQ ID NO:31:

(i) SEQUENCE CHARACTERISTICS:
   (A) LENGTH: 36 amino acids
   (B) TYPE: amino acid
   (D) TOPOLOGY: linear
(ii) MOLECULE TYPE: protein
(v) FRAGMENT TYPE: internal
(vi) ORIGINAL SOURCE:
   (A) ORGANISM: Pyrococcus furiosus
(viii) POSITION IN GENOME:
   (A) CHROMOSOME/SEGMENT: DNA polymerase
   (B) MAP POSITION: ending at 516
(xi) SEQUENCE DESCRIPTION: SEQ ID NO:31:
   Asp Tyr Arg Gln Lys Ala Ile Lys Leu Leu Ala Asn Ser Phe Tyr Gly
   1               5                   10                  15
   Tyr Tyr Gly Tyr Ala Lys Ala Arg Trp Tyr Cys Lys Glu Cys Ala Glu
               20                  25                  30
   Ser Val Thr Ala
           35

### (2) INFORMATION FOR SEQ ID NO:32:

(i) SEQUENCE CHARACTERISTICS:
   (A) LENGTH: 50 amino acids
   (B) TYPE: amino acid
   (D) TOPOLOGY: linear
(ii) MOLECULE TYPE: protein
(v) FRAGMENT TYPE: internal
(vi) ORIGINAL SOURCE:
   (A) ORGANISM: phi 29
(viii) POSITION IN GENOME:
   (A) CHROMOSOME/SEGMENT: DNA polymerase
   (B) MAP POSITION: ending at 485
(xi) SEQUENCE DESCRIPTION: SEQ ID NO:32:
   Trp Ala Arg Tyr Thr Thr Ile Thr Ala Ala Gln Ala Cys Tyr Asp Arg
   1               5                   10                  15
   Ile Ile Tyr Cys Asp Thr Asp Ser Ile His Leu Thr Gly Thr Glu Ile
               20                  25                  30
   Pro Asp Val Ile Lys Asp Ile Val Asp Pro Lys Lys Leu Gly Tyr Trp
           35                  40                  45
   Ala His
       50

### (2) INFORMATION FOR SEQ ID NO:33:

(i) SEQUENCE CHARACTERISTICS:
   (A) LENGTH: 60 amino acids
   (B) TYPE: amino acid
   (D) TOPOLOGY: linear
(ii) MOLECULE TYPE: protein
(v) FRAGMENT TYPE: internal
(vi) ORIGINAL SOURCE:
   (A) ORGANISM: Pyrococcus furiosus
(viii) POSITION IN GENOME:
   (A) CHROMOSOME/SEGMENT: DNA polymerase
   (B) MAP POSITION: ending at 576
(xi) SEQUENCE DESCRIPTION: SEQ ID NO:33:
   Trp Gly Arg Lys Tyr Ile Glu Leu Val Trp Lys Glu Leu Glu Glu Lys
   1               5                   10                  15
   Phe Gly Phe Lys Val Leu Tyr Ile Asp Thr Asp Gly Leu Tyr Ala Thr
               20                  25                  30
   Ile Pro Gly Gly Glu Ser Glu Glu Ile Lys Lys Lys Ala Leu Glu Phe
           35                  40                  45
   Val Lys Tyr Ile Asn Ser Lys Leu Pro Gly Leu Leu
       50                  55                  60

## Description

### BACKGROUND OF THE INVENTION

The present invention is directed to DNA polymerases, and more particularly, to a novel mutation of Thermus aquaticus and Thermus flavus DNA polymerases exhibiting enhanced thermostability over any form of these enzymes now known. The invention is also directed to recombinant DNA sequences encoding such DNA polymerases, and vector plasmids and host cells suitable for the expression of these recombinant DNA sequences. The invention is also directed to a novel formulation of the DNA polymerases of the present invention and other DNA polymerases, which formulation of enzymes is capable of efficiently catalyzing the amplification by PCR (the polymerase chain reaction) of unusually long and faithful products.

DNA polymerase obtained from the hot springs bacterium Thermus aquaticus (Taq DNA polymerase) has been demonstrated to be quite useful in amplification of DNA, in DNA sequencing, and in related DNA primer extension techniques because it is thermostable. Thermostable is defined herein as having the ability to withstand temperatures up to 95°C for many minutes without becoming irreversibly denatured, and the ability to polymerize DNA at high temperatures (60° to 75° C.). The DNA and amino acid sequences described by Lawyer et al., J. Biol. Chem. **264**:6427 (1989), GenBank Accession No. J04639, define the gene encoding Thermus aquaticus DNA polymerase and the enzyme Thermus aquaticus DNA polymerase as those terms are used in this application. The highly similar DNA polymerase (Tfl DNA polymerase) expressed by the closely related bacterium Thermus flavus is defined by the DNA and amino acid sequences described by Akhmetzjanov, A.A., and Vakhitov, V.A. (1992) Nucleic Acids Research **20**:5839, GenBank Accession No. X66105. These enzymes are representative of a family of DNA polymerases, also including Thermus thermophilus DNA polymerase, which are thermostable. These enzymes lack a 3'-exonuclease activity such as that which is effective for editing purposes in DNA polymerases such as E. coli DNA polymerase I, and phages T7, T3, and T4 DNA polymerases.

Gelfand et al., U.S. Patent 4,889,818 describe a wild-type (abbreviation used here: WT), native Thermus aquaticus DNA polymerase. Gelfand et al., U.S. Patent 5,079,352 describe a recombinant DNA sequence which encodes a mutein of Thermus aquaticus DNA polymerase from which the N-terminal 289 amino acids of Thermus aquaticus DNA polymerase have been deleted (claim 3 of '352, commercial name Stoffel Fragment, abbreviation used here: ST), and a recombinant DNA sequence which encodes a mutein of Thermus aquaticus DNA polymerase from which the N-terminal 3 amino acids of Thermus aquaticus DNA polymerase have been deleted (claim 4 of '352, trade name AmpliTaq, abbreviation used here: AT). Gelfand et al. report their muteins to be "fully active" in assays for DNA polymerase, but data as to their maximum thermostability is not presented.

The development of other enzymatically active mutein derivatives of Thermus aquaticus DNA polymerase is hampered, however, by the unpredictability of the impact of any particular modification on the structural and functional characteristics of the protein. Many factors, including potential disruption of critical bonding and folding patterns, must be considered in modifying an enzyme and the DNA for its expression. A significant problem associated with the creation of N-terminal deletion muteins of high-temperature Thermus aquaticus DNA polymerase is the prospect that the amino-terminus of the new protein may become wildly disordered in the higher temperature ranges, causing unfavorable interactions with the catalytic domain(s) of the protein, and resulting in denaturation. In fact, a few deletions have been constructed which appear to leave the identifiable domain for DNA polymerase intact, yet none of these deletions have thermostability at temperatures as high as 99° C. While Thermus aquaticus DNA polymerase has shown remarkable thermostability at much higher temperatures than that exhibited by other DNA polymerases, it loses enzymatic activity when exposed to temperatures above 95-97°C. Moreover, its fidelity at 72°C (the recommended temperature for DNA synthesis) is limited to an effective error rate of approximately 1/9000 bp. Gelfand et al.'s mutein ST of Thermus aquaticus DNA polymerase (with an N-terminal 289 a.a. deletion) is significantly more stable than AT, but ST exhibits significantly decreased activity when cycled to 98°C, and much less, if any, activity when cycled to 99°C, during the denaturation phase of PCR cycles.

Amplification of DNA spans by the polymerase chain reaction (PCR) has become an important and widespread tool of genetic analysis since the intro-duction of thermostable Taq DNA polymerase for its catalysis. Two limitations to prior art methods of PCR are the fidelity of the final product and the size of the product span that can be amplified. The fidelity problem has been partially addressed by the replacement of Taq DNA polymerase by Pfu (Pyrococcus furiosus) DNA polymerase, which exhibits an integral 3'-(editing) exonuclease that apparently reduces the mutations/bp/cycle from about 10⁻⁴ to about 10⁻⁵. However, experiments suggest that this enzyme is unable to amplify certain DNA sequences in the size range of 1.5 to 2 kb that Klentaq-278 (also known as Klentaq1) (N-terminal deletion of Taq DNA polymerase; WMB unpublished) or AmpliTaq (full-length Taq DNA polymerase; ref. 3) can amplify handily, and that Pfu is no more able (i.e. not able) to amplify DNA product spans in excess of 5-7 kb than is any form of Taq DNA polymerase. For full-length Taq DNA Polymerase and for N-terminally truncated variants Klentaq-278, Klentaq5 and Stoffel Fragment, PCR amplification apparently rapidly becomes inefficient or non-existent as the length of the target span exceeds 5-6 kb. This was shown even when 30 minutes was used during the extension step of each cycle.

Although there are several reports of inefficient but detectable amplification at 9-10 kb target length and one at 15 kb, most general applications are limited to 5 kb.

Kainze et al. (Analytical Biochem. **202**:4649(1992)) report a PCR amplification of over 10 kb: a 10.9 kb and a 15.6 kb product, utilizing an enzyme of unpublished biological source (commercially available as "Hot Tub" DNA polymerase). Kainze et al. report achieving a barely visible band at 15.6 kb after 30 cycles, starting with 1 ng of λ DNA template per 100 ul of reaction volume. The efficiency of this amplification was shown to be relatively low, although a quantitative calculation of the efficiency was not presented. Attempts by Kainze et al. to make WT Thermus aquaticus DNA polymerase perform in the 10-15 kb size range were not successful, nor have successful results been reported by anyone else for any form of Thermus aquaticus DNA polymerase in this size range. There is no report of any longer DNA products amplifiable by PCR.

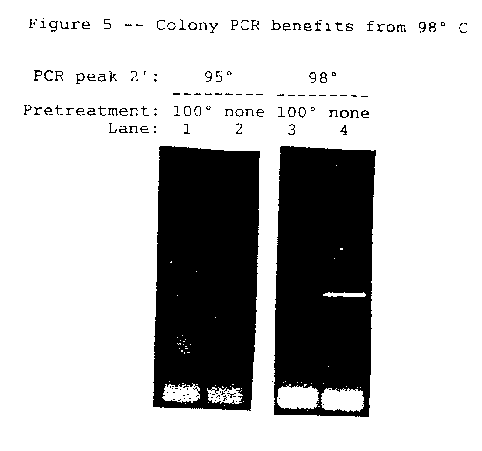

A DNA polymerase which retains its thermostability at 98° or 99°C would allow more efficient and convenient DNA analysis in several situations including "colony PCR" (see Figure 5), and/or allow thermal cycler overshoot without inactivation of the enzyme activity. A thermostable DNA polymerase or DNA polymerase formulation which exhibits improved fidelity relative to AT or WT Thermus aquaticus DNA polymerase at optimum temperatures for synthesis would be highly desirable for applications in which the target and product DNA is to be expressed rather than merely detected.

A DNA polymerase formulation capable of efficient amplification of DNA spans in excess of 6 kb would significantly expand the scope of applications of PCR. For instance, whole plasmids, and constructs the size of whole plasmids, could be prepared with this method, which would be especially valuable in cases in which a portion of the DNA in question is toxic or incompatible with plasmid replication when introduced into E. coli. If this thermostable DNA polymerase preparation simultaneously conferred increased fidelity to the PCR amplification, the resulting large products would be much more accurate, active and/or valuable in research and applications, especially in situations involving expression of the amplifed sequence. If the thermostable DNA polymerase preparation allowed, in addition, more highly concentrated yields of pure product, this would enhance the method of PCR to the point where it could be used more effectively to replace plasmid replication as a means to produce desired DNA fragments in quantity.

### SUMMARY OF THE INVENTION

Among the several objects of the invention, therefore, may be noted the provision of a DNA polymerase which can survive meaningful repeated exposure to temperatures of 99°C; the provision of a highly thermostable DNA polymerase which exhibits greater fidelity than Thermus aquaticus DNA polymerase when utilized at standard Thermus aquaticus DNA polymerase extension reaction temperatures; the provision of such a DNA polymerase which is useful for PCR amplification techniques from DNA templates and from single colonies of E. coli, single-stranded (linear) amplification of DNA, nucleic acid sequencing, DNA restriction digest filling, DNA labelling, in vivo footprinting, and primer-directed mutagenesis. Further objects of the invention include the provision of recombinant DNA sequences, vectors and host cells which provide for the expression of such DNA polymerase.

Additional objects include the provision of a formulation of DNA polymerase capable of efficiently catalyzing primer extension products of greater length than permitted by conventional formulations, including lengths up to at least 35 kilobases, that reduces the mutagencity generated by the PCR process, particularly in comparison with prior art DNA polymerase and for any target lengths, that maximizes the yield of PCR fragments and, concomitantly, enhances the intensity and sharpness of PCR product bands, without significant sacrifice in flexibility, specificity, and efficiency; and the provision of an improved process for amplification by PCR which can be utilized to reliably synthesize nucleic acid sequences of greater length and which can effectively utilize PCR products as primers.

Therefore, according to a first aspect of the present invention there is provided a recombinant DNA sequence encoding a DNA polymerase having an amino acid sequence selected from the group consisting of
a) the sequence identified in SEQ ID NO:6, and
b) a variant of SEQ ID NO:6 that encodes a DNA polymerase having the enzymatic activity and thermostability of the DNA polymerase of SEQ ID NO:6.

In a further aspect of the invention there is provided a recombinant DNA sequence encoding a DNA polymerase selected from the group consisting of
a) the amino acid sequence 280-831 of the DNA polymerase of Thermus flavus the polymerase being encoded by the sequence in GenBank Accession No. X66105, and
b) a variant of said amino acid sequence, the variant having the enzymatic activity and thermostability of said amino acid sequence.

A further aspect of the invention comprises a DNA polymerase having an amino acid sequence selected from the group consisting of
a) the sequence identified in SEQ ID NO:6, and
b) a variant of SEQ ID NO:6 that encodes a DNA polymerase having the enzymatic activity and thermostability of the DNA polymerase of SEQ ID NO:6.

An even further aspect of the invention comprises A DNA polymerase having an amino acid sequence selected from the group consisting of
a) the amino acid sequence 280-831 of the DNA polymerase of Thermus flavus, the polymerase being encoded by the sequence in GenBank Accession No. X66105, and
b) a variant of said amino acid sequence, the variant having the enzymatic activity and thermostability of said amino acid sequence.

In a further embodiment, the present invention is directed to a novel formulation of thermostable DNA polymerases, comprising a majority component comprised of at least one thermostable DNA polymerase lacking 3-exonuclease activity and a minority component comprised of at least one thermostable DNA polymerase exhibiting 3'-(editing)exonuclease activity.

The present invention is also directed to a formulation of DNA polymerase which includes at least one DNA polymerase which in wild-type form exhibits 3'-exonuclease activity and which is capable of catalyzing a temperature cycle type polymerase chain reaction, wherein the 3'-exonuclease activity of said at least one DNA polymerase has been reduced to between about 0.2% and about 7%, of the 3'-exonuclease activity of the at least one DNA polymerase in its wild-type form.

In another aspect, a formulation of DNA polymerase comprising E1 and E2 is provided wherein E1 is one or more DNA polymerases which lack any significant 3'-exonuclease activity and E2 is one or more DNA polymerases which exhibit significant 3'-exonuclease activity and wherein the ratio of the amounts of E1 to E2 is at least about 4:1 by DNA polymerase units or at least about 4:1 by amounts of protein by weight.

The invention is further directed to an improvement in a process for amplification of nucleic acid sequences by PCR of the repeating cycle type wherein two complementary strands, for each different specific sequence being amplified, are treated with a single, a pair, or a mixture of pairs of oligonucleotide primers, and a DNA polymerase or a mixture of DNA polymerases is used to catalyze synthesis of an extension product of each primer which is complementary to each nucleic acid strand under effective conditions for said synthesis, and wherein said primers are selected so as to be sufficiently complementary to different strands of each specific sequence to hybridize therewith such that the extension product synthesized from one primer, when it is separated from its complement, can serve as a template for synthesis of the complementary strand of the extension product by extending the same or another included primer, the primer extension products are separated from the templates on which they were synthesized to produce single-stranded molecules, and the single-stranded molecules thus generated are treated with the primers and a DNA polymerase or a mixture of DNA polymerases is used to catalyze synthesis of an extension product of each primer under effective conditions for the synthesis of a primer extension product. In one aspect the improvement comprises formulating DNA polymerase of the types described above and catalysing primer extension during the PCR process with said formulation of DNA polymerase, thus extending the applicable size range for efficient PCR amplification. Alternatively, the improvement comprises formulating a DNA polymerase comprising at least one DNA polymerase, which in wild-type form exhibits 3'-exonuclease activity and which is capable of catalyzing a temperature cycle type polymerase chain reaction, wherein said 3'-exonuclease activity of said at least one DNA polymerase has been reduced to no greater than about 7% of the 3'-exonuclease activity of said at least one DNA polymerase in its wild-type form, but not eliminated, and catalyzing primer extension with said formulation of DNA polymerase.

DNA polymerases such as those discussed in this application are commonly composed of up to three identifiable and separable domains of enzymatic activity, in the physical order from N-terminal to C-terminal, of 5'-exonuclease, 3'exonuclease, DNA polymerase. Taq DNA polymerase has never had a 3'exonuclease, but the invention in the first part is directed to a deletion of its 5'-exonuclease. Other DNA polymerases mentioned, such as Pfu DNA polymerase, do not have the 5'-exonuclease, but their 3'-exonuclease function is central to the aspect of the invention directed to mixtures of DNA polymerases E1 (lacking significant 3'-exonuclease) and E2 (having 3'-exonuclease). In these mixtures, the presence of 5'-exonuclease in either E1 or E2 has not been shown to be essential to the primary advantages of the present invention.

Other objects and features will be in part apparent and in part pointed out hereinafter.

### SUMMARY OF ABBREVIATIONS

The listed abbreviations, as used herein, are defined as follows:
Abbreviations:
- bp =: base pairs
- kb =: kilobase; 1000 base pairs
- nt =: nucleotides
- BME =: beta-mercaptoethanol
- PPᵢ =: sodium pyrophosphate
- Pfu =: Pyrococcus furiosus
- Taq =: Thermus aquaticus
- Tfl =: Thermus flavus
- Tli =: Thermococcus literalis

Klentaq-nnn = N-terminally deleted Thermus aquaticus DNA polymerase that starts with codon nnn+1, although that start codon and the next codon may not match the WT sequence because of alterations to the DNA sequence to produce a convenient restriction site.
WT = wild-type (full length) or deletion of only 3 aa
aa = amino acid(s)
ST = Stoffel fragment, an N-terminal deletion of Thermus aquaticus DNA polymerase that could be named Klentaq-288. -LA = Long and Accurate; an unbalanced mixture of two DNA polymerases, at least one lacking significant 3'-exonuclease activity and at least one exhibiting significant 3'-exonuclease activity.
PCR = (noun) 1. The Polymerase Chain Reaction

2. One such reaction/amplification experiment. 3.(verb) To amplify via the polymerase chain reaction.
ul = microliter(s)
ATCC = American Type Culture Collection
Megaprimer = double-stranded DNA PCR product used as primer in a subsequent PCR stage of a multi-step procedure.
Deep Vent = DNA polymerase from Pyrococcus species GB-D; purified enzyme is available from New England Biolabs.
Deep Vent exo- = mutant form of Deep Vent DNA polymerase lacking 3'(editing)-exonuclease.
Vent = DNA polymerase from Thermococcus litoralis; purified enzyme is available from New England Biolabs.
Vent exo- = mutant form of Vent DNA polymerase lacking 3'(editing)-exonuclease.

Pfu = DNA polymerase from Pyrococcus furiosus lacking 3'(editing)-exonuclease; purified enzyme is available from Stratagene Cloning Systems, Inc.

Pfu exo- = mutant form of Pfu DNA polymerase ; purified enzyme is available from Stratagene Cloning Systems, Inc.

Sequenase = A chemically modified or a mutated form of phage T7 or T3 DNA polymerase wherein the modification or mutation eliminates the 3'-exonuclease activity.

### BRIEF DESCRIPTION OF THE DRAWINGS

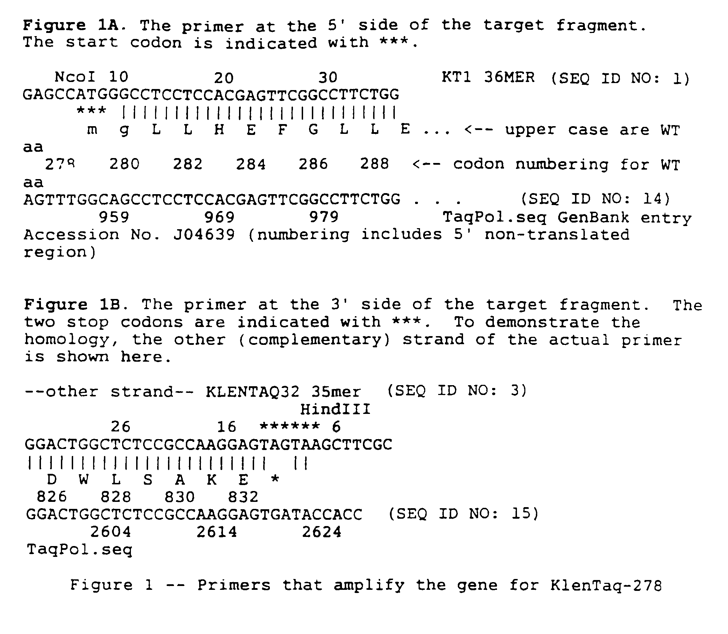

Figure 1 depicts the nucleotide sequence of primers that can be used for amplification of the gene for a preferred embodiment of the DNA polymerase of this invention. The bulk of the DNA sequence for the gene (between the primers) and the resultant amino acid sequence of the enzyme, is defined by the indicated GenBank entry.

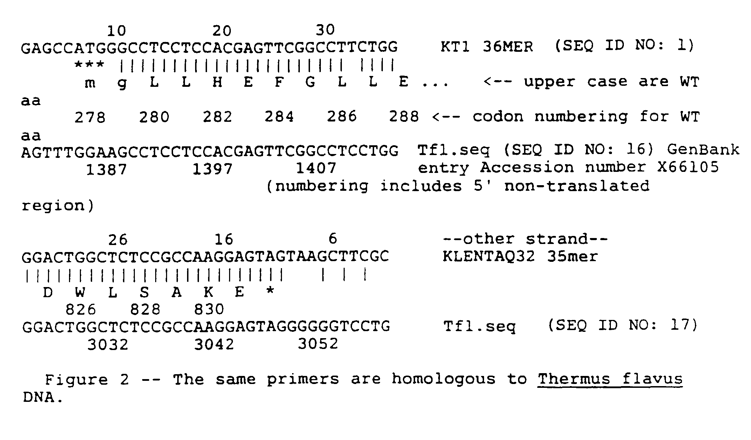

Figure 2 depicts the nucleotide sequence of the same primers as in Figure 1, and shows that these same primers can be used for amplification of the analogous gene from Thermus flavus.

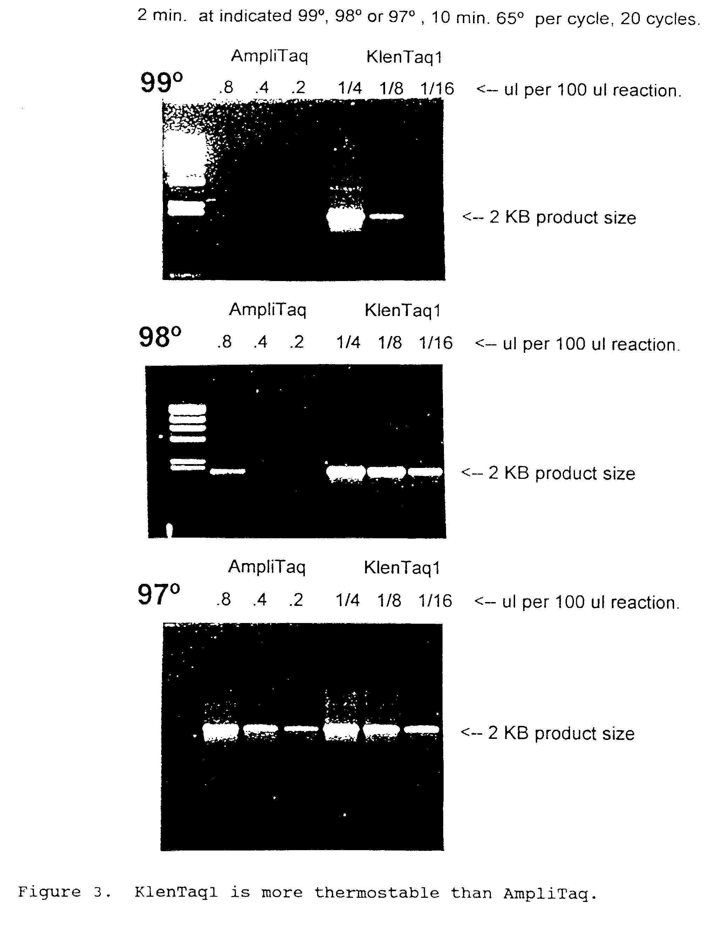

Figure 3 is a photograph of an agarose gel depicting a PCR amplification reaction conducted using the prior art enzyme Thermus aquaticus DNA polymerase (AmpliTaq; AT) and a preferred embodiment of the DNA polymerase of this invention, tested with differing peak denaturation temperatures lasting a full 2 min each for 20 cycles. Full activity at 98° and partial but useful activity at 99° is exhibited by the preferred embodiment of this invention, whilst AT is unable to withstand these temperatures. Figure 3 demonstrates that the enzyme of the present invention is more thermostable than AT in a practical test -- PCR amplification.

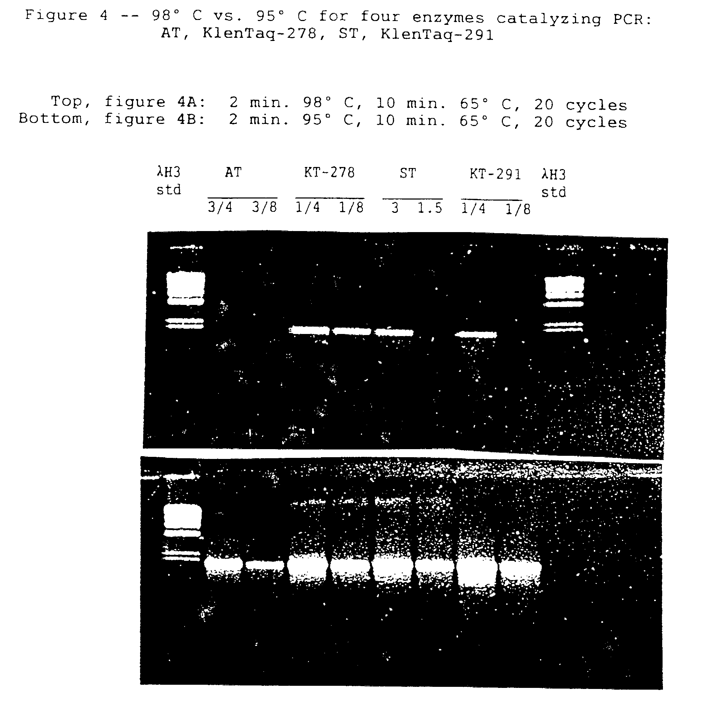

Figure 4 is a photograph of an agarose gel depicting a PCR amplification reaction conducted using 4 enzymes: the prior art enzyme Thermus aquaticus DNA polymerase (AmpliTaq; AT); the DNA polymerase of this invention (KlenTaq-278); the prior art enzyme AmpliTaq Stoffel Fragment (ST); and KlenTaq-291. All were tested with PCR denaturation steps carried out at 95°C (control standard temperature), and at 98°C. All were tested at two levels of enzyme, the lower level being as close as practicable to the minimum necessary to support the reaction at the control temperature.

Note that both KlenTaq-291 and ST behave identically, losing most, but not all, of their activity when used at 98° C, yet KlenTaq-278 is at least twice as able to withstand use of the higher denaturation temperature. AT is seen to be drastically reduced in effectiveness by exposure to 98° C. The behaviour of these enzymes is reproducible except for ST, which is at its best in the presented experiment, but performs more poorly when used in the amounts recommended by the manufacturer.

Figure 5 is a photograph of an agarose gel analysis of the products of colony PCR carried out at the standard peak denaturation temperature of 95° C compared to the newly available temperature of 98° C allowed by the enzyme of the present invention. Figure 5 demonstrates an application advantage of the use of the newly available peak denaturation temperature.

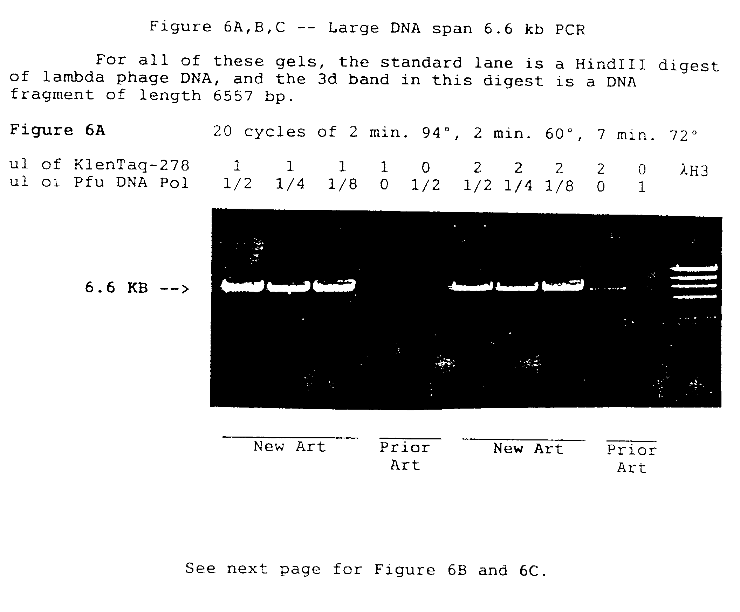

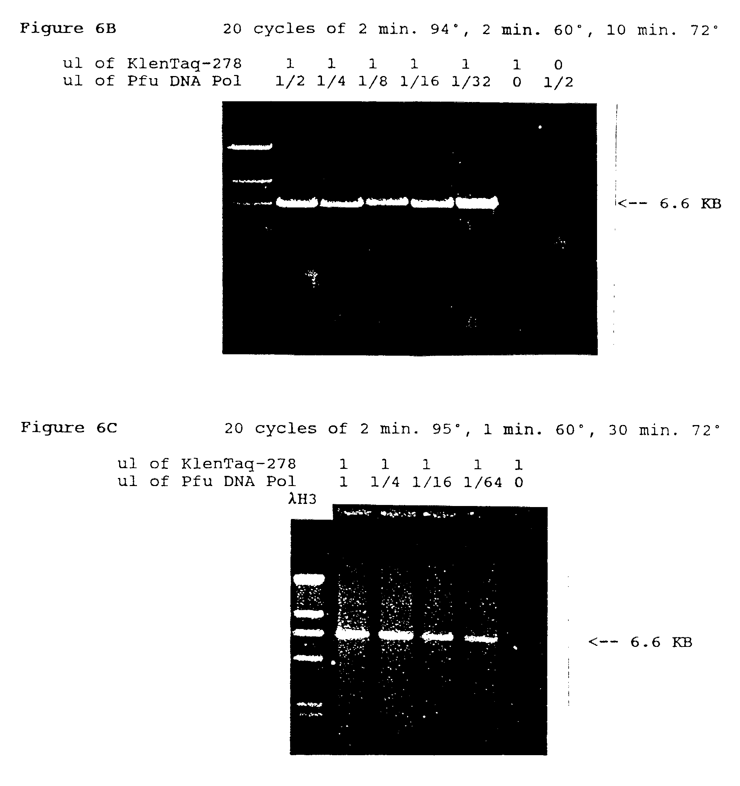

Figure 6(A-C) is three photographs, each of an agarose gel on which was loaded a portion of a test PCR experiment. Figure 6 demonstrates the large increase in efficiency of large DNA span PCR achieved by variations of a preferred embodiment of the enzyme formulation of the invention. Although KlenTaq-278 or Pfu DNA polymerase, alone, are shown to catalyze a low level of 6.6 kb PCR product formation, various combinations of the two are seen to be much more efficient. Lower and lower amounts of Pfu in combination with Klentaq-278 are seen to be effective, down to the minimum presented, 1/640. Of those shown, only a combination of Klentaq-278 and Pfu can catalyze efficient amplification of 6.6 kb. Per 100 ul, the indicated level of each enzyme (see Methods, example 8, for unit concentrations) was used to catalyze PCR reactions templated with 19 ng λplac5 DNA and primers MBL and MBR. 20 cycles of 94° 2 min., 60° 2 min., 72° 10 min.

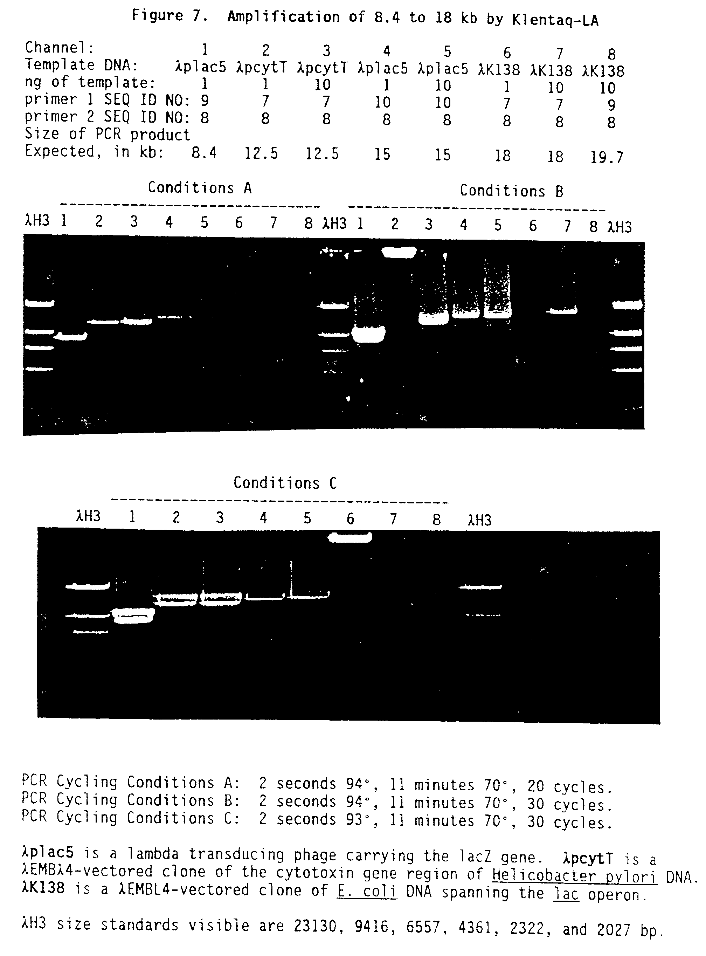

Figure 7 is a photograph of an agarose gel on which were analyzed the products of PCR experiments to test the performance of an embodiment of the invention in catalyzing the amplification of fragments even longer than 6.6 kb. Figure 7 demonstrates the ability to amplify 8.4 kb, 12.5 kb, 15 kb, and 18 kb with high efficiency and large yield, utiliziing the 1/640 ratio embodiment of the enzyme formulation of the invention. Target product size is indicated above each lane as kb:. Level of template per 100 ul is indicated as ng λ:. 20 or 30 cycles of PCR were each 2 sec. 94°, 11 min. 70°. These early amplifications were non-optimal in several respects compared to the current optimal procedure (see Methods, example 8): thick-walled tubes were employed instead of thin, catalysis was by 1 ul KlentaqLA-64 (63:1::Klentaq-278:Pfu) instead of KlentaqLA-16, the 27mer primers were used (see Table 1) instead of longer primers, the extension/annealling temperature was 70° instead of 68°, and the Omnigene thermal cycler was used.

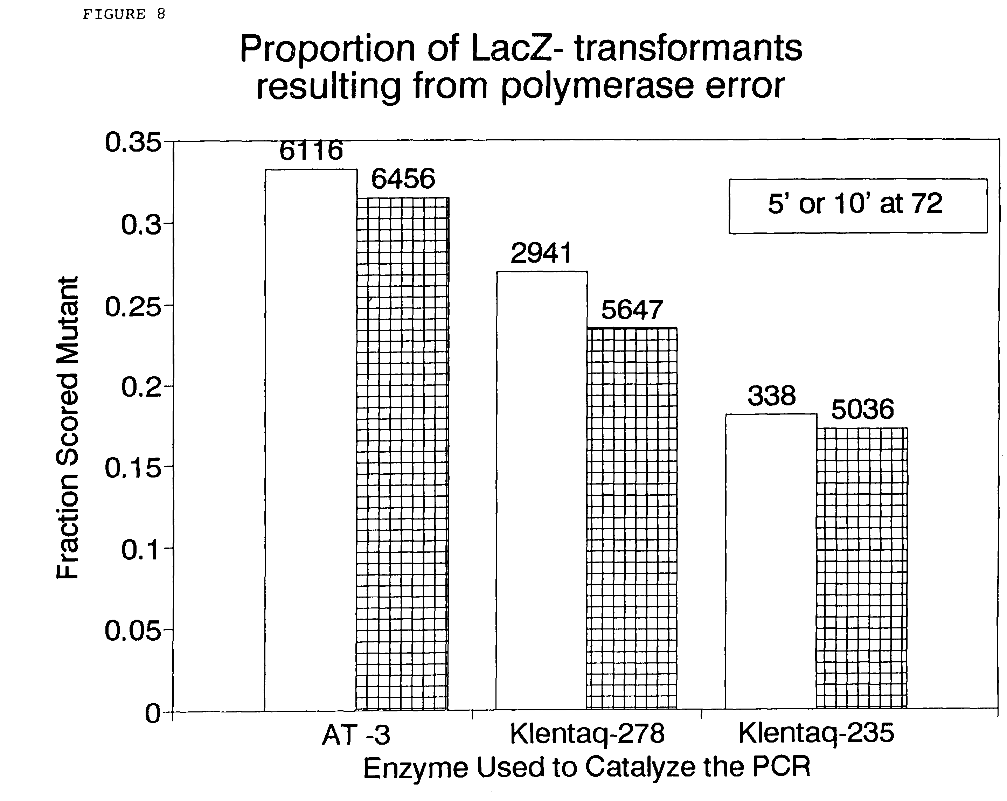

Figure 8 is a bar graph enumerating the differences in the number of mutations introduced into a PCR product, the lacZ gene, by the near full-length prior art -3 deletion of Thermus aquaticus DNA polymerase, compared to the number of mutations introduced by Klentaq-278.

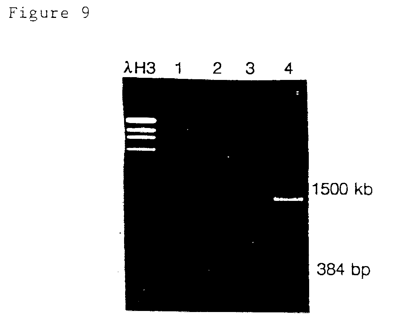

Figure 9 is a photograph of an agarose gel depicting a PCR amplification attempted using a 384 bp megaprimer (double-stranded PCR product) paired with a 43-mer oligonucleotide primer BtV5. Per 100 ul of reaction volume, the following enzymes (see Ex. 8, Methods for unit concentrations) were used to catalyze amplifications: lane 1, 1 ul Pfu DNA polymerase; lane 2, 1/16 ul Pfu; lane 3, 1 ul Klentaq-278; lane 4, both enzymes together (1 ul Klentaq-278 + 1/16 ul Pfu). The 384 bp band near the bottom of the gel is the megaprimer, which was originally amplified using Klentaq-278. λH3 = lambda DNA digested with HindIII. The only successful amplification resulted from the combination of the two enzymes (lane 4). Vent DNA polymerase could substitute for Pfu with the same result (data not shown).

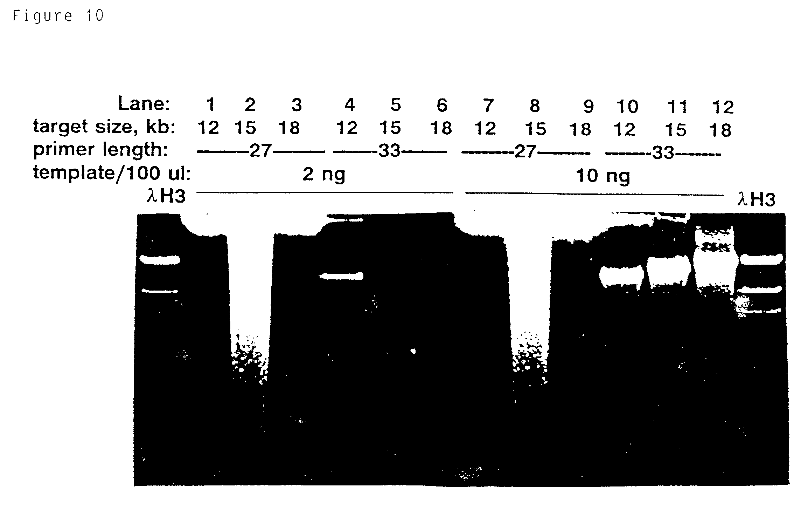

Figure 10 is a photograph of an agarose gel demonstrating that 33mers are better than 27mers. Per 100 ul of reaction volume, 2 ng (lanes 1-6) or 10 ng (lanes 7-12) of lambda transducing phage template were amplified using 27mer primers (lanes 1-3, 7-9) or 33mer primers (lanes 4-6. 10-12). Besides being longer, the 33mer lambda primer sequences were situated 100 bp to the left of primer MBL and 200 bp to the right of primer MBR on the lambda genome. KlentaqLA-16 in the amounts of 1.2, 1.4, and 1.6 ul was used to catalyze the amplifications of 12.5, 15, and 18 kb, respectively. 15 ul aliquots (equivalent to 0.3 or 1.5 ng of λ template) were analyzed by 0.8% agarose electrophoresis.

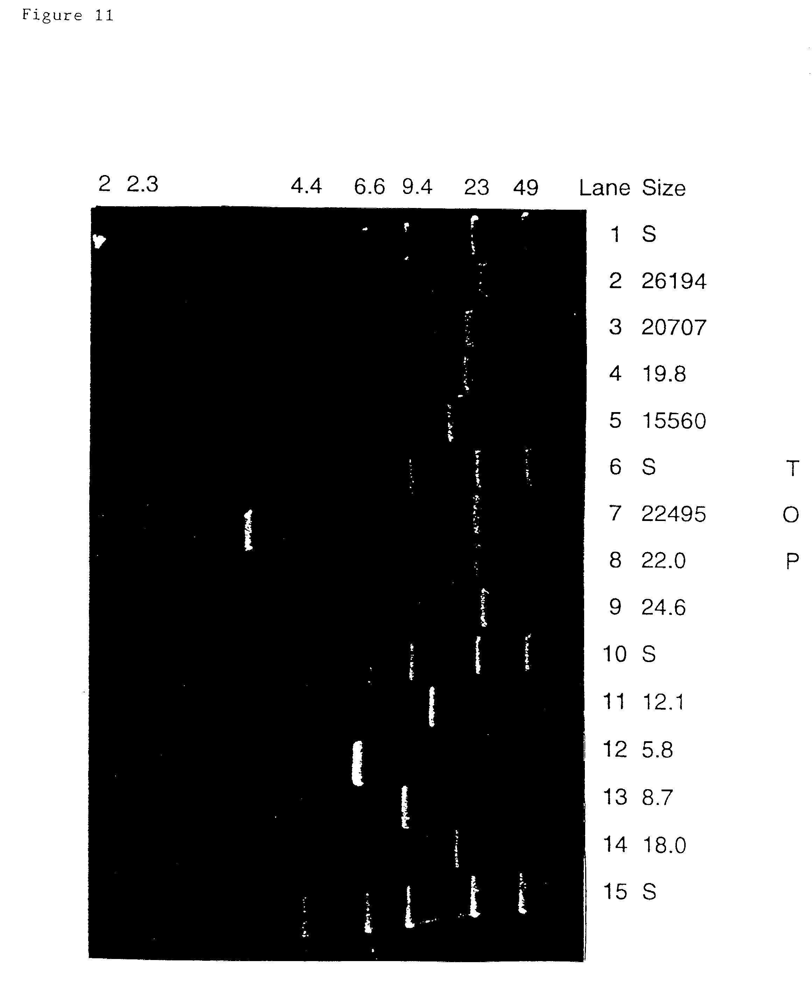

Figure 11 is a photograph of an agarose gel showing a CHEF pulse-field analysis (ref. 11, 4 sec. switching time) of large PCR products amplified by KlentaqLA-16 (1.2 ul) under conditions which were suboptimal with respect to pH (unmodified PC2 buffer was used) and thermal cycler (Omnigene). Starting template (see Table 1) was at 0.1 ng/ul and the time at 68° in each cycle was 21 min. for products over 20 kb, 13 min. for lanes 4 & 5, and 11 min. for lanes 11-14. The volumes of PCR reaction product loaded were adjusted to result in approximately equal intensity; in ul: 12,12,4,2; 10,10,10; 2,2,4,1. The standard size lanes (S) show full-length λplac5 DNA (48645 bp) mixed with a HindIII digest of λ DNA. As for Table 1, the sizes in 5 figures are in base pairs, as predicted from the primer positions on the sequence of λplac5 DNA, and sizes with decimal points are in kb, as determined from this gel.

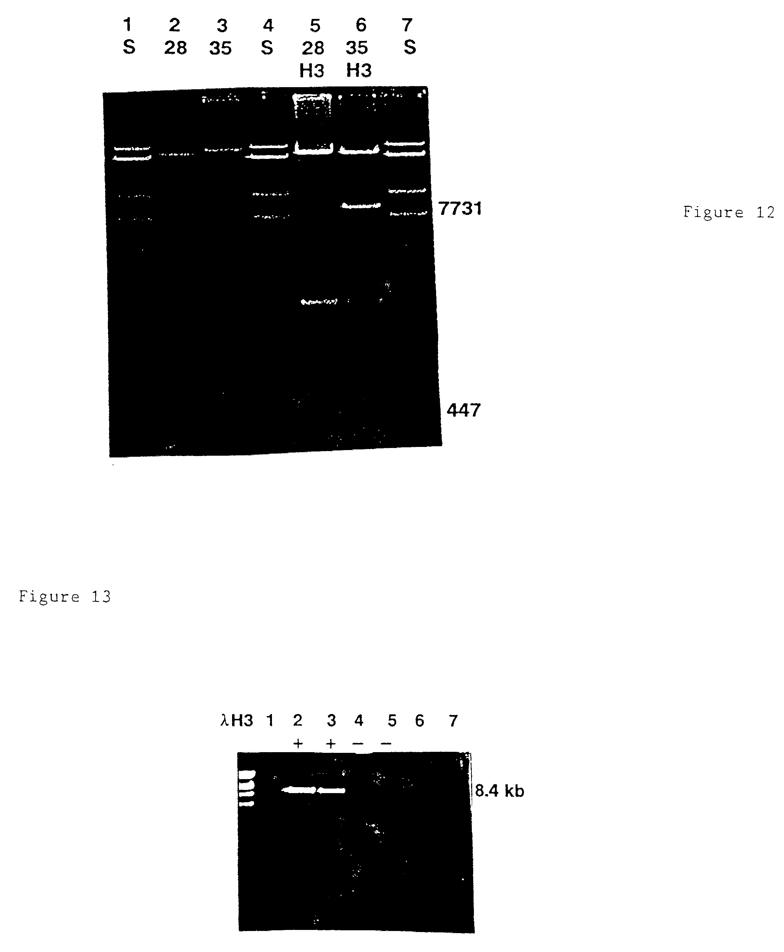

Figure 12 is a photograph of an agarose gel depicting 28 kb and 35 kb products without (lanes 2,3) and with (lanes 5,6) digestion by restriction enzyme HindIII. Before HindIII digestion, the 28 kb product was amplified with 21 min. extension time per cycle, and the 35 kb product was cycled with 24 min. extension times, both in the RoboCycler at optimum pH (see Ex. 8, Methods). Lanes S (1,4,7) contain markers of undigested λplac5 and HindIII-digested λplac5 DNA.

Figure 13 is a photograph of an agarose gel showing the results of a Pfu exo⁻ mutant test. PCR amplification of 8.4 kb by 30 units (0.7 ug) of Klentaq-278 alone (lanes 1,7) and in combination with a very small admixture (1/16 ul or 1/64 ul, equivalent to 1/6 or 1/25 unit) of archaebacterial Pfu wild type exo⁺ DNA polymerase (+; lanes 2,3) or a mutant thereof lacking the 3'-exonuclease activity (-; lanes 4,5). Lane 6 is the result if 1 ul (2.5 units) of solely Pfu DNA polymerase (wt, exo⁺) is employed.

### DESCRIPTION OF THE PREFERRED EMBODIMENT

Referring now to Figure 1, the primers and logic for amplification by PCR of the recombinant DNA sequence encoding a preferred embodiment of the DNA polymerase of the invention (referred to herein as Klentaq-278), are set forth. As depicted in Figure 1, an initiator methionine and a glycine residue occupy the first two N-terminal positions of Klentaq-278, previously occupied by residues 279 and 280 of WT Thermus aquaticus DNA polymerase, followed by the amino acid sequence of wild-type Thermus aquaticus DNA polymerase, beginning with the amino acid residue at position 281 as described by Lawyer et al. The codons encoding amino acid residues 1 through 280 of Thermus aquaticus DNA polymerase are therefore deleted, and the amino acids 1 thru 280 are not present in the resulting gene product. Another preferred embodiment of the DNA polymerase of the invention is depicted in Figure 2. In this embodiment, the same deletion mutation described above is made to the highly analogous enzyme Thermus flavus DNA polymerase.

It will be appreciated that minor variations incorporated into the DNA encoding for, or the amino acid sequence as described herein, which retain substantially the amino acid sequence as set forth above, and which do not significantly affect the thermostability of the polymerase are included within the scope of the invention.

Surprisingly, the mutant DNA polymerase Klentaq-278 exhibits thermostability at temperatures above those reported for any previous variant of Thermus aquaticus DNA polymerase and has demonstrated a fidelity in final PCR products which is greater than that of WT Thermus aquaticus DNA polymerase, when both are utilized at the 72° C temperatures recommended for DNA synthesis. Further, since Klentaq-278 does not have the 5'-exonuclease activity associated with Thermus aquaticus DNA polymerase (removed as a consequence of the N-terminal deletion), it is significantly superior to wild-type Thermus aquaticus DNA polymerase for DNA sequencing. Mutagenesis results, and mismatched primer testing, suggest that Klentaq-278 is less processive and is less likely to extend a mispaired base than wild-type Thermus aquaticus DNA polymerase.

Thermostability tests with Klentaq-278, Stoffel Fragment (ST, alternative designation, Klentaq-288) and Klentaq-291 have been carried out. The test used involves 20 PCR cycles with a full 2 minutes each at the peak test temperature, such as 97° C, 98° C, or 99° C, and the intensity of the resulting amplified bands is compared to 2 minutes at 97° C, or at a lower control denaturation temperature, such as 95° C (at which all of these variants are stable). These data indicate that ST and Klentaq-291 behave similarly, having thermolability at 98° C that is similar to each other yet distinct from Klentaq-278, which exhibits little detectable thermolability at 98° C in these tests. These data suggest that the number of N-terminal amino acids is important to the enhanced thermostability exhibited by the DNA polymerase of the invention. Evidently deletions ST and Klentaq-291 are in a class which has removed too many amino acids (10 more and 13 more) for the optimum stability demonstrated by the invention Klentaq-278.

The DNA polymerase from the bacterium sometimes designated Thermus flavus (and sometimes Thermus aquaticus -- see ATCC catalog) is highly homologous to the WT Thermus aquaticus DNA polymerase. In the region of the deletions being discussed here, the enzymes and genes are exactly homologous, and it is believed that the differences between the pair ST, Klentaq-291 and the superior Klentaq-278 would remain if the analogous deletions were constructed. Indeed, the primers in Figure 2 could be used on Thermus flavus DNA to construct KlenTfl-277 in exactly the manner described here for the construction and isolation of Klentaq-278. The Thermus flavus DNA polymerase -277 enzyme and variations thereof which exhibit similar thermostability are therefore also within the scope of this invention.

The invention also features a vector which includes a recombinant DNA sequence encoding a DNA polymerase comprising the amino acid sequence of Thermus aquaticus or Thermus flavus DNA polymerase, except that it adds a methionine and glycine residue at the N-terminal and excludes the N-terminal 280 amino acids of wild-type Thermus aquaticus DNA polymerase (see Lawyer et al., supra).

In preferred embodiments, the vector is that nucleic acid present as plasmid pWB254b (SEQ ID NO:5) deposited as ATCC No. 69244 or a host cell containing such a vector.

In a related aspect, the invention features a purified DNA polymerase having an amino acid sequence as discussed above. As used herein, "purified" means that the polymerase of the invention is isolated from a majority of host cell proteins normally associated with it. Preferably, the polymerase is at least 10% (w/w) of the protein of a preparation. Even more preferably, it is provided as a homogeneous preparation, e.g., a homogeneous solution.

In general, the recombinant DNA sequence of the present invention is amplified from a Thermus aquaticus genomic DNA or from a clone of the portion of the Thermus aquaticus DNA polymerase gene which is larger than the desired span, using the polymerase chain reaction (PCR, Saiki et al., Science 239:487, 1988), employing primers such as those in Figure 2 into which appropriate restriction sites have been incorporated for subsequent digestion.

The recombinant DNA sequence is then cloned into an expression vector using procedures well known to those in this art. Specific nucleotide sequences in the vector are cleaved by site-specific restriction enzymes such as NcoI and HindIII. Then, after optional alkaline phosphatase treatment of the vector, the vector and target fragment are ligated together with the resulting insertion of the target codons in place adjacent to desired control and expression sequences. The particular vector employed will depend in part on the type of host cell chosen for use in gene expression. Typically, a host-compatible plasmid will be used containing genes for markers such as ampicillin or tetracycline resistance, and also containing suitable promoter and terminator sequences.

In a preferred procedure, the recombinant DNA expression sequence of the present invention is cloned into plasmid pWB253 (expresses KlenTaq-235 deposited as ATCC No. 68431) or pWB250 (expresses luciferase/NPTII fusion), the backbone of which is pTAC2 (J.Majors, Washington University), a pBR322 derivative. The specific sequence of the resulting plasmid, designated pWB254b is SEQ ID NO: 5.

Bacteria, e.g., various strains of E. coli, and yeast, e.g., Baker's yeast, are most frequently used as host cells for expression of DNA polymerase, although techniques for using more complex cells are known. See, e.g., procedures for using plant cells described by Depicker, A., et al., J. Mol. Appl. Gen. (1982) 1:561. E. coli host strain X7029, wild-type F⁻, having deletion X74 covering the lac operon is utilized in a preferred embodiment of the present invention.

A host cell is transformed using a protocol designed specifically for the particular host cell. For E. coli, a calcium treatment, Cohen, S.N., Proc. Natl. Acad. Sci. 69:2110 (1972), produces the transformation. Alternatively and more efficiently, electroporation of salt-free E. coli is performed after the method of Dower et al. (1988), Nucleic Acids Research **16:**6127-6145. After transformation, the transformed hosts are selected from other bacteria based on characteristics acquired from the expression vector, such as ampicillin resistance, and then the transformed colonies of bacteria are further screened for the ability to give rise to high levels of isopropylthiogalactoside (IPTG)-induced thermostable DNA polymerase activity. Colonies of transformed E. coli are then grown in large quantity and expression of Klentaq-278 DNA polymerase is induced for isolation and purification.

Although a variety of purification techniques are known, all involve the steps of disruption of the E. coli cells, inactivation and removal of native proteins and precipitation of nucleic acids. The DNA polymerase is separated by taking advantage of such characteristics as its weight (centrifugation), size (dialysis, gel-filtration chromatography), or charge (ion-exchange chromatography). Generally, combinations of these techniques are employed together in the purification process. In a preferred process for purifying Klentaq-278 the E. coli cells are weakened using lysozyme and the cells are lysed and nearly all native proteins are denatured by heating the cell suspension rapidly to 80°C and incubating at 80-81° C for 20 minutes. The suspension is then cooled and centrifuged to precipitate the denatured proteins. The supernatant (containing Klentaq-278) then undergoes a high-salt polyethylene-imine treatment to precipitate nucleic acids. Centrifugation of the extract removes the nucleic acids. Chromatography, preferably on a heparin-agarose column, results in nearly pure enzyme. More detail of the isolation is set forth below in Example 3.

The novel DNA polymerase of the present invention may be used in any process for which such an enzyme may be advantageously employed. In particular, this enzyme is useful for PCR amplification techniques, nucleic acid sequencing, cycle sequencing, DNA restriction digest labelling and blunting, DNA labelling, in vivo DNA footprinting, and primer-directed mutagenesis.

### Amplification

Polymerase chain reaction (PCR) is a method for rapidly amplifying specific segments of DNA, in geometric progression, up to a million fold or more. See, e.g., Mullis U.S. Patent No. 4,683,202, which is incorporated herein by reference. The technique relies on repeated cycles of DNA polymerase-catalyzed extension from a pair of primers with homology to the 5' end and to the complement of the 3' end of the DNA segment to be amplified. A key step in the process is the heat denaturing of the DNA primer extension products from their templates to permit another round of amplification. The operable temperature range for the denaturing step generally ranges from about 93°C to about 95°C, which irreversibly denatures most DNA polymerases, necessitating the addition of more polymerase after each denaturation cycle. However, no additional DNA polymerase needs to be added if thermostable DNA polymerases such as Thermus aquaticus DNA polymerase are used, since they are able to retain their activity at temperatures which denature double-stranded nucleic acids. As described in Example 4, below, Klentaq-278 has demonstrated the ability to survive meaningful repeated exposure to temperatures of 99°C, higher than for any previously known DNA polymerase.

Klentaq-278 has also been demonstrated to have a higher fidelity than wild-type Thermus aquaticus DNA polymerase at 72°C, the recommended synthesis temperature. The data for this has been gathered by a method involving the PCR amplification of a lacZ DNA gene flanked by two selectable markers [Barnes, W.M. (1992) Gene 112, 29-25]. Representative data comparing the preferred embodiment of this invention Klentaq-278 to AT and another analogous N-terminal deletion, Klentaq-235, is shown in Figure 8, which demonstrates that different N-terminal deletions reproducibly exhibit differing fidelities as measured in the final PCR product.

Similar fidelity data for the enzyme ST is not available, since it is difficult for the commercial preparation of this enzyme to catalyze PCR of the long test fragment (4.8 kb) used for this assay. It is not yet known whether the difficulty with ST for these experiments is caused merely by formulation (its concentration is less, such that 10-15 times more volume is necessary for a 2 kb PCR amplification, and for these deletions more enzyme is needed for longer target DNAs), or whether ST may be intrinsically unable to catalyze such a long-target PCR amplification.

### DNA Sequencing

Particular DNA sequences may be elucidated by the Sanger Method (Sanger, F., Nicklen, S. and Coulson, A.R., DNA sequencing with chain-terminating inhibitors, Proc.Nat.Acad.Sci.USA, 74 (1977) 5463-5467), using dideoxy analogs. DNA polymerases are used in these methods to catalyze the extension of the nucleic acid chains. However, in its natural form, Thermus aquaticus DNA polymerase (like many other polymerases) includes a domain for 5'-exonuclease activity. This associated exonuclease activity can, under certain conditions including the presence of a slight excess of enzyme or if excess incubation time is employed, remove 1 to 3 nucleotides from the 5' end of the sequencing primer, causing each band in an alpha-labelled sequencing gel to appear more or less as a multiplet. If the label of the sequencing gel is 5', the exonuclease would not be able to cause multiplets, but it would instead reduce the signal. As a result of the deletion of the N-terminal 280 amino acid residues of Thermus aquaticus DNA polymerase, Klentaq-278 has no exonuclease activity and it avoids the sequencing hazards caused by 5'-exonuclease activity.

Klentaq-278 can be used effectively in thermostable DNA polymerase DNA sequencing. There are basically two types of dideoxy DNA sequencing that Klentaq-278 is good for -- **original dideoxy** (Sanger et al. supra; Innis et al., Proc. Natl. Acad. Sci. USA 85:9436, 1988) and **cycle sequencing.**

Innis et al. describe a good procedure for dideoxy sequencing, but when the WT Thermus aquaticus DNA polymerase is used this procedure is prone to doubled or tripled bands on the sequencing gel, as demonstrated in the patent application no. 07/594,637 which should be available to the examiner and which is incorporated herein by reference. Klentaq-278 is as effective in curing this problem as the subject of that patent application, Klentaq-235, a.k.a. DeltaTaq.

The procedure recommended for orignal-type (non-cycled, Innis et al.) dideoxy sequencing with Klentaq-278 is that in the USB Taquence 2.0 dideoxy sequencing kit, the title page of which is appended to this application (Appendix 1) and the article is wholly incorporated by reference.

The procedure recommended for cycle sequencing is that in the USB Cycle Sequencing Kit. The title page of this procedure is appended to this application (Appendix 2) and the article is wholly incorporated by reference.

### Other Uses

Klentaq-278 has also been used successfully for primer-directed mutagenesis, in vivo footprinting, DNA labelling and for preparation of non-sticky Lambda DNA fragment size standards. These procedures are discussed below.

Klentaq-278, especially in the formulation Klentaq-LA (discussed below), can be used to extend site-specific mutagenic primer(s) which are annealled to single-stranded templates. It substitutes for Klenow enzyme (the large fragment of E. coli DNA polymerase I) and T7 DNA polymerase in this process, showing more primer selectivity at 60-65°C than Klenow enzyme at 37°C, and working to completion or sufficient incorporation in 12 mins., as compared to the one hour or more required for Klenow enzyme.

Klentaq-278 has also been shown to be useful (and superior to wild-type Thermus aquaticus DNA polymerase) for the post-PCR labelling steps with the third (second nested) primer in ligase-mediated, PCR-assisted, in vivo footprinting. I am indebted to I. Ghattas, Washington University, St. Louis, MO for this information. These studies are similar to those of Garritty & Wold (Garrity, P.A., and Wold, B.J. (1992) Effects of different DNA polymerases in ligation-mediated PCR: enhanced genomic sequencing and in vivo footprinting. Proc Natl Acad Sci U S A 89, 1021-1025)

Klentaq-278 is also useful for DNA labelling. For random primers, a length of at least 9 nt is recommended, and preferably the reaction is warmed slowly (over 20-30 mins.) from 37 to 65°C. Most preferably, a programmable heat block, using procedures well-known to those in this art, is utilized for the DNA labelling.

Another use of Klentaq-278 is for the preparation of Lambda DNA restriction digests that do not have the sticky ends partially stuck together. As a result of including Klentaq-278 and the four DNA dNTPs in with a HindIII digest performed at 55°C, bands 1 and 4 are not partially attached to each other.

### Deposit

Strain pWB254b/X7029 was deposited with the American Type Culture Collection, Maryland, on February 18, 1993 and assigned the number ATCC 69244. Applicant acknowledges his responsibility to replace this culture should it die before the end of the term of a patent issued hereon, 5 years after the last request for a culture, or 30 years, whichever is the longer, and its responsibility to notify the depository of the issuance of such a patent, at which time the deposits will be made available to the public. Until that time the deposits will be made available to the Commissioner of Patents under the terms of 37 C.F.R. Section 1-14 nad 35 U.S.C. §112.

In a further aspect of the invention a target length limitation to PCR amplification of DNA has been identified and addressed. Concomitantly, the base pair fidelity, the ability to use PCR products as primers, and the maximum yield of target fragment were increased. These improvements were achieved by the combination of a DNA polymerase lacking significant 3'-exonuclease activity, preferably, Klentaq-278, with a low level of a DNA polymerase exhibiting significant 3'-exonuclease activity (for example, Pfu, Vent, or Deep Vent). Surprisingly, target fragments of at least 35 kb can be amplified to high yields from, for example, 1 ng lambda DNA template with this system.

Moreover, products in the range 6.6 to 8.4 kb can be efficiently amplified by a formulation of thermostable DNA polymerases consisting of a majority component comprised of at least one thermostable DNA polymerase lacking 3'-exonuclease activity and a minority component comprised of at least one thermostable DNA polymerase exhibiting 3'-exonuclease activity.

The prior art technology only allowed relatively inefficient and sporadic amplification of fragments in this size range, resulting in only relatively faint product bands or no detectable product at all. In light of the current discovery, I believe I understand (without limiting myself to any particular theory) the reason for the inefficiency of the prior art. It is believed that Thermus aquaticus DNA polymerase and its variants are slow to extend a mismatched base pair (which they cannot remove since they lack any 3'exonuclease). A couple of companies (New England Biolabs and Stratagene) have introduced thermostable enzymes which exhibit a 3'-(editing) exonuclease which should, one would think, allow the removal of mismatched bases to result in both efficient extension and more accurately copied products. In practice, these two enzymes (Vent and Pfu DNA polymerase) are unreliable and much less efficient than expected. One possible explanation for the unreliability of these enzymes for PCR is that the 3'-exonuclease often apparently attacks and partially degrades the primers so that little or no PCR is possible. This primer attack problem is worse for some primers than others. It has been reported (Anonymous, The NEB Transcript, New England Biolabs, (March, 1991) p. 4.) that the Vent DNA polymerase leaves the 5' 15 nt intact, so that if the annealling conditions allow that 15 nt to prime, PCR could presumably proceed. This would of course only allow annealling at lower, non-selective temperatures, and the 5' 15 nt of the primers must be exactly homologous to the template.

I have discovered that the beneficial effects of a 3'-exonuclease can be obtained with an unexpectedly minute presence of one or more DNA polymerases which exhibit significant (defined as being biochemically assayable) 3'-exonuclease activity (herein called "E2") such as certain Archaebacterial DNA polymerases, whilst efficient extension is being catalyzed by a large amount of one or more DNA polymerases which lack any significant 3'-exonuclease activity, such as Klentaq-278 or AT (herein called "E1"). As a minority component of a formulation or mixture of DNA polymerases, the unreliability and inefficiency of the 3'-exonuclease DNA polymerase, discussed above, is substantially reduced or eliminated. Moreover, since it is believed that the 3'-exonuclease is removing mismatches to eliminate pausing at the mismatches, the resulting DNA exhibits fewer base pair changes, which is a valuable decrease in the mutagenicity of PCR without sacrificing flexibility, specificity, and efficiency. In fact, the combination, even for KlenTaq-278/Pfu units ratios as high as 2000, exhibited greatly increased efficiency of amplification. For most applications, the mixture of DNA polymerases must be at a relative DNA polymerase unit ratio of E1 to E2 of at least about 4:1. before enhanced product length and yield can be achieved. When Pfu DNA polymerase was used in the formulation, the ratio preferably is in the range 80 to 1000 parts KlenTaq-278 per part (unit) Pfu, more preferably from about 150 to about 170:1, and most preferably, is about 160:1, depending somewhat on the primer-template combination. Similar ratios are preferred for mixtures of Pfu and Klentaq-291.

If Deep Vent is substituted for Pfu for use in combination with Klentaq-278 or -291, the most preferred ratios for most applications increases to from about 450 to about 500:1 E1 to E2; if full-length (WT) Taq or Amplitaq is included as E1, the most preferred ratio to Pfu or other E2 component is between about 10 and about 15:1 of E1 to E2.

E2 of the invention includes, but is not limited to, DNA polymerase encoded by genes from Pfu, Vent, Deep Vent, T7 coliphage, Tma, or a combination thereof. E1 of the invention includes, but is not limited to, a mutant, 3'-exonuclease negative form of an E2 DNA polymerase, or alternatively, a DNA polymerase which, in unmutated form, does not exhibit significant 3'-exonuclease activity, such as the DNA polymerases encoded by genes from Taq, Tfl, or Tth, or a combination thereof.

As discussed below, the formulation of DNA polymerases of the present invention also includes formulations of DNA polymerase wherein E1 comprises a reverse transcriptase such as Sequenase.

Additional examples of the formulations of the present invention include mixtures wherein E1 comprises or consists of a mutant or chemical modification of T7 or T3 DNA polymerase and E2 comprises or consists of a wild-type T7 or T3 DNA polymerase, or, in another variation, E1 comprises or consists of a Vent DNA polymerase lacking any significant 3'-exonuclease activity (sold by New England Biolabs as Vent exo-) and E2 comprises or consists of Vent.

The principal here discovered, namely the use of low levels of 3' exonuclease during primer extension by a DNA polymerase lacking 3' exonuclease, is applicable to general DNA polymerase primer extensions, including normal temperature incubations (i.e. using non-thermostable DNA polymerases) and including reverse transcriptase enzymes, which are known to lack a 3'-(editing) exonuclease (Battula & Loeb, 1976). An example of the former is the use of Sequenase (exo-) as the majority enzyme, and wild-type T7 DNA polymerase (exo+) or Klenow fragment as the minority component. An example of the latter is AMV (Avian Myoblastosis Virus) or MLV (Murine Leukemia Virus) Reverse Transcriptase as the major component, and Klenow fragment, T7 DNA polymerase, or a thermostable DNA polymerase such as Pfu or Deep Vent as the minor component. Because of the lower activity of thermostable DNA polymerases at the temperatures of 37 degrees and 42 degrees used by these reverse transcriptases, higher levels are likely to be required than are used in PCR. Although Klenow fragment DNA polymerse is not a preferred DNA polymerase using RNA as a template, it does function to recognize this template (Karkas, 1973; Gulati, Kacian & Spiegelman, 1974), particularly in the presence of added Mn ion. Added Mn ion is routinely used to achieve reverse transtription by thermostable DNA polymerase Tth, unfortunately (in the prior art) without the benefit of an exo⁺ component. It must be stressed that for the use of the exo⁺ component for reverse transcriptase reactions, extra care must be taken to ensure that the exo⁺ component is entirely free of contaminating RNAse.

The following references describe methods known in the art for using reverse transcriptases, and are hereby incorporated by reference.

Battula N. Loeb LA. On the fidelity of DNA replication. Lack of exodeoxyribonuclease activity and error-correcting function in avian myeloblastosis virus DNA polymerase. Journal of Biological Chemistry. **251(4):982-6, 1976 Feb 25.**

Gulati SC. Kacian DL. Spiegelman S. Conditions for using DNA polymerase I as an RNA-dependent DNA polymerase. Proceedings of the National Academy of Sciences of the United States of America. **71(4):1035-9, 1974 Apr.**

Karkas JD. Reverse transcription by Escherichia coli DNA polymerase I. Proc Natl Acad Sci U S A. **70(12)**:3834-8, 1973 Dec.

### DNA Polymerase with no polymerase activity, only 3'-exonuclease activity:

While not limiting himself to a particular theory, applicant believes that the enzymatic activity of value in the minor (E2) component is the 3'-exonuclease activity, not the DNA polymerase activity. In fact, it is further believed that this DNA polymerase activity is potentially troublesome, leading to unwanted synthesis or less accurate synthesis under conditions optimized for the majority (E1) DNA polymerase component, not the minority one. As taught by [Bernad, Blanco and Salas (1990) Site-directed mutagenesis of the YCDTDS amino acid motif of the phi 29 DNA polymerase, Gene **94:45-51.**] who mutated the "Region I" DNA conserved DNA polymerase motif of phi 29 DNA polymerase, either Region III or Region I of the Pfu DNA polymerase gene are mutated, which has been sequenced by [Uemori,T., Ishino,Y., Toh,H., Asada,F. and Kato,I. Organization and nucleotide sequence of the DNA polymerase gene from the archaeon Pyrococcus furiosus, Nucleic Acids Res. **21, 259-265 (1993)**].

In a further embodiment of the present invention, a formulation of DNA polymerase is provided including or consisting of at least one DNA polymerase which, in wild-type form, exhibits 3'-exonuclease activity and which is capable of catalyzing a temperature cycle type polymerase chain reaction in which the 3'-exonuclease activity of the one or more DNA polymerases discussed above has been reduced substantially, but not eliminated. The diminished 3'-exonuclease activity is obtained by mutation, or by chemical or other means known to those in this art. This formulation of DNA polymerase may then be used to catalyze primer extension in PCR amplification. The Spanish researchers [Soengas, M.S., Esteban, J.A., Lazaro, J.M. Bernad, S., Blasco, M. A.,.l Salas, M., and Blanco, L., Embo Journal **11**:4227-4237 (1992)], studying a distantly related DNA polymerase of the alpha class which includes Pfu, Vent and Deep Vent DNA polymerases, also identified and demonstrated mutation of the 3'-exonuclease domain as well separated and easily avoided while one is mutating the DNA polymerase motif(s). In this same report, they demonstrate that the exonuclease can be reduced to 4-7% activity by a change of the conserved Tyr residue to a Phe or a Cys, whilst a reduction to only 0.1 % activity is obtained by replacing the conserved Asp with Ala. For long and accurate PCR, the optimum exo-(3'-exonuclease negative) mutation to create in a thermostable DNA polymerase such as Pfu, Vent or Deep Vent, is one that reduces but does not eliminate the exonuclease, for instance a reduction to 0.2-7%, preferably, 0.5-7%, and most preferably, 1-7% of the 3'-exonuclease activity of the wild-type DNA polymerase.

As an alternative way to introduce the mutations (and as demonstrated for the Bt CryV gene example above) Klentaq-LA, the current invention, is used to introduce the changes into a small PCR product spanning the DNA sequence coding for the two homologies REGION III and REGION I. The changes are chosen to change conserved amino acids in REGION III and/or REGION I, using as a guide the conserved motifs displayed below with the aid of the MACAW computer program.

I (data details not shown, but analogous to Example 1 above and readily carried out by one skilled in the art) have PCR-amplified the ORF of the Pfu DNA polymerase gene from Pfu DNA, using the sequence published by Uemori et al (1993) as a guide. Analogously to Example 2 above, I have cloned this ORF into the same expression vector as used for expression of Klentaq-278, and I have shown that the DNA polymerase can be purified by the same procedure as described here for Klentaq-278 in Example 3 above.

As an alternative way (alternative to Soengas et. al, supra) to introduce the mutations (and as demonstrated for the Bt CryV gene example above) Klentaq-LA, the current invention, would be used to introduce the changes into a small PCR product spanning the DNA sequence coding for the two homologies REGION III and REGION I. The changes would be chosen to change conserved amino acids in REGION III and/or REGION I, using as a guide the conserved motifs displayed below with the aid of the MACAW computer program.

The following is numbered output from the computer program MACAW, demonstrating two of the DNA polymerase conserved motifs. A useful presentation of these and other DNA polymerase motifs can also be found in Perler et al (1992) P.N.A.S. 89, 5577-5581.

The following examples illustrate the invention.

### EXAMPLE 1

### Construction of an Expressible Gene for Klentaq-278

In order to construct the Klentaq-278 DNA polymerase gene having a recombinant DNA sequence shown as the nucleotide sequence of Figure 1, the following procedure was followed.

The mutated gene was amplified from 0.25 ug of total Thermus aquaticus DNA using the polymerase chain reaction (PCR, Saiki et al., Science 239:487, 1988) primed by the two synthetic DNA primers of Figure 1. Primer KT1, SEQ ID NO:1, has homology to the wild-type DNA starting at codon 280; this primer is designed to incorporate a NcoI site into the product amplified DNA. Primer Klentaq32, SEQ ID NO:3, a 33mer spanning the stop codon on the other strand of the wild-type gene encoding Thermus aquaticus DNA polymerase, and incorporating a HindIII site and a double stop codon into the product DNA.

The buffer for the PCR reaction was 20 mM Tris HCI pH 8.55, 2.5 mM MgCl₂, 16 mM (NH₄)₂SO₄, 150 ug/ml BSA, and 200 uM each dNTP. The cycle parameters were 2' 95°, 2' 65°, 5' 72°.

In order to minimize the mutations introduced by PCR (Saiki et al., supra), only 16 cycles of PCR were performed before phenol extraction, ethanol precipitation, and digestion with the restriction enzymes NcoI and HindIII.

### EXAMPLE 2

### Preparation of an Expression Vector

The product NcoI and HindIII fragment was cloned into plasmid pWB254b which had been digested with NcoI, HindIII, and calf intestine alkaline phosphatase. The backbone of this plasmid, previously designated pTAC2 and obtained from J. Majors, carries the following elements in counterclockwise direction from the PvuII site of pBR322 (an apostrophe ' designates that the direction of expression is clockwise instead of counter clockwise): a partial lacZ' sequence, lacI', lacPUV5 (orientation not known), two copies of the tac promoter from PL Biochemicals Pharmacia-LKB; catalog no. 27-4883), the T7 gene 10 promoter and start codon modified to consist of a NcoI site, a HindIII site, the trpA terminator (PL no. 27-4884-01), an M13 origin of replication, and the Amp^{R} gene of pBR322. Expression of the cloned gene is expected to be induced by 0.1 mM IPTG.

Ampicillin-resistant colonies arising from the cloning were assayed by the single colony thermostable DNA polymerase assay of Sagner et al. (1991) [GENE **97**:119-23] and 4 strong positives were sized by the toothpick assay (Barnes, Science **195**:393, 1977). One of these, number 254.7, was of the expected size except for a small proportion of double insert. This plasmid was further purified by electroporation into E. coli X7029 and screened for size by the toothpick assay, and one plasmid of the expected size with no double insert contamination was designated pWB254b. This plasmid was used for the production of Klentaq-278 described herein.

### EXAMPLE 3

### Purification of Large Amounts of Klentaq-278

Plasmid pWB254 has a double (tandem repeat) tac promoter and the T7 gene 10 leader sequence, an ATG start codon, a glycine codon and then codons 280-832 of Thermus aquaticus DNA polymerase, then a tandem pair of stop codons followed by the trp transcription terminator. The pBR322-based plasmid vector (pTac2 from John Majors) is ampicillin resistant. The cells are grown on very rich medium (see below). Bacterial host X7029 is wild-type F⁻ E. coli except for deletion X74 of the lac operon.

Medium: Per liter water, 100 mg ticarcillin (added when cool), 10 g Y.E., 25 g. Tryptone, 10 g. glucose, 1XM9 salts with no NaCI (42 mM Na₂PO₄, 22 mM KH₂PO₄,19 mM NH₄Cl). Do not autoclave the glucose and the 10XM9 together; instead, autoclave one of them separately and mix in later. Adjust pH to 8 with 5 M NaOH (about 1 ml). Add IPTG to 0.1 mM at OD₅₅₀ = 1 or 2, and shake well at 30° C. From OD = 2 up to 8 or 10, every half hour or so do the following:
1. Read the pH with pH sticks 5-10. Adjust to pH 8.5 with 5 M NaOH and swirling (2 to 5 ml per liter) whenever the pH falls below 8.
2. Read and record the OD₅₅₀, usually as a 1/10 or 1/50 dilution.
3. This addition of glucose is optional and not necessarily of any value (evaluation of this question is incomplete at this time.) Read the glucose level with glucose sticks, and add an additional 0.5% (10 ml of 50%) if the level falls below 0.2%.

If it is late, the cells can shake at 30° C all night after the last pH adjustment. Alternatively, set them in the cold room if they have not grown much in a few hours.

Concentrate the cells e.g. by centrifugation in a GS3 rotor for 8 minutes at 8 krpm. Pour off the supernatant and add culture to spin more down onto the same pellets.

### Lysis:

Resuspend the cells in milliliters of TMN buffer equal to twice the packed cell weight in grams: (50 mM Tris-HCl pH 8.55, 10 mM MgCl₂, 16 mM (NH₄)₂SO₄).

To each 300 ml of cell suspension add 60 mg lysozyme and incubate the cells at 5-10° C. with occasional swirling for 15 minutes. Then add NP40 or Triton X100 to 0.1%, and Tween 20 to 0.1%, by adding 1/100 volume of a solution of 10% in each. Then heat the cell suspension rapidly to 80° C. by swirling it in a boiling water bath, then maintain the cells (fast becoming an extract) at 80-81° C. for 20 minutes. Use a clean thermometer in the cells to measure temperature. Be sure the flask and bath are covered, so that even the lip of the flask gets the full heat treatment. After this treatment, which is expected to have inactivated all but a handful of enzymes, cool the extract to 37° C. or lower in an ice bath and add 2 ml of protease inhibitor (100 mM PMSF in isopropanol). From this point forward, try not to contact the preparation with any flask, stir bar, or other object or solution that has not been autoclaved. (Detergents and BME are not autoclavable. The PEI and ammonium sulfate are also not autoclaved.) The purpose of the autoclaving is not only to avoid microbial contamination, but also to avoid contamination with DNA or nucleases.

Distribute into centrifuge bottles and centrifuge at 2° C. (for instance, 30 minutes at 15 krpm in a Sorval SS-34 rotor or 14 h at 4 krpm in a GS3 rotor). The supernatant is designated fraction I, and can be assayed for DNA polymerase activity.

### High-salt PEI precipitation

**After rendering fraction I 0.25 M in NaCl** (add 14.6 g per liter), add five percent Polymin-P (PEI, polyethylene-imine, Sigma) dropwise with stirring on ice to precipitate nucleic acids. To determine that adequate Polymin-P has been added, and to avoid addition of more than the minimum amount necessary, test 1/2 ml of centrifuged extract by adding a drop of Polymin-P, and only if more precipitate forms, add more Polymin-P to the bulk extract, mix and retest. Put the test aliquots of extract back into the bulk without contaminating it.

To confirm that enough PEI has been added, centrifuge 3 ml and aliquot the supernatant into 1/2 ml aliquots. Add 0, 2, 4, 6 or 10 ul of 5% PEI. Shake, let sit on ice, and centrifuge in the cold. Load 15 ul of these aliquot supernatants onto an agarose gel containing ethidium bromide and electrophorese until the blue dye has travelled 2 cm. Inspect the gel on a UV light box for detectable DNA or RNA in the supernatant. For the bulk extract, use about 1/100 volume (i.e. 2-3 ml for a 300 ml extract) excess 5% PEI over the minimum necessary to remove all DNA by the agarose gel test.

Stir in the cold for at least 15 minutes. Centrifugation of the extract then removes most of the nucleic acids. Keep the supernatant, avoiding any trace of the pellet.

Dilute the PEI supernatant with KTA buffer until the conductivity is reduced to at or below the conductivity of KTA buffer with added 22 mM ammonium sulfate. (Check conductivity of 1/40 dilution compared to similar dilution of genuine 22 mM A.S. in KTA.) Usually this is about a 5-fold dilution.

Chromatography with Bio-Rex 70 (used by Joyce & Grindley) (Joyce, C.M. & Grindley, N.D.E. (1983) Construction of a plasmid that overproduces the large proteolytic fragment (Klenow fragment) of DNA polymerase I of E. coli, Proc. Natl. Acad. Sci. U.S.A. 80, 1830-1834) is unsuccessful (no binding), but unavoidable, since without it, the next column (heparin agarose) will not work efficiently. We believe that the important function of the Bio-Rex 70 step is to remove all excess PEI, although it is possible that some protein is removed as well. CM-cellulose does not substitute for Bio-Rex 70.

Pass the diluted PEI supernatant through equilibrated Bio-Rex 70 (10 ml per 100 g. cells). The polymerase activity flows through. Rinse the column with 2 column volumes of 22 mM A.S. / KTA. Our procedure is to set up the following heparin agarose column so that the effluent from the Bio-REX 70 column flows directly onto it.

**Heparin Agarose Chromatography** (room temperature, but put fractions on ice as they come off.)

Load the Bio-Rex flow-through slowly onto heparin agarose (Sigma; 10 ml per 100 grams of cells [this could be too little heparin agarose].) Wash with several column volumes of KTA + 22 mM A.S., then three column volumes of KTA + 63% glycerol + 11 mM A.S., then elute the pure enzyme with KTA + 63% glycerol + 222 mM A.S. + 0.5% Thesit (this is more Thesit for the final eluate.)

Pool the peak of polymerase activity or OD₂₈₀/(starts about at 2/3 of one column volume after 222 mM starts, and is about 2 column volumes wide). Store pool at -20° C.

The storage buffer is a hybrid of, and a slight variation of, AmpliTaq storage buffer as recommended by Perkin-Elmer Cetus and Taq storage buffer used by Boehringer-Mannheim: 50% glycerol (v/v; 63 % w/v), 222 mM ammonium sulfate (diluted to about 50 mM for bench-strength samples), 20 mM Tris-HCI pH 8.55,0.1 mM EDTA, 10 mM mercaptoethanol, 0.5% Thesit).

The Thesit causes some thickening and cloudiness below -10° C. This seems to cause no harm, but we suggest you warm the enzyme to 0° C. on ice before aliquoting for use. Thesit replaces the combination of 0.5% Triton-X100, 0.5% Tween 20, which you may want to consider as an alternative.

We have had sporadic reports that freezing can inactivate the enzyme. Exercise caution in this regard. This question is under current investigation. Storage at -80° (after quick-cooling with liquid nitrogen) is being tested and looks promising, but more than one freeze-thaw cycle has been deleterious to the enzyme preparation on some occasions.

Our final yield of enzyme from 7 liters (100 g cells) was once 28 ml at a concentration of 120,000 units per ml (4 x bench-strength).

1/4 ul of bench-strength enzyme will support the PCR of a 2 kb span of DNA in a 100 ul reaction. Template is 5-10 ng of plasmid DNA. Each cycle consists of 1 min 98° C, 1 min 65° C, 6 min 72° C. Cycle number is 16-20. Less enzyme is needed for smaller-sized products (1/8 ul for 500 bp) and more enzyme is needed for larger products (1 ul for 5 kb).

| **KTA Buffer** | **per liter** |
|---|---|
| 20 mM Tris 8.55 | 10 ml of 2 M |
| 10 mM BME | 0.7 ml neat |
| 10% w/v Glycerol | 100 g. |
| 0.1 mM EDTA | 0.2 ml of .5 M |
| 0.1% w/v Thesit | 10 ml of 10% |

### Rough Incorporation Assay

1 X PC2 Buffer (20 mM Tris-HCl pH 8.55, 2.5 mM MgCl₂,
   16 mM (NH₄)₂SO₄, 100 ug/ml BSA)
200-250 ug/ml activated salmon sperm DNA
40 uM each dNTP + 10-50 uCi α-³²P-dATP per ml

To 25 ul assay mix on ice add 0.2 ul of enzyme fraction, undiluted, or diluted in 8 ul of IXPC2 buffer (or a 1/5 or 1/25 dilution thereof.) Prepare standard Klentaq or Amplitaq, zero enzyme and total input samples, also. Incubate 10 min. at 72° C., then chill. Spot 5 or 8 ul onto filter paper and wash twice for 5 - 10 min. with 5% TCA, 1 % PPᵢ. If pieces of paper were used, count each using Cerenkov radiation or hand monitor. If a single piece of 3 MM paper was used, autoradiograph for 60'.

### PCR Assay to give 2 kb product.

Make up 1 ml of PCR reaction containing 50 ng of plasmid pLc (a clone of an R color control cDNA from maize. PNAS 86:7092; Science 247:449), 200 pmoles each of primers Lc5 (SEQ ID NO:11) and Lc3 (SEQ ID NO:12), PC2 buffer and 200 uM dNTPs, but no enzyme.

Distribute 100 ul into tube one, and 50 ul into the rest of 8-10 tubes. Add 1 ul of final pool of KlenTaq to tube one and mix. Then remove 50 ul to tube two and mix that, and so on down the series, which will then contain decreasing amounts of enzyme in two-fold steps. Cover each 50 ul reaction with a drop of mineral oil, spin, and PCR 16 cycles at 2' 95° C, 2' 65° C, 5' 72° C.

### Final Bench-Strength KlenTaq-278 Enzyme

Using 63% glycerol / KTA (.5% Thesit) buffer with 222 mM ammonium sulfate, dilute the pool conservatively so that 1/4 ul should easily catalyze the amplification the 2 kb span by PCR. Do not decrease the ammonium sulfate concentration below 50 nM. Store at -20° C.

### EXAMPLE 4

### DNA Amplification

As reported in Figure 3, a PCR amplification assay to produce 2 kb of DNA product was conducted using Thermus aquaticus DNA polymerase (AmpliTaq) (prior art DNA polymerase) and Klentaq-278. To test polymerase thermostability at elevated temperatures, the DNA denaturation step of the PCR amplification reactions were conducted for 2 min. at 970C, 98°C and 990C, respectively, using graduated concentrations of DNA polymerase.

The amplification procedures used followed approximately the protocol for amplifying nucleic acid sequences outlined by Saiki et al., Science 239:487, 1988. A 1 ml reaction mixture was prepared containing 100 ng of plasmid pLC, 200 pmoles each of primers Lc5 (SEQ ID NO:11) and Lc3 (SEQ ID NO:12), reaction buffer (20 mM Tris-HCI pH 8.55, 16 mM ammonium sulfate, 2.5 mM MgCl₂ and 150 ug/ml BSA), 200 uM dNTPs, but no enzyme. 100 ul of the reaction mixture was placed into tubes. Aliquots of AmpliTaq and Klentaq-278 were then added and 20 cycles of PCR were undertaken.

Figure 3 shows the results of the experiment to compare the practical thermostability limits. The only change between the 3 panels shown is the temperature of the 2 min. denaturation step: 97° C, 98° C, or 99° C. A range of enzyme concentrations was used in order to be able to detect small effects on the effective PCR catalysis activity. The template was 10 ng of pLc (a clone of an R color control cDNA from maize. PNAS 86:7092, Science 247:449). The primers were Lc5 (SEQ ID NO:11) and Lc3 (SEQ ID NO:12). Other details of the reactions are given in the assay section of Example 3.

It can be seen in this experiment that 98° C was not detectably detrimental to KlenTaq-278, yet AT was nearly completely inactivated by this temperature.

In the experiment shown in Figure 4, each of four enzymes (AT, KlenTaq-278, ST, and KlenTaq-291) was tested for thermostability at 98° C. Each was tested in pairs of two concentrations differing by a factor of 2. The volumes of actual enzyme preparation are indicated above each lane in ul. The amount used was adjusted from previous titrations (conducted as described for the 2 kb PCR assay in Example 3 and the legend to Figure 3) so that a 2-fold drop-off in activity would be detectable. Note the large amount of ST necessary to function at the 95° C control PCR. A previous attempt at this experiment (data not shown) used only 1/4 these volumes of ST (which would have been equivalent standard DNA polymerase incorporation units compared to KT-291 and KT-278), and no product was obtained.

### EXAMPLE 5

### Single Colony PCR

The analysis of single E. coli colonies by PCR is a convenient screen for the presence and/or orientation of desired DNA fragments during a cloning or recloning procedure. In the prior art, the bacteria may not be simply added to a complete PCR reaction, since they evidently do not lyse efficiently enough to release the plasmid DNA that is to be the template for the PCR. Instead, and cumbersomely, since it requires a complete extra set of labelled test tubes, bacteria must first be suspended in water, not buffer, in the optional but recommended (Riggs et al.) presence of chelating resin, and heated to 100° C for several (such as 10) minutes. Then 1-10 ul of the heated bacterial suspension is added to an otherwise complete PCR reaction, which is then cycled and analyzed normally.

**The improvement here** is that, since Klentaq-278 can withstand 98-99° C. during the denaturation step of each PCR cycle, the bacteria can be added directly and conveniently to a complete (including Klentaq-278 enzyme) PCR reaction and then the PCR cycling can begin without further pretreatment. The only difference from a normal PCR cycling is that the full 98° C (2 min.) or 99° C (1 min.) temperature is used during each denaturation step (or at least the first 5-10 steps) of the PCR. The experiment in Figure 5 used 2 min. at 98° C for all 25 cycles, and demonstrates that this method gives rise to a more intense and reliably distinguished product band even than the prior art method which utilizes a 10' 100° C separate treatment. This improvement is not possible with AT enzyme, since AT enzyme is inactivated at 98° C (as shown in figures 3 and 4).

Figure 5 is a photograph of an agarose gel of a demonstration of the advantage of a 98° C denaturation step in colony PCR, compared to the standard 95° C temperature. Lanes 1 and 3 employed the prior art pre-treatment of the bacteria in distilled water at 100° C for 10 minutes before addition to the PCR reaction. Lanes 2 and 4 conveniently dispensed with this step and the same amount of bacterial suspension (about 2 to 4 X 10⁶ cells, but the identical volume of the same bacterial suspension) was simply introduced into the complete PCR reaction (including buffer, triphosphates, primers and enzyme KlenTaq-278.) Lanes 1 and 2 employed the standard 95° C, and lanes 3 and 4 employed the newly possible 98° C denaturation/cell-disruption temperature. The cycle conditions were 2 min. at 98° C or 95° C, 2 min. at 65° C, and 5' at 72° C., for 25 cycles. The primers used were KT2 (37mer GAG CCA TGG CCA ACC TGT GGG GGA GGC TTG AGG GGG A) and KlenTaq32 (see Figure 1). The bacterial cells were X7029 containing plasmid pWB319, a broad-host range plasmid containing the coding region of the gene for KlenTaq-278.

Lane 4 is the most convenient and the most effective method, and it takes advantage of the new stability of KlenTaq-278.

### EXAMPLE 6

### Efficent and Accurate PCR Amplification of Long DNA Targets: (Part A)

A preferred embodiment of the above formulation (designated KlenTaq-LA): Starting with the purified enzymes in storage buffer, mix 1 ul of Pfu DNA polymerase at 2.5 u./ul with 64 ul of KlenTaq-278 at 25 u./ul. Store at -20° C.

Larger amounts of Pfu are detrimental to some PCR amplifications, perform equally for some, and are beneficial for some. For testing of the optimum level of Pfu, several reactions complete with KlenTaq-278 are aliquoted in the amount left to right of 75 ul, 25 ul, 25 ul, and as many additional 25 ul aliquots as desired. Then 3/8 ul of Pfu (equivalent to 0.5 ul per 100 ul -- this is about the most that one would ever want) is added to the leftmost, 75 ul reaction and mixed. Serial, two-fold dilutions are then made as 25 ul + 25 ul left to right along the row of tubes, adding no Pfu to the last one, as a control of KlenTaq-278 alone. A reaction of 1/2 or 1 ul (per 100 ul) of Pfu alone should also be run.

Reaction buffer is PC2 as above, supplemented with 200 uM of each dNTP and 800 uM of MgCl₂ (total Mg⁺⁺ 3.3 mM), and per 100 ul of reaction volume, 20 pmoles of each primer MBL (SEQ ID NO:7) and MBR (SEQ ID NO:8), and 30 ng of λplac5 intact phage. Per 100 ul of reaction volume, I or 1/2 ul of KTLA are effective levels of enzyme. Suitable PCR cycling conditions are two-temperature: 20 seconds at 94° C, 11 minutes at 70° C, for 20 cycles. Alternate cycling conditions include two-temperature PCR with 1 minute at 98° C and 10 minutes at 65° C. 10 to 16 ul are loaded onto an agarose gel for product analysis by staining with ethidium bromide. See Figure 6 for other details and variations. The template was λplac5, which carries a portion of the lac operon region of the E. coli genome. Thirty ng of phage DNA were included in each 100 ul of reaction volume, introduced as intact phage particles. The primers are homologous to wild-type lambda DNA and amplify λ DNA, not the lac DNA. Primer MBL No. 8757 (5' nucleotide matches base pair 27914 of λ DNA) is GCT TAT CTG CTT CTC ATA GAG TCT TGC (SEQ ID NO:7). Primer MBR No. 8835 (5' nucleotide matches bp 34570 of λ DNA) is ATA ACG ATC ATA TAC ATG GTT CTC TCC (SEQ ID NO:8). The size of the amplified product is therefore predicted to be 6657 bp.

As shown in Figure 6A and 6B, each DNA polymerase enzyme (KlenTaq-278 or Pfu) alone gives rise to a faint product band (except for some reactions, when Pfu alone does not work at all), but the combinations all give rise to product bands that are 20 to 50 times more intense than either enzyme can catalyze on its own.

Figure 6C, second lane from the right, shows the surprising result of adding as little as 1/64 ul of Pfu to 1 ul of KlenTaq-278 (a units ratio of 1/640). Not shown are data that as little as 1/200 ul (1/2000 in units) of Pfu contributed a noticeable improvement to the efficiency of this test amplification.

Vent DNA polymerase required 10-fold higher amounts (yet still minority amounts) for similar functionality.

An additional, beneficial, and unexpected attribute to the PCR reactions catalyzed by KlenTaq-LA was a phenomenal, never previously observed intensity and sharpness to the PCR product bands. In part, this increased yield is manifested by a dark area in the middle of the bands as photographed. This darker area in the ethidium flourescence is believed to be due to UV absorbance by the outside portions of the band, reducing the potential UV-activated flourescence. The system apparently allowed a much greater yield of product then did the prior art, which tended to create a broad smear of product, and increasing amounts of side product, when amplification was allowed to proceed to this extent.

### EXAMPLE 7

### Efficent and Accurate PCR Amplification of Long DNA Targets: (Part B)

Efficient amplification of 8.4 kb, 12.5 kb, 15 kb, and 18 kb was demonstrated by the experiment depicted in Figure 7. This experiment extended the demonstrated performance of the a preferred embodiment of the invention, 1/640 KlenTaq-LA, even further. The amplification was highly successful for the size range 8.4 to 15 kb, detectably successful for 18 kb, but not successful for an attempted 19.7 kb.

Eight different PCR reactions were run in this experiment, differing from each other in the template or amount of template or in the primer pair employed, as shown in the legend on Figure 7. Each reaction was divided 3 ways and cycled differently in parts A, B, and C. Between parts A and B, this experiment compared 20 cycles to 30 cycles at 94° denaturation phase. In parts B and C, this experiment compared 94° to 93° for 30 cycles. This experiment utilized 1.3 ul of Klentaq-LA (at a Klentaq-278/Pfu ratio of 640) per 100 ul of reaction. This may have been a little too much enzyme, since high enzyme has been associated in previous experiments with the catastrophic synthesis of product which cannot enter the gel, as occurred here for the reaction products in channels 2B and 6C. At the current stage of development of long PCR using the invention, this poor outcome occurs about 10% of the time.

Comparing conditions B and C, it is apparent that somewhat lower denaturation temperature is desirable. This is consistent with similar experiments comparing time at 94° C., in which yield of long PCR products was found to be decreased as the denaturation time increased in the order 2, 20, 60, and 180 seconds at 94° C for the denaturation step of each cycle. These data indicate that there was at least one weak link, i.e. least thermostable component, in the reactions which is subject to inactivation at 94°. Since 94° is below the temperature known to damage the DNA polymerase activity and the DNA, it is believed that it is not the thermolabile element. In an alternative embodiment of this aspect of the invention Pfu DNA polymerase is replaced as the minority component with a more thermostable 3'-exonuclease of a DNA polymerase such as, but not limited to. that from the Archaebacterium strain ES4, which can grow at temperatures up to 114° C [Pledger, R.J. and Baross, J.A., J. Gen. Microbiol. **137** (1991)], which maximum growth temperature exceeds that of the source of the Pfu DNA polymerase (103° C.; Blumentals, I.I. et al. (1990) Annals of the N.Y. Acad. Sci. **589**:301-314.)

In the experiment in Figure 7 the final intensity of the 15 kb band matched in only 20 cycles the yield obtained by Kainze et al.supra in 30 cycles for a band of similar size and from similar λDNA template amounts. This was a measure of the improved efficiency provided by the invention, and the further result was that the yield catalyzed by the invention in 30 cycles greatly exceeded the yield reported by these authors for 30 cycles. Accurate quantitation has not yet been carried out to measure the efficiency of the two methods, but inspection of Figure 7 compared to the figure published by Kainze et al. shows a yield for the 15 kb fragment that is estimated to be some 100 times higher. This corresponds approximately to a doubled efficiency of PCR extension.

### EXAMPLE 8

### Efficent and Accurate PCR Amplification of Long DNA Targets: (Part C)

### Materials and Methods

**DNA Polymerases.** DNA polymerases Vent and Deep Vent were supplied by New England Biolabs. Pfu DNA polymerase and its exo⁻ mutant were supplied by Stratagene at 2.5 units/ul. Klentaq-278 is an N-terminal deletion variant of Taq DNA polymerase (WMB, unpublished ). The deletion endpoint is between that of Klentaq5 (10) and Stoffel Fragment (3). Purified Klentaq-278 was as supplied by Ab Peptides, St. Louis, MO, USA at 25-35 units/ul (a protein concentration of about 0.7 ug/ul). One unit of DNA polymerase activity incorporates 10 nmoles of nucleotide in 30 min. at 72° C., utilizing activated (partially degraded) calf thymus DNA as template. Since activated calf thymus DNA is a somewhat undefined substrate and is structurally different from PCR reaction substrate, this assay was routinely eschewed in favor of a PCR-based assay to set the above stock concentration of Klentaq-278: the concentration of Klentaq-278 stock was adjusted so that 0.25 ul effectively (but .12 ul less effectively) catalyzes the amplification of a 2 kb target span from 10 ng of plasmid substrate with cycling conditions including 7 min. of annealing / extension at 65°. The mixture of 15/16 ul Klentaq-278 + 1/16 ul Pfu DNA polymerases is designated KlentaqLA-16.

**Agarose gel** electrophoresis employed 0.7% to 1 % agarose in 1XGGB (TEA) buffer [40 mM Tris acetate pH 8.3, 20 mM sodium acetate, 0.2 mM EDTA] at 2-3 v/cm, with 3% ficoll instead of glycerol in the loading dye. Figure 11 employed 1% agarose pulsed-field CHEF (11) with a switching time of 4 sec. Standard DNA fragment sizes in every figure are, in kilobases (kb): 23.1, 9.4, 6.6, 4.4, 2.3, 2.0, and 0.56. Figure 11 and 12 also have a full-length λplac5 standard band, 48645 bp.

All agarose gels were run or stained in ethidium bromide at 0.5 ug/ml and photographed (35 mm ASA 400 black and white film) or videographed (Alpha Innotech or Stratagene Eagle Eye) under UV illumination. While printing the gel photographs, the left halves of Figure 7 and 10 were exposed 50% less than the right halves.

**DNA primers** are listed in Table 1 and in the Sequence Listing.

**Lambda DNA templates**. λvacA, a gift from S. Phadnis, is a λEMBL4-vectored clone of the cytotoxin gene region of Helicobacter pylori DNA. This DNA was extracted and stored frozen. The other phage template DNAs λplac5 (12) and λK138 (13) were added as intact phage particles that had been purified by CsCI equlibrium centrifugation, dialyzed, and diluted in 1X PC2 buffer.

**Long and Accurate PCR.** PC2 Reaction buffer (10) consisted of 20 mM Tris-HCI pH 8.55 at 25°, 150 ug/ml BSA, 16 mM (NH_{42,}S04, 3.5 mM MgCl₂, 250 uM each dNTP. For success above 28 kb (at 35 kb), 1.5 ul of 2 M Tris base was added to each reaction, corresponding to pH 9.1 measured for the Tris-HCI component only at 20 mM in water at 25° C. Contact with a pH probe was detrimental to the reactions, so pH was only measured on separate aliquots, and found to be 8.76 in the final reaction at 25° C. Each 100 ul of reaction volume contained 20 pmoles of each primer, and 0. to 10 ng of phage DNA template. 0.8 or 1.2 ul of KlentaqLA-16 was appropriate for under 20 kb and over 20 kb, respectively. Reaction volumes per tube were 33-50 ul, under 40 ul of mineral oil in thin-walled (PGC or Stratagene) plastic test tubes.

PCR reactions utilizing the primers at the ends of λ required a preincubation of 5 min. at 68 °-72° to disrupt the phage particles and to allow fill-in of the λ sticky ends to complete the primer homology. Optimal cycling conditions were in a multiple-block instrument (Robo Cycler, Stratagene) programmed per cycle to 30 sec. 99°, 30 sec. 67°, and 11 to 24 min. at 68°, depending on target length over the range shown in Table 1. The second-best cycler was the Omnigene (HybAid), programmed under tube control per cycle to 2 sec. at 95°, then 68° for similar annealing/extension times. Unless otherwise stated, all of the experiments reported here used 24 cycles.

For reported results of comparison of conditions such as cycling temperatures and times, thermal cycler machines, thick and thin-walled tubes, etc., reactions were made up as 100 ul complete and then split into identical aliquots of 33 ul before subjecting to PCR cycling.

**Table 1.**

| Primer and template combinations. | | | | | |
|---|---|---|---|---|---|
| Product Size | SEQ ID | Left Primer | SEQ ID | Right Primer | Template DNA |
| 5.8 | 25 | MBL101 | 28 | MSA1933 | λK138 |
| 6657 | 7 | MBL | 8 | MBR | λplac5 |
| 8386 | 9 | MBL-1.7 | 8 | MBR | λplac5 |
| 8.7 | 26 | MBR001 | 29 | XR36 | λK138 |
| 12.1 | 22 | lacZ333 | 27 | MBR202 | λK138 |
| 12.5 | 7 | MBL 27mer | 8 | MBR 27mer or | λvacAl |
| | 25 | or MBL101 33mer | 27 | MBR202 33mer | |
| 15560 | 10 | MSA1928mer | 27 | MBR202 | λplac5 |
| | 28 | or MSA1933 33mer | | | |
| 18.0 | 25 | MBL101 | 27 | MBR202 | λK138 |
| 19.8 | 20 | λL36 | 24 | MBL002 | λK138 |
| 20707 | 25 | MBL101 | 29 | λR36 36mer | λplac5 |
| 19584 | 20 | λL36 | 22 | lacZ333 | λplac5 |
| 13971 | 26 | MBR00133mer | 29 | λR36 | λplac5 |
| 22.0 | 20 | λL36 | 21 | lacZ'533 | λK138 |
| 24.6 | 20 | λL36 | 28 | MSA1933 | λK138 |
| 22495 | 20 | λL36 | 23 | lacZ536 | λplac5 |
| 26194 | 21 | lacZ'533 | 29 | λR36 | λplac5 |
| | | | | | |
| 28083 | 20 | λL36 | 24 | MBL002 | λplac5 |
| 34968 | 20 | λL36 | 27 | MBR202 | λplac5 |

### Legend to Table 1.

Product sizes in integer base pairs are as predicted from the sequence and structure of λ and λplac5 as documented in Genbank accession no. J02459 and ref. (21). Product sizes with decimal points in kb were determined by comparison with these products and with the λ+HindIII size standards labelled λH3. The sequence of the primers is given in the Sequence Listing.

**Megaprimer** consisted of gel-purified 384 bp PCR product DNA homologous to the region between the BamH1 site and EcoRI site of the gene coding for the CryV ICP of Bacillus thuringiensis (14), and primer-modified to remove these restriction sites. The PCR reactions in Figure 10 each employed megaprimer (300 ng) , primer BtV5 and 20 ng of genomic DNA from Bacillus thuringiensis strain NRD12 (15), and enzyme as indicated in the descripiton above of Figure 9. Cycling conditions were 30 sec. 95°, 7 min. 60°, for 20 cycles.

**HindIII digestion**. Unfractionated, total PCR reactions for 28 and 35 kb targets were supplemented with 1/10 volume of IOXNaTMS (ΔX = 50 mM NaCI, 10 mM Tris-HCI pH 7.7, 10 mM MgCl₂, 10 mM mercaptoethanol) and 2 ul (10 units) of restriction enzyme Hindlll, and incubated at 55° C. for 90 ruin.

**Test of exo- Pfu.** Each 100 ul of reaction (incubated as 33 ul under 40 ul of oil) contained 2 ng λplac5 DNA as purified phage particles, 20 pmoles each of primers MBL-1.7 and MBR , reaction buffer PC2 and 1 ul of Klentaq-278 (0.7 ug), except for reaction 6, which contained 1 ul Pfu DNA polymerase (2.5 u.) alone. Other details are in the description of Fig. 12. Thermal conditions were 24 cycles of 2 sec.at 94°, 11 min. at 70°.

The discovery leading to the DNA polymerase mixture of the present invention was made during attempts to utilize in PCR a primer with a mismatched A-A base-pair at its 3' end. In fact the primer was itself a PCR product "megaprimer" of 384 bp, and the mismatched A had been added by Klentaq-278 using non-templated terminal transferase activity common to DNA polymerases (16). Neither Klentaq-278 (Figure 9, lane 3) nor Pfu DNA polymerase (Figure 9, lanes 1 & 2 and other levels of enzyme not shown) could catalyze amplification of the 1500 bp target that lay between the PCR-product megaprimer and a 42mer oligonucleotide primer. The combination of the two enzymes, however, was well able to catalyze amplification of the desired target fragment (Fig 9, lane 4). Evidently, the Pfu DNA polymerase removed the presumed 3' A-A mismatch, allowing Klentaq-278 catalysis to proceed efficiently for each step of the PCR. The same result was obtained with Vent DNA polymerase substituted for Pfu (data not shown).

I hypothesized that mismatched 3'-ends are a general cause of inefficient primer extension during PCR of targets larger than a few kb. As a test system I employed a 6.6 kb lambda DNA target which was amplified detectably but poorly by AmpliTaq, Klentaq-278 or Pfu DNA polymerase in a variety of standard conditions. Per 100 ul reaction volume, 1 ul of Klentaq-278 was combined with various amounts of Pfu DNA polymerase, from 1/2 ul down to as little as 1/200 ul of Pfu. Since the Pfu stock (2.5 units/ul) was at least 10 times less concentrated than the Klentaq-278 stock (25-30 units/ul), the actual ratios tested were 1/20 to 1/2000 in DNA polymerase units. Representative results of these tests are shown in Figure 6B. A high yield of target band was observed for all tested combinations of the two enzymes, yet several levels of each enzyme on its own failed to catalyze more than faintly detectable amplification. The lowest level of Pfu tested, 1/200 ul, exhibited only a slight beneficial effect. The apparent broad optimum ratio of Klentaq-278:Pfu1 was 16 or 64 by volume, which is about 160 or 640 on the basis of DNA polymerase incorporation units. When tested at 6-8 kb (data not shown), other combinations of 3'-exo⁻ and 3'-exo⁺ thermostable DNA polymerases also showed the effect, including Amplitaq/Pfu, Klentaq-278/Vent, Klentaq5 (DeltaTaq, USB) / Pfu, Stoffel Fragment/Pfu, Klentaq-278/Deep Vent (our second choice), and Pfu exo- / Pfu exo+. Although comparatively few trials and optimizations were carried out, no other combination tried was as effective as Klentaq-278/Pfu.

**A very short heat step is preferred.** I next attempted to amplify DNA in the size range 8.4 to 18 kb from lambda transducing phage template. Our early cycling protocol employed a denaturation step of 1 or 2 minutes at 95° or 98° C, but no useful product in excess of 8.4 kb was obtained until the parameters of this heat step were reduced to 2 sec. or 20 sec. at 93° or 94° C. In an experiment with the denaturation step at 94° for 20, 60, or 180 sec, the 8.4 kb product exhibited decreasing yield with increased length of this heat step (data not shown). Apparently, a component of the reaction is at its margin of thermostability. Figure 7 shows that, using the short 2 sec. denaturation step, target fragment was obtained for some reactions at all sizes in the range 8.4 to 18 kb, with very high product yields up to 15 kb if 30 PCR cycles were employed. Figure 7 also shows some failed reactions which I cannot explain. The failure mode that gives rise to massive ethidium staining in the sample well (30-cycle lane 2) was particularly common, especially at high enzyme levels.

**Longer Primers.** A change in primer length from 27 to 33 greatly reduced the frequency of failed reactions. Figure 10 demonstrates improved reliability for amplification of 12.5, 15 and 18 kb with the longer 33mer primers, under conditions of otherwise optimally high enzyme levels in which the 27mer primers failed to give rise to desirable target product. This result does not represent an extensive survey of primer length, and it has not yet been repeated with the improvements below. Therefore the optimum primer length for long PCR remains to be determined. Some of the amplifications analyzed in Figure 11 utilized 36mer primers from the very ends of λ. A 2-5 min. preincubation at 68-72° (22) was necessary to release the template DNA from the phage particles and to fill in the sticky ends of lambda to complete the template homology with primers λL36 and λR36.

**Rapid cycling.** A change to thin-walled tubes, which have lower heat capacity and conduct heat more efficiently, further improved the reactions. Figure 11 shows a CHEF pulse-field agarose gel analysis of successful amplifications of DNA spans 6-26 kb in size. The target of 28 kb was not amplifiable in the Omnigene thermal cycler (data not shown), but did appear (Figure 12, lane 2) when the RoboCycler was employed.

Several models of thermal cycler have been employed, and although not all have been optimized, some are preferable to others for long PCR. As may be concluded from the advantage of thin-walled tubes noted above, success seems to be positively correlated with a high speed of temperature change made possible by the design of the thermal cycler. The RoboCycler achieves rapid temperature change by moving tubes from block to block, and observations with a thermistor temperature probe indicate that it raises the reactions to 93-95° for only 5 sec. under the denaturation conditions employed (30 sec. in the 99° block), before rapidly (within 30 sec) returning the reaction to 68°.

**Higher pH.** The current record 35 kb (Figure 12, lane 3) was only amplifiable if the pH was increased. A preliminary scan of higher pH was carried out (data not shown), and this resulted in the appearance of the 35 kb band at pH 8.8 to 9.2, with the optimum at 9.1 as described in Methods (above). Further improvement to a high yield of the 35 kb product was achieved by lengthening the extension time to 24 min. Other than the higher pH, the long PCR procedure has not yet realized any potential benefits from changes in buffer conditions from those optimized for 8.4 kb. For Targets over 20 kbs extension times exceeding 20 min. are preferred and the extension temperature is preferably below 69° C.

**Identity of long PCR products.** It can be seen in Figures 7, 10 and 11 that the mobilities of the successful large DNA products agree with those predicted in Table 1 from the known map positions of the primers used.

HindIII restriction enzyme digestion of the unpurified 28 and 35 kb products (Figure 12, lanes 6 and 7) resulted in the expected left arm of lambda (23 kb) and 2.3 kb band from both, and the predictable bands terminated by the right PCR primer: 447 bp (barely visible) from the 28 kb product and 7331 bp from the 35 kb product.

**Exonuclease mutant.** The available mutant of Pfu DNA polymerase (8) which is defective in the 3'-exonuclease activity was tested. Figure 13 shows that the 3'-exo⁻ mutant of Pfu DNA polymerase fails to promote efficient amplification of a long DNA target. This supports our hypothesis that the 3'-exonuclease activity is important for the efficiency of PCR amplification in this size range.

**Fidelity test.** Since the biological purpose of 3'-exonuclease is to edit base pair mismatches for high replication fidelity, we tested the fidelity of the PCR product using an assay involving the amplification and molecular cloning of an entire lacZ (β-galactosidase) gene flanked by two selectable markers (10). Heretofore the highest reported fidelity of PCR amplification is that catalyzed by Pfu DNA polymerase (2). Table 2 shows that the fidelity of the product amplified by the 640:1 mixture of Klentaq-278 and Pfu DNA polymerase at least matches that obtained for Pfu DNA polymerase, alone, when each are used for 16 cycles of PCR. Our designation of the enzyme mixture as Klentaq-LA (KlenTaq Long and Accurate) reflects this high fidelity performance.

**Table 2.**

| Non-silent mutations introduced into the lacZ gene by 16 cycles of PCR (10). | | | | | | |
|---|---|---|---|---|---|---|
| Enzyme | LacZ+ Blue or White | + LacZ- Light Blue | % mutant | Effective cycle no. (c) | Errors per 10⁵ bp (b) | Fold Improvement over full-length Taq |
| KTLA-64 | 571 | 34 | 5.6 | 12 | 1.05 | 12.7 |
| Pfu | 528 | 37 | 6.5 | 8 | 1.9 | 6.9 |
| Klentaq5^{a} | 442 | 85 | 16.1 | 8 | 5.1 | 2.6 |
| Klentaq1 | 3225 | 985 | 26.4 | 8 | 9.0 | 1.5 |
| Amplitaq | 525 | 301 | 36.4 | 8 | 13.4 | 1.0 |

| | | | | | | |
|---|---|---|---|---|---|---|
| (a) Klentaq⁵ is the N-terminal deletion of Taq DNA polymerase described in ref. 10. (b) Equation 1 of reference 10 was rearranged to be as follows to solve for errors per bp: X = -(ln(2F^{(1/m-1)} - 1 ) )/1000, where X is the errors per bp incorporated, 1000 is the effective target size in the lacZ gene (10), F is the fraction of blue colonies, and m is the effective cycle number. (c) As in ref. 10, the effective cycle number was estimated at less than the machine cycles to reflect the actual efficiency of the reaction, yet higher than the minimum calculated from the fold-amplification. Strand loss due to incomplete synthesis of product strands is a probable cause of lower than ideal amplification efficiency. Therefore successful (not lost) product molecules are judged to have undergone more than the calculated minimum number of replications. KTLA-64 (Klentaq-278:Pfu::64:1 by volume) was assigned a higher effective cycle number since its reactions started with 10 times less DNA (1.5 ng vs. 15 ng plasmid pWB305) to result in comparable levels of product. | | | | | | |

### DISCUSSION

The previous length limitation for PCR amplification is postulated to have been caused by low efficiency of extension at the sites of incorporation of mismatched base pairs. Although it would have seemed that the cure for these mismatches would be to employ enzymes with 3'-(editing)-exonucleases, I believe that when Pfu and Vent DNA polymerase are used to catalyze our amplifications on their own, their failure is due to degradation of the PCR primers by their 3'-exonucleases, especially during the required long synthesis times and at optimally high DNA polymerase levels. Evidently, low levels of 3'-exonuclease are sufficient and optimal for removal of the mismatches to allow the Klentaq-278 and amplification to proceed. It has been demonstrated that the optimally low level of 3'-exonuclease can be set effectively, conveniently, and flexibly by mixing and dilution.

Preferably the ratio of exo-/exo+ enzyme is high. If equal levels of the two types of enzymes are used (or where the E2 component is in excess), or if the ratio of exo-/exo+ is 4 or less, the effectiveness of the long PCR, even under optimal cycling conditions discussed below, is non-existent or much reduced.

It is preferred, and for certain applications, important that the length and temperature of the heat denaturation step of the PCR be kept to a minimum. Further, the improvement obtained by increasing the pH slightly may correspond to a decrease in template depurination. If so, further improvements may result if depurination can be reduced, or if a majority DNA polymerase component can be found which is able to bypass depurination sites.

The short denaturation time found to be optimal, preferably less than 20 sec., and most preferably, 5 sec. or less in the reaction itself at 95°, is surprisingly effective for the amplification of 35 kb, whereas it might have been expected that longer PCR targets would need longer denaturation time to become completely denatured. If complete denaturation is required for PCR, and if longer DNA requires more time to unwind at 95°, the required unwinding time may eventually become significantly more than 5 seconds. This could limit the size of amplifiable product because of the increased depurination caused by longer denaturation times.

These amplifications were successful with several different target sequences, with several primer combinations, and with product sizes up to nearly twice the maximum size of inserts cloned into λ. Whole viruses and plasmids up to 35 kb in length should now be amplifiable with this system. Should this method prove applicable to DNA of higher complexity than λ, it could prove a boon to genomic mapping and sequencing applications, since in vitro amplification is convenient and avoids the DNA rearrangement and gene toxicity pitfalls of in vivo cloning.

In view of the above, it will be seen that the several objects of the invention are achieved and other advantageous results attained.

As various changes could be made in the above methods and products without departing from the scope of the invention, it is intended that all matter contained in the above description shall be interpreted as illustrative and not in a limiting sense.

### References

1. Saiki, R.K., Gelfand, D.H., Stoffel, S., Scharf, S.J., Higuchi, R., Horn, G.T., Mullis, K.B., and Erlich, H.A. (1988) Science **239**, 487-491.
2. Lundberg, K.S., Shoemaker, D.D., Adams, M.W.W., Short, J.M. Sorge, J.A., and Mathur, E.J. (1991) Gene **108**, 1-6.
3. Lawyer, F.C., Stoffel, S., Saiki, R.K., Chang, S-Y., Landre, P.A., Abramson, R.D., and Gelfand, D.H. (1993) PCR Methods and Applications **2**, 275-287.
4. Jeffreys, A.J., Wilson, V., Neumann, R., and Keyte, J. (1988) Nucleic Acids Res. **16**, 10953-10971.
5. Krishnan, B.R., Kersulyte, D., Brikun, I., Berg, C.M. & Berg, D.E. (1991) Nucleic Acids Res. **19**, 6177-6182.
6. Maga, E.A., & Richardson, T. (1991) BioTechniques **11**: 185-186.
7. Ohler, L.D. & Rose, E. A. (1992) PCR Methods and Applications **2**, 51-59.
8. Rychlik, W., Spencer, W.J., and Rhoads, R.E. (1990) Nucleic Acids Res. **18**: 6409.
9. Kainz, P. Schmiedlechner, A., & Strack, H.B. (1992) Analytical Biochem. **202**, 46-49.
10. Barnes, W.M. (1992) Gene **112**, 29-35.
11. Chu, G.D., Vollrath, D. and Davis, R.W. (1986) Science **234**, 1582-.
12. Ippen, K., Shapiro, J.A., and Beckwith, J.R. (1971) J.Bact. **108**, 5-9.
13. Kohara, Y. (1990) pp 29-42 in The Bacterial Chromosome, eds Drlica, K. & Riley, M., ASM Washington D.C.
14. Tailor, R., Tippett, J., Gibb, G., Pells, S., Pike, D., Jordan, L., Ely, S. (1992) Mol. Microbiol. **6**, 1211-1217.
15. Dubois, N.R. Reardon, R.C., Kolodny-Hirsh, D.M. J.Econ.Entomol. **81**, 1672 (1988)],18.
16. Clark, J.M. (1988) Nucleic Acids Res. **16,** 9677-.
17. Brewer, A.C., Marsh, P.J and Patient, R.K. (1990) Nucleic Acids Res. **18**, 5574.
18. Lindahl, T. (1993) Nature **362**: 709-715.
19. Lindahl, T. & Nyberg, B. (1972) Biochemistry **11:** 3611-3618.
20. Sigma Chemical Co. Technical Bulletin No. 106B.
21. Shpakovski, G.V., Akhrem, A.A., and Berlin, Y.A. (1988) Nucleic Acids Res **16**, 10199 (1988).

### Table of Contents of Sequence Listing

SEQ ID NO:1: PCR primer KT1.
SEQ ID NO:2: N-terminus of Klentaq-278.
SEQ ID NO:3: PCR primer Klentaq32.
SEQ ID NO:4: C-terminus of Klentaq-278 and Taq DNA polymerase.
SEQ ID NO:5: pWB254b plasmid expression vector.
SEQ ID NO:6: Amino acid sequence of Klentaq-278, complete.
SEQ ID NO:7: PCR primer MBL on left side of lambda EMBL4 inserts.
SEQ ID NO:8: PCR primer MBR on right side of lambda EMBL4 inserts.
SEQ ID NO:9: PCR primer MBL-1.7, 1729 bp to left of MBL.
SEQ ID NO:10: PCR primer MSA19, at C-terminus of lacZ.
SEQ ID NO:11: PCR primer Lc5
SEQ ID NO:12: PCR primer Lc3
SEQ ID NO:13: PCR primer KT2
SEQ ID NO:14: Taq DNA polymerase gene, KT1 end
SEQ ID NO:15: Taq DNA polymerase gene, 3' end
SEQ ID NO:16: Tfl DNA polymerase gene, KT1 end
SEQ ID NO:17: Tfl DNA polymerase gene, 3' end
SEQ ID NO:18: PCR primer BtV3
SEQ ID NO:19: PCR primer BtV5
SEQ ID NO:20: PCR primer λL36
SEQ ID NO:21: PCR primer lacZ'533
SEQ ID NO:22: PCR primer lacZ333
SEQ ID NO:23: PCR primer lacZ536
SEQ ID NO:24: PCR primer MBL002
SEQ ID NO:25: PCR primer MBL101
SEQ ID NO:26: PCR primer MBR001
SEQ ID NO:27: PCR primer MBR202
SEQ ID NO:28: PCR primer MSA1933
SEQ ID NO:29: PCR primer λR36
SEQ ID NO:30: Phi 29 fragment 1, Region III
SEQ ID NO:31: Pfu fragment 1, Region III
SEQ ID NO:32: Phi 29 fragment 2, Region I
SEQ ID NO:33: Pfu fragement 2, Region I

## Claims

1. A recombinant DNA sequence encoding a DNA polymerase having an amino acid sequence selected from the group consisting of
a) the sequence identified in SEQ ID NO:6, and
b) a variant of SEQ ID NO:6 having the enzymatic activity and thermostability of the DNA polymerase of SEQ ID NO:6.

2. A DNA polymerase having an amino acid sequence selected from the group consisting of
a) the sequence identified in SEQ ID NO:6, and
b) a variant of SEQ ID NO:6 that encodes a DNA polymerase having the enzymatic activity and thermostability of the DNA polymerase of SEQ ID NO:6.

3. A DNA polymerase that is encoded by a DNA sequence contained in a plasmid having the DNA sequence of SEQ ID NO:5.

4. A recombinant DNA sequence encoding a DNA polymerase selected from the group consisting of
a) the amino acid sequence 280-831 of the DNA polymerase of Thermus flavus, the polymerase being encoded by the sequence in GenBank Accession No. X66105, and
b) a variant of said amino acid sequence, the variant having the enzymatic activity and thermostability of said amino acid sequence.

5. The recombinant DNA sequence of claim 4 encoding a DNA polymerase comprising amino acids 280-831 of the DNA polymerase of Thermus flavus encoded by the sequence in GenBank Accession No. X66105.

6. A DNA polymerase having an amino acid sequence selected from the group consisting of
a) the amino acid sequence 280-831 of the DNA polymerase of Thermus flavus, the polymerase being encoded by the sequence in GenBank Accession No. X66105, and
b) a variant of said amino acid sequence, the variant having the enzymatic activity and thermostability of said amino acid sequence.

7. The DNA polymerase of claim 6 comprising amino acids 280-831 of the DNA polymerase of Thermus flavus encoded by the sequence in GenBank Accession No. X66105.

8. A formulation of thermostable DNA polymerase comprising a majority component comprised of at least one thermostable DNA polymerase lacking 3'-exonuclease activity and a minority component comprised of at least one thermostable DNA polymerase exhibiting 3'-exonuclease activity.

9. A formulation of thermostable DNA polymerase as set forth in claim 8 wherein said at least one thermostable DNA polymerase lacking 3'-exonuclease activity is the enzyme of SEQ ID NO:6.

10. A formulation of thermostable DNA polymerase as set forth in claim 8 wherein said at least one thermostable DNA polymerase exhibiting 3'-exonuclease activity is selected from the group consisting of Pfu DNA polymerase from Pyrococcus furiosus, the Tli DNA polymerase from Thermococcus litoralis, a variant of the Pfu DNA polymerase and a variant of the Tli DNA polymerase wherein the DNA polymerase activity of said DNA polymerase has been diminished or inactivated.

11. A formulation of thermostable DNA polymerases as set forth in claim 8 wherein the majority component and minority component of the formulation are present in a ratio of from about 10 units to about 2000 units of the majority component to 1 unit of the minority component.

12. A formulation of thermostable DNA polymerase as set forth in claim 11 wherein the majority component and minority component of the formulation are present in a ratio of from about 100 to about 600 units of the majority component to 1 unit of the minority component.

13. A formulation of DNA polymerase comprising at least one DNA polymerase which in wild-type form exhibits 3'-exonuclease activity and which is capable of catalyzing a temperature cycle type polymerase chain reaction wherein said 3'-exonuclease activity of said at least one DNA polymerase has been reduced to between about 0.2% and about 7%, of the 3'-exonuclease activity of said at least one DNA polymerase in its wild-type form.

14. A formulation of DNA polymerase comprising E1, wherein E1 is one or more DNA polymerases which lack any significant 3'-exonuclease activity, and E2, wherein E2 is one or more DNA polymerases which exhibit significant 3'-exonuclease activity, and wherein the ratio of the amounts of El to E2 is at least about 4:1 by DNA polymerase units or (b) at least about 4:1 by amounts of protein by weight.

15. A formulation of DNA polymerase as set forth in claim 14 wherein E2 is selected from the group consisting of DNA polymerase encoded by genes from Pyrococcus furiosus, Thermococcus literalis, Thermococcus species GB-D, T7 coliphage, Thermotoga maritima or a combination thereof, and wherein E1 is selected from the group consisting of a mutant, 3'-exonuclease negative form of E2 or of DNA polymerases which, in unmutated form, do not exhibit significant 3'-exonuclease activity, such as DNA polymerase encoded by genes from Thermus aquaticus, Thermus flavus, or Thermus thermophilus.

16. A formulation of DNA polymerase as set forth in claim 14 wherein E2 comprises Pfu DNA polymerase from Pyrococcus furiosus and the unit ratio of E1 to E2 is between about 150 to 170:1.

17. A formulation of DNA polymerases as set forth in claim 14 comprising a unit ratio of from about 150 to about 170:1 of Klentaq-278 to Pyrococcus furiosus DNA polymerase.

18. A formulation of DNA polymerases as set forth in claim 14 comprising a unit ratio of from about 150 to about 170:1 of Klentaq-291 to Pyrococcus furiosus DNA polymerase.

19. A formulation of DNA polymerases as set forth in claim 14 wherein E2 comprises the DNA polymerase from Pyrococcus species GB-D and the unit ratio of El to E2 is between about 450 to 500.

20. A formulation of DNA polymerase as set forth in claim 14 comprising a unit ratio of from about 10 to about 15:1 of wild-type or nearly full-length Thermus aquaticus DNA polymerase to Pyrococcus furiosus DNA polymerase.

21. A formulation of DNA polymerase as set forth in claim 14 wherein E1 comprises a reverse transcriptase.

22. A formulation of DNA polymerase as set forth in claim 14 wherein E1 comprises a mutant or chemical modification of T7 or T3 DNA polymerase and E2 comprises a wild-type T7 or T3 DNA polymerase.

23. A formulation of DNA polymerase as set forth in claim 14 wherein E1 comprises Thermus flavus or Thermus thermophilus DNA polymerase.

24. A formulation of DNA polymerase as set forth in claim 14 wherein E1 comprises a Thermococcus literalis DNA polymerase variant lacking any significant 3'-exonuclease activity and E2 comprises wild-type Thermococcus literal is DNA polymerase.

25. A method for amplifying nucleic acid sequences by polymerase chain reaction, of the repeating cycle type wherein two complementary strands, for each different specific sequence being amplified, are treated with a single, a pair, or a mixture of pairs of oligonucleotide primers, and a DNA polymerase or a mixture of DNA polymerases is used to catalyze synthesis of an extension product of each primer which is complementary to each nucleic acid strand under effective conditions for said synthesis, and wherein said primers are selected so as to be sufficiently complementary to different strands of each specific sequence to hybridize therewith such that the extension product synthesized from one primer, when it is separated from its complement, can serve as a template for synthesis of the complementary strand of the extension product by extending the same or another included primer, the primer extension products are separated from the templates on which they were synthesized to produce single-stranded molecules, and the single-stranded molecules thus generated are treated with the primers and a DNA polymerase or a mixture of DNA polymerases is used to catalyze synthesis of an extension product of each primer under effective conditions for the synthesis of a primer extension product, wherein the improvement comprises formulating the DNA polymerase as set forth in claim 8, and catalyzing primer extension with said formulation of DNA polymerase.

26. A method for amplifying nucleic acid sequences by polymerase chain reaction, of the repeating cycle type wherein two complementary strands, for each different specific sequence being amplified, are treated with a single, a pair, or a mixture of pairs of oligonucleotide primers, and a DNA polymerase or a mixture of DNA polymerases is used to catalyze synthesis of an extension product of each primer which is complementary to each nucleic acid strand under effective conditions for said synthesis, and wherein said primers are selected so as to be sufficiently complementary to different strands of each specific sequence to hybridize therewith such that the extension product synthesized from one primer, when it is separated form its complement, can serve as a template for synthesis of the complementary strand of the extension product by extending the same or another included primer, the primer extension products are separated from the templates on which they were synthesized to produce single-stranded molecules, and the single-stranded molecules thus generated are treated with the primers and a DNA polymerase or a mixture of DNA polymerases is used to catalyze synthesis of an extension product of each primer under effective conditions for the synthesis of a primer extension product, wherein the improvement comprises formulating the DNA polymerase as set forth in claim 9, and catalyzing primer extension with said formulation of DNA polymerase.

27. A method as set forth in claim 26 wherein one or more of the primers utilized in any repeating cycle is itself a product of PCR amplification.

28. A method as set forth in claim 26 further comprising a denaturation step in each repeating cycle wherein the denaturation step had a duration of less than about 20 seconds in the reaction mixture.

29. A method as set forth in claim 28 wherein the denaturation step has a duration of less than about 5 seconds in the reaction mixture.

30. A method for amplifying nucleic acid sequences by polymerase chain reaction, of the repeating cycle type wherein two complementary strands, for each different specific sequence being amplified, are treated with a single, a pair, or a mixture of pairs of oligonucleotide primers, and a DNA polymerase or a mixture of DNA polymerases is used to catalyze synthesis of an extension product of each primer which is complementary to each nucleic acid strand under effective conditions for said synthesis, and wherein said primers are selected so as to be sufficiently complementary to different strands of each specific sequence to hybridize therewith such that the extension product synthesized from one primer, when it is separated from its complement, can serve as a template for synthesis of the complementary strand of the extension product by extending the same or another included primer, the primer extension products are separated from the templates on which they were synthesized to produce single-stranded molecules, and the single-stranded molecules thus generated are treated with the primers and a DNA polymerase or a mixture of DNA polymerases is used to catalyze synthesis of an extension product of each primer under effective conditions for the synthesis of a primer extension product, wherein the improvement comprises formulating a DNA polymerase comprising at least one DNA polymerase, which in wild-type form exhibits 3'-exonuclease activity and which is capable of catalyzing a temperature cycle type polymerase chain reaction, wherein said 3'-exonuclease activity of said at least one DNA polymerase has been reduced to no greater than about 7% of the 3'-exonuclease activity of said at least one DNA polymerase in its wild-type form, but not eliminated, and catalyzing primer extension with said formulation of DNA polymerase.

## Patentansprüche

1. Rekombinante DNA-Sequenz, die eine DNA-Polymerase mit einer Aminosäuresequenz kodiert, die ausgewählt ist aus der Gruppe bestehend aus
a) der in SEQ ID NO:6 angegebenen Sequenz, und
b) einer Variante von SEQ ID NO:6 mit der enzymatischen Aktivitat und Thermostabilität der DNA-Polymerase von SEQ ID NO:6.

2. DNA-Polymerase mit einer Aminosäuresequenz, ausgewählt aus der Gruppe bestehend aus
a) der in SEQ ID NO:6 angegebenen Sequenz, und
b) einer Variante von SEQ ID NO:6, welche eine DNA-Polymerase kodiert, die die enzymatische Aktivität und Thermostabilität der DNA-Polymerase von SEQ ID NO:6 aufweist.

3. DNA-Polymerase, welche von einer DNA-Sequenz kodiert wird, die in einem Plasmid mit der DNA-Sequenz von SEQ ID NO:5 enthalten ist.

4. Rekombinante DNA-Sequenz, die eine DNA-Polymerase kodiert, die ausgewählt ist aus der Gruppe bestehend aus
a) der Aminosäuresequenz 280-831 der DNA-Polymerase von Thermus flavus, wobei die Polyrnerase von der Sequenz in GenBank Hinterlegungsnummer X66105 kodiert wird, und
b) einer Variante der besagten Aminosäuresequenz, wobei die Vanante die enzymatische Aktivität und Thermostabilität der gesagten Aminosäuresequenz aufweist.

5. Rekombinante DNA-Sequenz nach Anspruch 4, die eine DNA-Polymerase kodiert, welche die Aminosäuren 280-831 der DNA-Polymerase von Thermus flavus umfaßt, die von der Sequenz in GenBank Hinterlegungsnummer X66105 kodiert wird.

6. DNA-Polymerase mit einer Aminosäuresequenz ausgewählt aus der Gruppe bestehend aus
a) der Aminosäuresequenz 280-831 der DNA-Polymerase von Thermus flavus, wobei die Polymerase von der Sequenz in GenBank Hinterlegungsnummer X66105 kodiert wird, und
b) einer Variante der besagten Aminosäuresequenz, wobei die Variante die enzymatische Aktivität und Thermostabilität der besagten Aminosäuresequenz aufweist.

7. DNA-Polymerase nach Anspruch 6, umfassend die Aminosäuren 280-831 der DNA-Polymerase von Thermus flavus, die von der Sequenz in GenBank Hinterlegungsnummer X66105 kodiert wird.

8. Formulierung einer thermostabilen DNA-Polymerase, umfassend einen Hauptbestandteil, der mindestens eine thermostabile DNA-Polymerase enthält, welcher eine 3'-Exonuklease-Aktivität fehlt, und einen Nebenbestandteil, der mindestens eine thermostabile DNA-Polymerase enthält, welche eine 3'-Exonuklease-Aktivität aufweist.

9. Formulierung einer thermostabilen DNA-Polymerase wie in Anspruch 8 angegeben, worin die mirdestens eine thermostabile DNA-Polymerase, welcher eine 3'-Exonuklease-Aktivität fehlt, das Enzym von SEQ ID NO:6 ist.

10. Formulierung einer thermostabilen DNA-Polymerase wie in Anspruch 8 angegeben, worin die mindestens eine thermostabile DNA-Polymerase, welche eine 3'-Exonuklease-Aktivität aufweist, ausgewählt ist aus der Gruppe bestenend aus Pfu-DNA-Polymerase von Pyrococcus furiosus, der Tli-DNA-Polymerase von Thermococcus litoralis, einer Vanante der Pfu-DNA-Polymerase und einer Vanante der Tli-DNA-Polymerase, worin die DNA-Polymerase-Aktivität der DNA-Polymerase verringert oder inaktiviert worden ist.

11. Formulierung von thermostabilen DNA-Polymerasen. wie in Anspruch 8 angegeben, worin der Hauptbestandteil und der Nebenbestandteil der Formulierung in einem Verhältnis von etwa 10 Einheiten bis etwa 2000 Einheiten des Hauptbestandteils zu 1 Einheit des Nebenbestandteils vorhanden sind.

12. Formulierung einer thermostabilen DNA-Polymerase, wie in Anspruch 11 angegeben, worin der Hauptbestandteil und der Nebenbestandteil der Formulierung in einem Verhältnis von etwa 100 bis etwa 600 Einheiten des Hauptbestandteils zu 1 Einheit des Nebenbestandteils vorhanden sind.

13. Formulierung von DNA-Polymerase umfassend mindestens eine DNA-Polymerase, welche in der Wildtypform eine 3'-Exonuklease-Aktivität aufweist und eine temperaturzyklusartige Polymerase-Kettenreaktion katalysieren kann, worin die 3'-Exonuklease-Aktivität der besagten mindestens einen DNA-Polymerase auf zwischen etwa 0,2% und etwa 7% der 3'-Exonuklease-Aktivität der besagten mindestens einen DNA-Polymerase in ihrer Wildtypform verringert worden ist.

14. Formulierung von DNA-Polymerase umfassend E1, worin E1 eine oder mehrere DNA-Polymerasen darstellt, welchen jegliche signifikante 3'-Exonuklease-Aktivität fehlt, und E2, worin E2 eine oder mehrere DNA-Polymerasen darstellt, welche eine signifikante 3'-Exonuklease-Aktivität aufweisen, und wobei das Verhältnis der Mengen von E1 zu E2 mindestens etwa 4:1, bezogen auf DNA-Polymeraseeinheiten, oder (b) mindestens etwa 4:1, bezogen auf die Proteinmengen, bezogen auf das Gewicht, beträgt.

15. Formulierung von DNA-Polymerase, wie in Anspruch 14 angegeben, worin E2 ausgewählt ist aus der Gruppe bestehend aus DNA-Polymerase, die von Genen von Pyrococcus furiosus, Thermococcus literalis, Thermococcus Spezies GB-D, T7 Coliphage, Thermotoga maritima oder einer Kombination davon kodiert wird, und worin E1 ausgewählt ist aus der Gruppe bestehend aus einer mutierten 3'-Exonuklease-negativen Form von E2 oder aus DNA-Polymerasen, welche in nichtmutierter Form keine signifikante 3'-Exonuklease-Aktivität aufweisen, wie etwa DNA-Polymerase, die von Genen von Thermus aquaticus, Thermus flavus oder Thermus thermophilus kodiert wird.

16. Formulierung von DNA-Polymerase, wie in Anspruch 14 angegeben. worin E2 Pfu-DNA-Polymerase von Pyrococcus furiosus umfaßt und das Einheitenverhältnis von El zu E2 zwischen etwa 150 bis 170:1 liegt.

17. Formulierung von DNA-Polymerasen, wie in Anspruch 14 angegeben, umfassend ein Einheitenverhältnis von etwa 150 bis etwa 170:1 von Klentaq-278 zu Pyrococcus furiosus-DNA-Polymerase.

18. Formulierung von DNA-Polymerasen, wie in Anspruch 14 angegeben, umfassend ein Einheitenverhältnis von etwa 150 bis etwa 170:1 von Klentaq-291 zu Pyrococcus furiosus-DNA-Polymerase.

19. Formulierung von DNA-Polymerasen, wie in Anspruch 14 angegeben, worin E2 die DNA-Polymerase von Pyrococcus Spezies GB-D umfaßt und das Einheitenverhältnis von E1 zu E2 zwischen etwa 450 bis 500 liegt.

20. Formulierung von DNA-Polymerase, wie in Anspruch 14 angegeben, umfassend ein Einheitenverhältnis von etwa 10 bis etwa 15:1 von der Wildtyp- oder nahezu Voll-Längen-Form der Thermus aquaticus-DNA-Polymerase zu Pyrococcus furiosus-DNA-Polymerase

21. Formulierung von CNA-Polymerase, wie in Anspruch 14 angegeben, worin E1 eine reverse Transkriptase umfaßt.

22. Formulierung von DNA-Polymerase, wie in Anspruch 14 angegeben, worin E1 eine Mutante oder chemische Modifikation von T7- oder T3-DNA-Polymerase umfaßt und E2 eine Wildtyp-T7- oder T3-DNA-Polymerase umfaßt.

23. Formulierung von DNA-Polymerase, wie in Anspruch 14 angegeben, worin E1 Thermus flavus- oder Thermus thermophilus-DNA-Polymerase umfaßt.

24. Formulierung von DNA-Polymerase, wie in Anspruch 14 angegeben, worin E1 eine Thermococcus literalis-DNA-Polymerase-Variante umfaßt, der jegliche signifikante 3'-Exonuklease-Aktivitat fehlt und E2 Wildtyp-Thermococcus literalis-DNA-Polymerase umfaßt.

25. Verfahren zum Amplifizieren von Nukleinsäuresequenzen durch eine Polymerase-Kettenreaktion von der Art eines sich wiederholenden Zyklus, wonn zwei komplementäre Stränge für jede verschiedene spezifische Sequenz, die amplifiziert wird, mit einem einzelnen, einem Paar oder einem Gemisch von Paaren von Oligonukleotidprimern behandelt werden und eine DNA-Polymerase oder ein Gemisch von DNA-Polymerasen verwendet wird, um die Synthese eines Extensionsprodukts von jedem Primer, welches zu jedem Nukleinsäurestrang komplementär ist, unter für diese Synthese effektiven Bedingungen zu katalysieren, und worin die Primer so ausgewählt werden, daß sie ausreichend komplementär zu verschiedenen Strängen von jeder spezifischen Sequenz sind, um damit zu hybridisieren, so daß das von einem Primer synthetisierte Extensionsprodukt, wenn es von seinem komplementären Strang abgetrennt ist, als Matrize für die Synthese des komplementären Strangs des Extensionsprodukts durch Ausdehnen desselben oder eines anderen eingeschlossenen Primers dienen kann, die Primerextensionsprodukte von den Matrizen, auf denen sie synthetisiert wurden, abgetrennt werden, um einzelsträngige Moleküle herzustellen, und die so erzeugten einzelsträngigen Moleküle mit den Primern behandelt werden und eine DNA-Polymerase oder ein Gemisch aus DNA-Polymerasen verwendet wird, um die Synthese eines Extensionsprodukts von jedem Primer unter für die Synthese eines Primerextensionsprodukts effektiven Bedingungen zu katalysieren. wobei die Verbesserung das Formulieren der DNA-Polymerase wie in Anspruch 8 angegeben und das Katalysieren der Primer-Extension mit der Formulierung der DNA-Polymerase umfaßt.

26. Verfahren zum Amplifizieren von Nukleinsäuresequenzen durch eine Polymerase-Kettenreaktion von der Art eines sich wiederholenden Zyklus, worin zwei komplementäre Stränge für jede verschiedene spezifische Sequenz, die amplifiziert wird, mit einem einzelnem, einem Paar und einem Gemisch von Paaren vcn Oligonukleotidprimern behandelt werden und eine DNA-Polymerase oder ein Gemisch von DNA-Polymerasen verwendet wird, um die Synthese eines Extensionsprodukts von jedem Primer, welches zu jedem Nukleinsäurestrang komplementär ist, unter für diese Synthese effektiven Bedingungen zu katalysieren, und wonn die Primer so ausgewählt werden, daß sie ausreichend komplementär zu verschiedenen Strängen von jeder spezifischen Sequenz sind, um damit zu hybridisieren, so daß das von einem Primer synthetisierte Extensionsprodukt, wenn es von seinem komplementären Strang abgetrennt ist, als Matrize für die Synthese des komplementären Strangs des Extensionsprodukts durch Ausdehnen desselben oder eines anderen eingeschlossenen Primers dienen kann, die Primerextensionsprodukte von den Matrizen, auf denen sie synthetisiert wurden, abgetrennt werden, um einzelsträngige Moleküle herzustellen, und die so erzeugten einzelsträngigen Moleküle mit den Primem behandelt werden und eine DNA-Polymerase oder ein Gemisch von DNA-Polymerasen verwendet wird, um die Synthese eines Extensionsprodukts von jedem Primer unter für die Synthese eines Primerextensionsprodukts effektiven Bedingungen zu katalysieren, wobei die Verbesserung das Formulieren der DNA-Polymerase wie in Anspruch 9 angegeben und das Katalysieren der Primer-Extension mit der Formulierung der DNA-Polymerase umfaßt.

27. Verfahren wie in Anspruch 26 angegeben, worin einer oder mehrere der Primer, die in einem sich wiederholenden Zyklus verwendet werden, selbst ein Produkt einer PCR-Amplifikation sind.

28. Verfahren wie in Anspruch 26 angegeben, ferner umfassend einen Denaturierungsschritt in jedem sich wiederholenden Zyklus, worin der Denaturierungsschritt eine Dauer von weniger als etwa 20 Sekunden in dem Reaktionsgemisch aufwies.

29. Verfahren wie in Anspruch 28 angegeben, worin der Denaturierungsschritt eine Dauer von weniger als etwa 5 Sekunden in dem Reaktionsgemisch aufweist.

30. Verfahren zum Amplifizieren von Nukleinsäuresequenzen durch eine Polymerase-Kettenreaktion von der Art eines sich wiederholenden Zyklus, worin zwei komplementäre Strenge für jede verschiedene spezifische Sequenz, die amplifiziert wird, mit einem einzelnen, einem Paar oder einem Gemisch von Paaren von Oligonukleotidprimern behandelt werden und eine DNA-Polymerase oder ein Gemisch von DNA-Polymerasen verwendet wird, um die Synthese eines Extensionsproduktes von jedem Primer, welches zu jedem Nukleinsäurestrang komplementär ist, unter für diese Synthese effektiven Bedingungen zu katalysieren, und worin die Primer so ausgewählt werden, daß sie ausreichend komplementär zu verschiedenen Strängen vcn jeder spezifischen Sequenz sind, um damit zu hybridisieren, so daß das von einem Primer synthetisierte Extensionsprodukt, wenn es von seinem komplementären Strang abgetrennt ist, als Matrize für die Synthese des komplementären Stranges des Extensionsproduktes durch Ausdehnen desselben oder eines anderen eingeschlossenen Primers dienen kann, wobei die Primerextensionsprodukte von den Matrizen, auf denen sie synthetisiert wurden, abgetrennt werden, um einzelsträngige Moleküle herzustellen, und die so erzeugten einzelsträngigen Moleküle mit den Primem behandelt werden und eine DNA-Polymerase oder ein Gemisch von DNA-Polymerasen verwendet wird, um die Synthese eines Extensionsprodukts von jedem Primer unter für die Synthese eines Primerextensionsproduktes effektiven Bedingungen zu katalysieren, worin die Verbesserung das Formulieren einer DNA-Polymerase umfassend mindestens eine DNA-Polymerase, welche in der Wildtypform eine 3'-Exonuklease-Aktivität aufweist und welche eine temperaturzyklusartige Polymerase-Kettenreaktion katalysieren kann, wobei die 3'-Exonuklease-Aktivität der besagten mindestens einen DNA-Polymerase auf nicht mehr auf etwa 7% der 3'-Exonuklease-Aktivität der besagten mindestens einen DNA-Polymerase in ihrer Wildtypform verringert worden, aber nicht eliminiert worden ist, und das Katalysieren der Primerextension mit der Formulierung der DNA-Polymerase umfaßt.

## Revendications

1. Séquence d'ADN recombiné codant une ADN polymérase ayant une séquence d'amino-acides choisie dans le groupe consistant en
a) la séquence identifiée dans SEQ ID NO : 6, et
b) un variant de SEQ ID NO : 6 ayant l'activité enzymatique et la thermostabilité de l'ADN polymérase de SEQ ID NO : 6.

2. ADN polymérase ayant une séquence d'amino-acides choisie dans le groupe consistant en
a) la séquence identifiée dans SEQ ID NO : 6, et
b) un variant de SEQ ID NO : 6 qui code une ADN polymérase ayant l'activité enzymatique et la thermostabilité de l'ADN polymérase de SEQ ID NO : 6.

3. ADN polymérase codée par une séquence d'ADN contenue dans un plasmide ayant la séquence d'ADN de SEQ ID NO : 5.

4. Séquence d'ADN recombiné codant une ADN polymérase choisie dans le groupe consistant en
a) la séquence d'amino-acides 280-831 de l'ADN polymérase de Thermus flavus, la polymérase étant codée par la séquence No X66105 d'entrée dans la banque génomique GenBank, et
b) un variant de ladite séquence d'amino-acides, le variant ayant l'activité enzymatique et la thermostabilité de ladite séquence d'amino-acides.

5. Séquence d'ADN recombiné suivant la revendication 4, codant une ADN polymérase comprenant les amino-acides 280-831 de l'ADN polymérase de Thermus flavus codée par la séquence No. X66105 d'entrée dans la banque génomique GenBank.

6. ADN polymérase ayant une séquence d'amino-acides choisie dans le groupe consistant en
a) la séquence d'amino-acides 280-831 de l'ADN polymérase de Thermus flavus, la polymérase étant codée par la séquence No. X66105 d'entrée dans la banque génomique GenBank, et
b) un variant de ladite séquence d'amino-acides, le variant ayant l'activité enzymatique et la thermostabilité de ladite séquence d'amino-acides.

7. ADN polymérase suivant la revendication 6, comprenant les amino-acides 280-831 de l'ADN polymérase de Thermus flavus codée par la séquence No. X66105 d'entrée dans la banque génomique GenBank.

8. Formulation d'ADN polymérase thermostable comprenant un composant majoritaire constitué d'au moins une ADN polymérase thermostable dépourvue d'activité de 3'-exonucléase et un composant minoritaire constitué d'au moins une ADN polymerase thermostable présentant une activité de 3'-exonucléase.

9. Formulation d'ADN polymérase thermostable suivant la revendication 8, dans laquelle l'ADN polymérase thermostable en question dépourvue d'activité de 3'-exonucléase est l'enzyme de SEQ ID NO : 6.

10. Formulation d'ADN polymérase thermostable suivant la revendication 8, dans laquelle l'ADN polymérase thermostable en question présentant une activité de 3'-exonucléase est choisie dans le groupe consistant en Pfu ADN polymérase dérivée de Pyrococcus furiosus, la Tli ADN polymérase dérivée de Thermococcus litoralis, un variant de la Pfu ADN polymérase et un variant de la Tli ADN polymérase dans lesquels l'activité d'ADN polymérase de l'ADN polymérase a été réduite ou inactivée.

11. Formulation d'ADN polymérases thermostables suivant la revendication 8, dans laquelle le composant majoritaire et le composant minoritaire de la formulation sont présents dans un rapport d'environ 10 unités à environ 2000 unités du composant majoritaire pour 1 unité du composant minoritaire.

12. Formulation l'ADN polymérase thermostable suivant la revendication 11, dans laquelle le composant majoritaire et le composant minoritaire de la formulation sont présents dans un rapport d'environ 100 à environ 600 unités du composant majoritaire pour 1 unité du composant minoritaire.

13. Formulation d'ADN polymérase comprenant au moins une ADN polymérase qui, dans sa forme de type sauvage, présente une activité de 3'-exonucléase, et qui est capable de catalyser une réaction de polymérisation en chaîne du type à cycle de température dans laquelle l'activité de 3'-exonucléase de l'ADN polymérase en question a été réduite à une valeur comprise entre environ 0,2 %, et environ 7 % de l'activité de 3'-exonucléase de cette ADN polymérase dans sa forme de type sauvage.

14. Formulation d'ADN polymérase comprenant E1, où E1 consiste en une ou plusieurs ADN polymérase qui sont dépourvues de toute activité importante de 3'-exonucléase, et E2, où E2 consiste en une ou plusieurs ADN polymérases qui présentent une activité de 3'-exonucléase importante, et dans laquelle le rapport de la quantité de E2 à la quantité de E2 est (a) d'au moins environ 4 :1 en unités d'ADN polymérase ou (b) d'au moins environ 4 : 1 en quantités en poids de protéine

15. Formulation d'ADN polymérase suivant la revendication 14, dans laquelle E2 est choisi dans le groupe consistant en ADN polymérase codée par des gènes issus de Pyrococcus furiosus, Thermococcus litoralis, Thermococcus species GB-D, coliphage T7, Thermotoga maritima ou une association de ces gènes et dans laquelle E1 est choisi dans le groupe consistant en une forme mutante sans activité de 3'-exonucléase de E2 ou d'ADN polymérases qui, sous la forme non mutée, ne présentent pas une activité importante de 3'-exonucléase, telles que l'ADN polymérase codée par des gènes issus de Thermus aquaticus, Thermus flavus ou Thermus thermophilus.

16. Formulation d'ADN polymérase suivant la revendication 14, dans laquelle E2 comprend la Pfu ADN polymérase issue de Pyrococcus furiosis et le rapport unitaire de E1 à E2 va de 150 à 170 :1.

17. Formulation d'ADN polymérases suivant la revendication 14, comprenant un rapport unitaire de Klentaq-278 à l'ADN polymérase de Pyrococcus furiosus d'environ 150 à environ 170 :1.

18. Formulation d'ADN polymérases suivant la revendication 14, comprenant un rapport unitaire de Klentaq-291 à l'ADN polymérase de Pyrococcus furiosus d'environ 150 à environ 170 :1.

19. Formulation d'ADN polymérases suivant la revendication 14, dans laquelle E2 comprend l'ADN polymérase de Pyrococcus species GB-D et le rapport unitaire de E1 à E2 est compris entre environ 450 et 500

20. Formulation d'ADN polymérase suivant la revendication 14, comprenant un rapport unitaire d'environ 10 à environ 15 : 1 de l'ADN polymérase de Thermus aquaticus à l'ADN polymérase de Pyrococcus furiosus de type sauvage ou presque entier.

21. Formulation d'ADN polymérase suivant la revendication 14, dans laquelle E1 comprend une rétrotranscriptase.

22. Formulation d'ADN polymérase suivant la revendication 14, dans laquelle E1 comprend un mutant ou une modification chimique d'ADN polymérase de T7 ou T3 et E2 comprend une ADN polymérase de T7 ou T3 de type sauvage.

23. Formulation d'ADN polymérase suivant la revendication 14, dans laquelle E1 comprend l'ADN polymérase de Thermus flavus ou Thermus thermophilus.

24. Formulation d'ADN polymérase suivant la revendication 14, dans laquelle E1 comprend un variant d'ADN polymérase de Thermococcus literalis dépourvu de toute activité importante de 3'-exonucléase et E2 comprend de l'ADN polymérase de Thermococcus literalis dc type sauvage.

25. Procédé d'amplification dc séquences d'acides nucléiques par réaction de polymérisation en chaîne, du type à cycles répétés dans lequel deux brins complémentaires, pour chaque séquence spécifique différente à amplifier, sont traités avec une seule amorce, une paire ou un mélange de paires d'amorces oligonucléotidiques, et une ADN polymérase ou un mélange d'ADN polymérases est utilisé pour catalyser la synthèse d'un produit d'extension de chaque amorce qui est complémentaire de chaque brin d'acides nucléiques dans des conditions efficaces pour ladite synthèse, et dans lequel ces amorces sont choisies de manière à être suffisamment complémentaires de brins différents de chaque séquence spécifique pour s'hybrider avec eux afin que le produit d'extension synthétisé à partir d'une amorce puisse, lorsqu'il est séparé de son complément, servir de matrice pour la synthèse du brin complémentaire dit produit d'extension par l'extension de la même amorce ou d'une autre amorce incluse, les produits d'extension sont séparés des matrices sur lesquelles ils ont été synthétisés pour produire des molécules monocaténaires et les molécules monocaténaires ainsi générées sont traitées avec les amorces, et une ADN polymérase ou un mélange d'ADN polymérases est utilisé pour catalyser la synthèse d'un produit d'extension de chaque amorce dans des conditions efficaces pour la synthèse d'un produit d'extension des amorces, où le perfectionnement comprend la formulation de l'ADN polymérase suivant la revendication 8 et la catalyse de l'extension des amorces avec ladite formulation d'ADN polymérase.

26. Procédé d'amplification de séquences d'acides nucléiques par réaction de polymérisation en chaîne, du type à cycles répétés dans lequel deux brins complémentaires, pour chaque séquence spécifique différente à amplifier, sont traités avec une seule amorce, une paire ou un mélange de paires d'amorces oligonucléotidiques, et une ADN polymérase ou un mélange d'ADN polymérases est utilisé pour catalyser la synthèse d'un produit d'extension de chaque amorce qui est complémentaire de chaque brin d'acides nucléiques dans des conditions efficaces pour ladite synthèse, et dans lequel ces amorces sont choisies de manière qu'elles soient suffisamment complémentaires de brins différents de chaque séquence spécifique pour s'hybrider avec eux afin que ce produit d'extension puisse servir, lorsqu'il est séparé de son complément, de matrice pour la synthèse du brin complémentaire du produit d'extension par l'extension de la même amorce ou d'une autre amorce incluse, les produits d'extension des amorces sont séparés dcs matrices sur lesquelles ils ont été synthétisés pour former des molécules monnocaténaires, et les molécules monocaténaires ainsi générées sont traitées avec les amorces, et une ADN polymérase ou un mélange d'ADN polymérases est utilisé pour catalyser la synthèse d'un produit d'extension de chaque amorce dans des conditions efficaces pour la synthèse d'un produit d'extension d'amorces, où le perfectionnement comprend la formulation de l'ADN polymérase suivant la revendication 9 et la catalyse de l'extension d'amorces avec ladite formulation d'ADN polymérase.

27. Procédé suivant la revendication 26, dans lequel une ou plusieurs des amorces utilisées dans tout cycle répété est elle-même un produit d'amplification par réaction de polymérisation en chaîne.

28. Procédé suivant la revendication 26, comportant en outre une étape de dénaturation dans chaque cycle répété, l'étape de dénaturation ayant une durée de moins d'environ 20 secondes dans le mélange réactionnel.

29. Procédé suivant la revendication 28, dans lequel l'étape de dénaturation a une durée de moins d'environ 5 secondes dans le mélange réactionnel.

30. Procédé d'amplification de séquences d'acides nucléiques par réaction de polymérisation en chaîne, du type à cycles répétés dans lequel deux brins complémentaires, pour chaque séquence spécifique différente à amplifier, sont traités avec une seule amorce, une paire ou un mélange de paires d'amorces oligonucléotidiques, et une ADN polymérase ou un mélange d'ADN polymérases est utilisé pour catalyser la synthèse d'un produit d'extension de chaque amorce qui est complémentaire de chaque brin d'acides nucléiques dans des conditions efficaces pour ladite synthèse, et dans lequel les amorces sont choisies de manière qu'elles soient suffisamment complémentaires de brins différents de chaque séquence spécifique pour s'hybrider avec eux, afin que lorsque le produit d'extension synthétisé à partir de l'une des amorces est séparé de son complément, il puisse servir de matrice pour la synthèse du brin complémentaire du produit d'extension par l'extension de la même amorce ou d'une autre amorce incluse, les produits d'extension des amorces sont séparés des matrices sur lesquelles ils ont été synthétisés pour produire des molécules monocaténaires, et les molécules monocaténaires ainsi générées sont traitées avec les amorces, et une ADN polymérase ou un mélange d'ADN polymérases est utilisé pour catalyser la synthèse d'un produit d'extension de chaque amorce dans des conditions efficaces pour la synthèse d'un produit d'extension d'amorces, où le perfectionnement comprend la formulation d'une ADN polymérase constituée d'au moins une ADN Polymérase, qui présente dans la forme de type sauvage une activité de 3'-exonucléase et qui est capable de catalyser unc réaction de polymérisation en chaîne du type à cycle de température, dans laquelle cette activité de 3'-exonucléase de l'ADN polymérase en question a été réduite à une valeur non supérieure à environ 7% de l'activité de 3'-exonucléase de cette ADN polymérase dans sa forme sauvage, mais non éliminée, et la catalyse de l'extension d'amorces avec cette formulation d'ADN polymérase.
